# EUROPEAN PATENT APPLICATION

(11) **EP 2 436 689 A1**
(43) Date of publication of application: **04.04.2012**
(21) Application number: 11183557.5
(22) Date of filing: 30.09.2011
(51) Int. Cl.: C07K 14/15, C12N 9/22, G06F 19/16

(54) **Novel dimeric association of the catalytic core domain of a retrovirus integrase and crystal structure thereof**

(30) Priority: 30.09.2010 EP 10290516
(71) Applicant: Universite Claude Bernard Lyon 1, 69622 Villeurbanne Cedex (FR)
(72) Inventor: Ballandras, Allison, 69100 Villeurbanne (FR); Gouet, Patrice, 69100 Villeurbanne (FR); Robert, Xavier, 69007 Lyon (FR); Ronfort-Vertadier, Corinne, 69740 Genas (FR); Moreau, Karen, 38230 Tignieu (FR); Haser, Richard, 69440 Taluyers (FR)
(74) Representative: Marcadé, Véronique

(57) **Abstract**

The present invention pertains to a crystal structure of a catalytic core domain of retroviral integrase. This structure can be used as a structural model for determining further integrase structures, and to identify, either *in silico* or by chemical screening, new anti-retroviral drugs.

## Description

The present invention pertains to a crystal structure of a catalytic core domain of retroviral integrase. This structure can be used as a structural model for determining further integrase structures, and to identify, either *in silico* or by chemical screening, new anti-retroviral drugs.

During the replicative cycle of retroviruses, the retrotranscribed viral DNA is integrated into the host chromosome by the viral integrase protein (IN) (1). The integration reaction is essential for the viral life cycle; therefore, IN is a key target for antiretroviral drug design (2-5). Retroviral integration proceeds in three steps, two of which are catalysed by IN. First, during the 3' processing, the two 3' terminal nucleotides of each viral DNA end are removed to generate CA-3'-OH ends with a two-base 5' overhang. Then, during the strand transfer, the recessed 3'-OH viral ends attack the phosphodiester bonds of the cellular DNA at cleavage sites separated by four to six base pairs (depending on the virus) and the viral DNA is joined to the host DNA. Finally, gap filling and DNA ligation are performed, probably by cellular enzymes (6-8).

Retroviral IN consists of three domains: the zinc-binding N-terminal domain (NTD), the catalytic core domain (CCD) and the C-terminal domain (CTD). The IN proteins of the Avian Leukaemia and Sarcoma Virus (ALSV) and Human Immunodeficiency Virus (HIV) are approximately 280 amino acids long. The NTD binds viral DNA (9) and target DNA (10,11) and promotes IN oligomerisation (12). The NTD is required for 3' processing and strand transfer *in vitro.* The CTD is known to bind both viral DNA and non-specific host DNA (13) and is also involved in oligomerisation (14). The central CCD contains an invariant D,D(35)E motif, which forms a catalytic triad with two sites that coordinate divalent metal cofactors (Mg²⁺ or Mn²⁺) or inhibitors (Zn²⁺ for the strand transfer reaction) (15-17). The single CCD is sufficient to perform an *in vitro* reaction termed disintegration, which is the reverse of the strand transfer reaction (18). This domain, which is the most conserved domain across retroviral INs (>20% sequence identity), appears to be the keystone of the integration process. The CCD belongs to the ribonuclease H-like superfamily (19,20) and consists of a five-stranded mixed β-sheet flanked by α-helices. It has always been solved as a dimer in partial or entire IN structures from lentivirus (HIV-1, HIV-2, Simian Immunodeficiency Virus (SIV), Maedi-Visna Virus (MVV), alpharetrovirus (Rous Sarcoma Virus (RSV)) and spumavirus (Prototype Foamy Virus (PFV)) with an intermolecular interface that always involves two pairs of facing α-helices(21).

The three domains are connected by flexible loops, making the full-length enzyme difficult to crystallise. Hence, the structure of IN was first investigated in fragments, such as the two-domain HIV-1 IN fragment (22,23) and the two-domain RSV IN fragment (24). Recently, 3D models of negatively stained full-length HIV-1 IN, alone or complexed with the cellular cofactor LEDGF/p75 and either viral or cellular DNA, were determined by electron microscopy (25,26). The crystal structure of the full-length IN from PFV complexed with its cognate viral DNA was determined soon thereafter (27). The EM and crystal structures confirm that two IN dimers are necessary for concerted integration. In each dimer, only one CCD active site binds viral DNA and performs the 3' processing and strand transfer. The two remaining CCD active sites of the tetramer lie far from the bound DNA ends and have no apparent role. Taken together, these structures further suggest that the NTD and the CTD can move during integration, and their positions diverge with respect to the CCD.

Rous-associated virus type 1 (RAV-1) is a replication-competent alpharetrovirus that is involved in ALSV, and RAV-1 IN is a good model for HIV IN. The inventors have now determined the crystal structure of the CCD of RAV-1 IN to 1.8 Å resolution. The resulting structure exhibits an unexpected new dimeric arrangement with potential biological implications. They have shown by docking calculations that this novel dimeric form could accommodate a single-stranded nucleic acid. The experimental data below also explain how crystallisation conditions, as well as the single amino-acid substitution between the RAV-1 IN CCD and the well-studied RSV IN CCD (equivalent to Avian Sarcoma Virus IN CCD), can favour either dimeric form during crystal growth.

A first aspect of the present invention is a crystal of a catalytic core domain (CCD) of retroviral integrase obtainable in a MES buffer at pH 6.0. The method for obtaining such a crystal is described in more details in the experimental part.

In a particular embodiment of the crystal according to the invention, said CCD consists of residues 54-199 of RAV-1 integrase.

In another particular embodiment, the MES buffer also comprises Zn(II).

The structure of RAV-1 CCD as defined by the coordinates listed in Table 2, is also part of the invention.

According to the invention, this structure or part thereof can be used as a structural model.

This structural model can be used for high-throughput chemical screening, and/or for the identification of one or more molecular species that modulate the CCD of retroviral integrase to inhibit at least part of its activity.

The structural model according to the invention can advantageously be used for the identification of an anti-retroviral drug, in particular for the identification of a drug for preventing, alleviating or curing an HIV infection.

The structural model according to the invention can also be used for determining at least a portion of the three-dimensional structure of a molecular species.

The present invention also pertains to a machine-readable data storage medium which comprises a data storage material encoded with machine readable data defined by the structure coordinates of the CCD of RAV-1 according to Table 2 or a homologue of this structure.

Other characteristics of the invention will also become apparent in the course of the description which follows of the experimental assays which have been performed in the framework of the invention and which provide it with the required experimental support, without limiting its scope.

### FIGURE LEGENDS

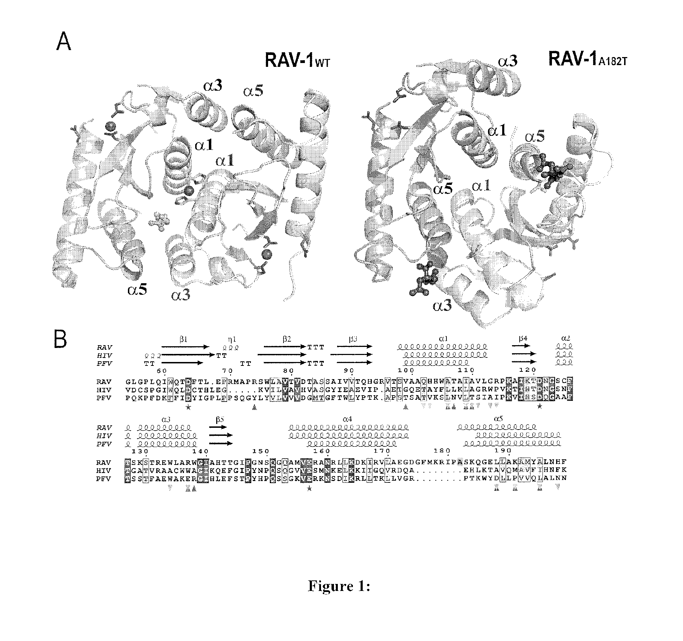

**Figure 1****: Primary, secondary and tertiary structure representations of RAV-1 IN CCD and RAV-1 IN CCD_{A182T} homodimers. (A)** Ribbon representation of RAV-1 IN CCD (left) and RAV-1 IN CCD_{A182T} (right) with monomer A in blue and monomer B in yellow. The three catalytic residues (D64, D121 and E157) are represented by red sticks, H103 by green sticks and residue 182 (Ala in RAV-1_{WT}, Thr in RAV-1_{A182T}) by cyan sticks. The MES molecule located at the interface of RAV-1 is shown in orange, while zinc ions are represented by dark spheres. Citrate molecules are coloured purple in RAV-1_{A182T}. (**B**) Multiple sequence alignment of RAV-1, HIV-1 and PFV IN CCDs. Secondary structure elements are indicated above the alignment. Identical and conserved residues according to physicochemical criteria are highlighted in red and yellow boxes, respectively. Pink and green triangles indicate residues involved in the dimeric interfaces of RAV-1 IN CCD (novel) and RAV-1 IN CCD_{A182T} (conventional), respectively. Red stars indicate the three invariant catalytic residues, while residue A182 of RAV-1 is highlighted in cyan. Figures 1A and 1B were generated with PyMOL (DeLano Scientific, http://www.pymol.org) and ESPript (55), respectively.

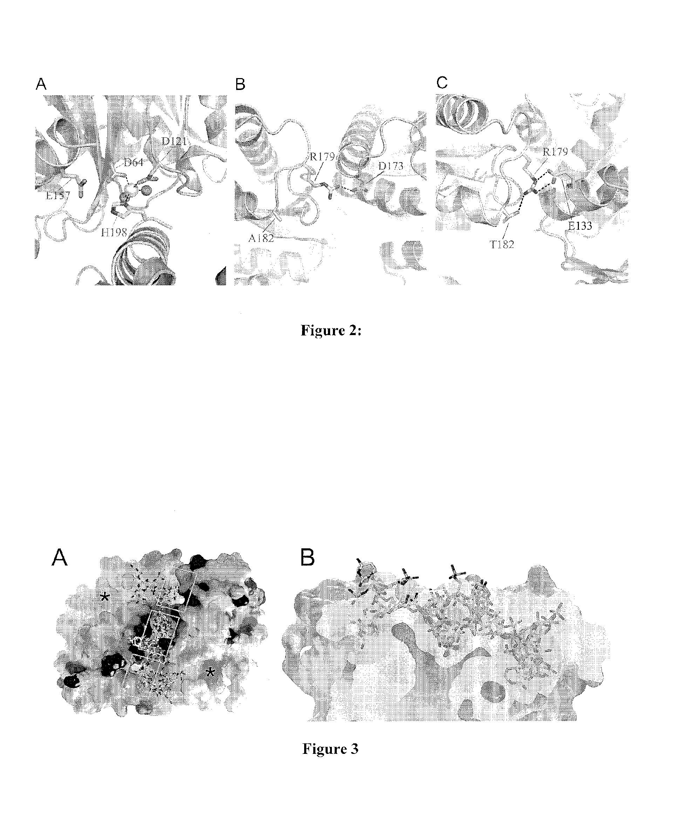

**Figure 2****: Important crystallographic contacts in RAV-1 IN CCD and RAV-1 IN CCD_{A182T}**. (**A**) A close view of the active site of RAV-1 IN CCD. The zinc ion in site I is represented by a yellow sphere. This ion is penta-coordinated by D64 and D121 (blue), H198 from a symmetry-related monomer (green) and two water molecules (red spheres). (**B**) A close view of RAV-1 IN CCD, focused on residue R179 near A182. Monomers are coloured in light blue and pale orange (symmetry-related molecule). (**C**) The counterpart region for RAV-1 IN CCD_{A182T}. The A182T mutation promotes a reorientation of R179 side-chain.
**Figure 3****: Representations of the novel RAV-1 IN CCD dimer structure in complex with a 4-base RNA fragment, as predicted by docking experiments. (A)** The molecular surface of the RAV-1 IN CCD dimer with the five top scored docking solutions (in stick representation) bound at the level of the median basic groove. Surfaces are coloured according to electrostatic potential (red negative charge; blue positive charge). The three binding pockets are boxed in yellow while locations of the two active sites are indicated by black asterisk. (**B**) A cross-section of the molecular surface of the RAV-1 IN CCD dimer along the main axis of the basic groove demonstrating the five top scored docking conformations. Cavities are coloured light grey while the three binding pockets are indicated by green circles. The electrostatic potential map and the molecular surfaces were calculated with GRASP (56).

### EXPERIMENTAL DATA

### MATERIALS AND METHODS

### Cloning the RAV-1 IN CCD sequence: pETG10a-INRAV1_{CCD}

The DNA sequence encoding the IN catalytic core domain of RAV1 (residues 53-199) was amplified by PCR from the pET30a-INRAV1 plasmid (28). The fragment was cloned using Gateway Technology (Invitrogen); the 3' *att*B PCR primer was designed with a thrombin cleavage site. pDONR223 was used as donor vector to generate an entry clone. pETG10a, containing a hexahistidine tag, was used as the expression vector. The constructed expression vector was confirmed by DNA sequencing.

### Site-directed mutagenesis

The pETG10a-INRAV1_{CCD}A182T and pETG10a-INRAV1_{CCD}H103A sequences were created by site-directed mutagenesis (Stratagene QuickChange kit) using pETG10a-INRAV1_{CCD} as a template for the following mutagenic primers (mutations are shown as lowercase letters):
5'-GTTCCCCCTGTTTGCTggtGGGGATTCTTTTCATAAAG-3' (F-A182T);
5'-CTTTATGAAAAGAATCCCCaccAGCAAACAGGGGGAAC-3' (R-A182T); and
5'-GCCGTGGCCCAATGagcTTGTGCAGCAACCG-3' (F-H103A),
5'-CGGTTGCTGCACAAgctCATTGGGCCACGGC-3' (R-H103A).

Mutations to the expression vector were confirmed by DNA sequencing. pETG10a-INRAV1_{CCD}, pETG10a-INRAV1_{CCD}A182T and pETG10a-INRAV1_{CCD}H103A were introduced into *E*. *coli* BL21 (DE3) pLysS competent cells (Novagen) for protein expression.

### Expression and purification of RAV-1 IN CCD, RAV-1 IN_CCD_{A182T} and RAV-1 IN CCD_{H103A}

An overnight culture (5 ml) from a single colony containing the desired plasmid was used to inoculate 11 fresh LB medium in the presence of ampicillin (50 µg/ml). The culture was incubated at 37 °C, and shaken at 220 rpm until an A₆₀₀ = 0.8~0.9 was reached. Overexpression of the proteins was induced by 1 mM isopropyl-β-D-thiogalactopyranoside (IPTG) overnight at 25 °C. Then, bacteria were harvested by centrifugation at 3000 × g for 10 min and stored at -80 °C. For purification, the thawed bacterial pellet was sonicated in 20 ml of buffer A (0.5 M NaCl, 10 mM imidazole, 5 mM β-mercaptoethanol, 20 mM Tris-HCl, pH 8.0) in the presence of 100 µl Halt Protease Inhibitor Cocktail (Pierce) and DNAse/RNAse (final concentration 50 µg ml⁻¹). The lysate was cleared by centrifugation (45 min, 10,000 rpm at 4 °C) and then filtered through a 0.45 µm filter. The supernatant containing soluble His-tagged proteins was loaded on an Ni²⁺ charged 1 ml HiTrap Chelating HP column (GE Healthcare) using the ÄKTA chromatography system. The column was extensively washed with buffer B (0.5 M NaCl, 20 mM imidazole, 5 mM β-mercaptoethanol, 20 mM Tris-HCl, pH 8.0) and with 5 ml Buffer B2 (1 M NaCl, 20 mM imidazole, 5 mM β-mercaptoethanol, 20 mM Tris-HCl, pH 8.0). IN proteins were eluted with 0.5 M NaCl, 500 mM imidazole, 5 mM β-mercaptoethanol, 20 mM Tris-HCl, pH 8.0 using a linear gradient. Eluted fractions were collected and analysed by SDS-PAGE. The hexahistidine tag was removed by overnight digestion with thrombin protease (Amersham Biosciences) at 4°C. Digested protein solutions were loaded on an Ni²⁺ charged 1 ml HiTrap Chelating HP column (GE Healthcare); undigested proteins and free tags were fixed on the column while digested protein was recovered in the flow through. The purity of the recovered protein was analyzed by SDS-PAGE and silver staining. Protein concentration was determined according to a Bradford assay (Bio-Rad Laboratories) using BSA as standard and then concentrated to 10 mg ml⁻¹ using a 10 kDa molecular-weight cut-off membrane (Vivascience).

### Crystallisation of RAV-1 IN CCD and RAV-1 IN CCD_{A182T}

Crystallisation conditions were searched for RAV-1 IN CCD using the sitting-drop vapour-diffusion method and commercial kits from Hampton Research, Molecular Dimensions Limited (MDL) and Qiagen. Droplets composed of 0.3 µl protein solution at 10 mg ml⁻¹ and an equal volume of crystallisation solution were equilibrated against 100 µl reservoir solution within a sealed well at 18°C. A crystal was observed for condition 24 of MDL PACT *premier* (10 mM ZnCl₂, 20% (w/v) PEG 6000, 100 mM MES, pH 6.0). Crystals reached maximum dimensions of 20 × 20 × 5 µm³ within a week. Their size was improved to 150 × 150 × 80 µm³ with a macroseeding technique using drops containing a 2 µl protein solution and a 2 µl precipitant solution. Crystals were mounted in a nylon loop and cryoprotected by adding 0.4 µl ethylene glycol to the hanging drop before flash-freezing in liquid nitrogen.

Crystals of RAV-1 IN CCD_{A182T} were obtained in hanging drops at 18 °C. by mixing 1 µl protein solution (5 mg ml⁻¹) and 1 µl reservoir solution with either the crystallisation condition of the wild-type or a crystallisation condition for RSV IN CCD (20% (w/v) PEG 4000, 10% isopropanol and 0.1 M Na citrate pH 6.2). Microcrystals were obtained under the first set of conditions and large crystals with maximum dimensions of 200 × 150 × 150 µm³ were obtained under the second set of conditions.

### Data collection and structure determination

A synchrotron data set for RAV-1 IN CCD was collected to 1.8 Å resolution from a crystal cooled to 100 K at ESRF beamline ID29 (Grenoble, France) at a wavelength of 1.281060 Å. Data were processed with XDS/XSCALE (29). The phase problem was solved by molecular replacement using the program AMoRe (30). The final crystal structure, with two polypeptide chains named A and B, was obtained by alternating cycles of restrained refinement in REFMAC5 (31) and manual rebuilding in COOT (32). WHATCHECK (33) was used to assess the geometric quality of the model (94.2 % of the residues in the most favoured region of the Ramachandran plot). A second synchrotron data set was collected to 1.55 Å resolution at the ESRF beamline BM30A at a wavelength of 0.979680 Å, under cryo-conditions (100 K), from a crystal of RAV-1 IN CCD_{A182T}. The phase problem was solved by rigid-body refinement with REFMAC5 prior to restrained refinement. Additional data collection and refinement statistics are presented in **Table 1** for both structures.

**Table 1: Data collection and refinement statistics.**

| | **RAV1IN_CCD** | **RAV1 IN_CCD_{A182T}** |
|---|---|---|
| **Data collection** | | |
| Space group | P6₁ | P4₃2₁2 |
| Cell dimensions | | |
| a, b, c (Å) | 107.5, 107.5, 50.9 | 65.5, 65.5, 79.2 |
| a, β, γ (°) | 90, 90, 120 | 90, 90, 90 |
| Vₘ (Å³.Da⁻¹) | 2.6 | 2.8 |
| Solvent content (%) | 52 | 55 |
| Resolution range (Å) | 19.7-1.8 (1.95₋1.8) | 1.55) 20.0-1.55 (1.60- |
| Total number of reflections | 162065 (33802) | 111679 (7650) |
| Number of unique | 30860 (6613) | 24970 (2171) |
| reflections | | |
| R_{sym}(%) | 7.6 (39.9) | 4.9 (25.6) |
| I/σ(I) | 11.6 (3.4) | 20.9 (5.4) |
| Completeness (%) | 99.0 (99.0) | 97.4 (95.3) |
| Redundancy | 5.3 (5.1) | 4.5 (3.5) |
| | | |
| **Refinement** | | |
| Resolution (Å) | 1.8 | 1.55 |
| R_{factor} / R_{free} (%) | 19.3/22.8 | 16.4/21.1 |
| No. atoms | | |
| Protein (non- | 2099 | 1079 |
| hydrogen) | | |
| Ligand/ion | 15 | 13 |
| Water | 186 | 122 |
| Overall *B*-factor (Å²) | 31.7 | 15.2 |
| R.m.s. deviations | | |
| Bond lengths (Å) | 0.020 | 0.026 |
| Bond angles (°) | 2.34 | 1.72 |

| | | |
|---|---|---|
| Values in parentheses are for highest-resolution shell. | | |

### Docking experiments

A single-stranded RNA molecule comprising four bases was designed. The number of bases employed was determined by a computational limit to the number of rotatable bonds allowed. The 1.1 Å atomic resolution crystal structure of the DNA octanucleotide d(pATTCATTC) was used as the template (PDB entry 284D). We truncated and modified this structure to obtain our final RNA fragment, pCAUUp. This ligand and the receptor structure of RAV-1 IN CCD were then prepared with AUTODOCKTOOLS (34). A "blind docking" was subsequently carried out on the entire surface of the dimer with the program AUTODOCK VINA (35). Once a binding area was identified, new docking cycles were achieved with a reduced search space encompassing the site of interest. Flexible-ligand docking with grid-based energy scoring was conducted with the program's standard protocol (35).

### RESULTS

### Structure determination and refinement

The CCD of RAV-1 IN, consisting of residues 54-199, was expressed in *Escherichia coli* and purified as described in the 'Methods' section. This fragment differs by a single residue from the CCD of RSV IN (A182T substitution), for which numerous crystal structures have been solved (15,16,36-38). Crystallisation conditions similar to those published for the CCD of RSV IN that is, citrate buffer at an acidic pH and HEPES buffer at an alkaline pH (38), were tried, but this approach proved unsuccessful. Hence, a broad screening of conditions was performed. Crystals were obtained in the presence of Zn²⁺ and MES (2-(N-morpholino)ethanesulphonic acid) at pH 6.0. They belonged to the hexagonal space group P6₁ and contained two molecules in the asymmetric unit. Synchrotron data were collected to 1.8 Å resolution near the Zn-K absorption edge. The phase problem was solved by molecular replacement using the monomer of RSV IN CCD (PDB entry 1VSD) structure as the search model. After a few cycles of crystallographic refinement alternated with manual rebuilding, the final crystal structure was obtained with an R value of 19.3% (R_{free} 22.8%). The structure contains 271 amino acids in two monomers termed A and B, 186 water molecules, three Zn²⁺ ions and one MES molecule (Figure 1A, left). The N-terminal ends 54-57 of the two monomers, as well as the loops formed by 145A-152A (monomer A) and 145B-149B (monomer B) were not observed in electron density maps and are not included in the model. These loops are often disordered in retroviral INs (39). The three coordinated Zn²⁺ correspond to the highest peaks in the calculated anomalous difference Fourier map (29σ to 33σ).

As a control, the A182T mutant of RAV-1 IN CCD (termed RAV-1 IN CCD_{A182T}), corresponding to the RSV IN CCD sequence, was purified and the crystallisation conditions for both RAV-1 IN CCD and RSV IN CCD were tested; that is, a MES buffer at pH 6.0 and a citrate buffer at pH 6.2, respectively. Microcrystals were observed with the first set of conditions, but were too small to give measurable Bragg peaks. Large crystals were obtained with the second set of conditions. Further, the huge crystals of RAV-1 IN CCD_{A182T} were isomorphous to those of RSV IN CCD obtained under the same conditions. They belonged to space group P4₃2₁2 with one molecule in the asymmetric unit. Synchrotron data for these crystals were collected to 1.55 Å resolution. The phase problem was solved by a simple rigid-body refinement followed by restrained refinement using the structure of RSV IN CCD as the starting model. The refined structure of RAV-1 IN CCD_{A182T} contains 137 residues, 1 citrate molecule and 122 water molecules (Figure 1A, left). The 145-152 loop is disordered and is not observed in the electron density map, as in RSV IN CCD.

### Overall structure of RAV-1 IN_CCD

The 1.8 Å crystal structure of RAV-1 IN CCD consists of two similar polypeptide chains, termed A and B. The two monomers can be superimposed with an r.m.s. deviation of 0.4 Å on 132 Cα pairs after a 180° rotation. The main differences between the two Cα traces are due to crystal contacts. The differences arise at residues 174-176, located in a turn between helices α4 and α5 (0.8-1.1 Å between Cα pairs), and at residues 198-199 at the C-terminal end (3-8 Å between Cα pairs). In the latter case, the short C-terminal loop following helix α5 folds back toward the protein core to cap a MES molecule in molecule A, whereas it protrudes into the solvent in molecule B. The non-crystallographic A/B homodimer is compact and approximates a globular ellipsoid with dimensions of 55 × 40 × 40 Å (Figure 1A). Thus, the tertiary structure of RAV-1 IN CCD is nearly identical to that of RSV IN CCD, equivalent to that of RAV1 IN CCD_{A182T} (r.m.s. deviation of 0.4 Å on 132 Cα pairs) and respects the ribonuclease H fold (Figure 1A). The A182T substitution does not affect the tertiary structure of avian INs.

### The active site of RAV-1 IN CCD

The invariant acidic residues of the catalytic triad (Asp-64, Asp-121 and Glu-157 in RAV-1 IN) were accurately orientated in the electron density map. They form a pocket at the surface of the RAV-1 IN CCD monomer, which is located 15 Å away from the A/B dimeric interface. The carboxylate group of Asp-64 is situated at the centre of the triad and interacts with Asp-121 through a Zn²⁺ ion from the crystallisation solution. This ion occupies the divalent cation-binding site termed site I in retroviral INs. It also coordinates two water molecules and the imidazole group of His-198, which belongs to a symmetry-related molecule (Figure 2A). Such a penta-coordinated metal ion has never been observed in the active site of INs before. The side chain of Glu-157 points freely towards the solvent, as observed in the structure of RSV IN CCD in complex with one Mg²⁺ coordinated at site I (PDB entry 1VSD). This residue rotates when accommodating the second catalytic Zn²⁺ in site II (16). Interestingly, the present active site of RAV-1 IN with its coordinated histidine is very similar to that of influenza virus polymerase, which was solved recently (40). In this structure, acidic and basic catalytic residues coordinate two divalent cations, which are very likely to be responsible for the endonuclease activity of the whole protein. A further structural comparison was performed, with the active site of full-length PFV IN in complex with Mg²⁺ and raltegravir, an antiretroviral drug that targets the catalytic site of INs (PDB entry 3L2T). It appeared that the coordinated side chain of the symmetry-related His-198 occupies the position of two chelating oxygen atoms linked to the pyrimidine group of the IN inhibitor. The same observation was made in a comparison with PFV IN complexed with Mg²⁺ and elvitegravir, another antiretroviral molecule (PDB entry 3L2U). Again, the IN inhibitors occupy the position devoted to His-198 that mimics the coordinated nucleotide in the crystal structure of RAV-1 IN CCD.

### A new dimeric assembly

Of extreme interest, the conventional dimeric interface of RSV IN CCD (equivalent to RAV-1 IN CCD_{A182T}) which generally involves two pairs of facing α-helices of each monomer (pairs α1/α5; Figure 1A, right), is not visible in the crystal packing of RAV-1 IN CCD. The present A/B dimer buries three pairs of facing helices (α3A/α5B, α1A/α1B, α3B/α5A; Figure 1A, left) in a new intermolecular interface, which can be obtained from the crystallographic dimer of RSV IN CCD by a 15 Å translation of one monomer along the other. Thus, helix α5 faces helix α1 of the complementary monomer in RSV IN CCD, and helix α3 of the complementary monomer in RAV-1 IN CCD. Moreover, helices α1 of monomers A and B now run almost parallel to the non-crystallographic two-fold axis and are locked together via a buried Zn²⁺ that coordinates the imidazole rings of His-103A and His-103B (Figure 1A). This central Zn²⁺ also coordinates two water molecules in a perfectly tetrahedral coordination sphere. The area of the buried surface at the new CCD/CCD interface, 740 Å² per monomer, is similar to that previously observed in RSV IN; that is, 750 Å² per monomer. The distance between the active sites of the two CCDs is preserved (35 Å), as is the distance between the two CCD N-termini (25 Å), while the distance between the two CCD C-termini increases significantly (from 20 Å to 35 Å). The novel CCD-CCD interface is identified as a standard molecular interface by the PISA web server (41). It buries an equal number of polar and non-polar residues (Supplementary Table 1) and more than 50% of contacting residues are preserved between RAV-1 IN CCD and RSV IN CCD (Figure 1B). For example, the ion pair between His-103 (helix α1) and Glu-187 (helix α5) of the complementary monomer that was highlighted in RSV IN CCD (42) is substituted by an equivalent intermolecular contact between Arg-137 (helix α3) and the same Glu-187 (helix α5) in RAV-1 IN CCD.

Surprisingly, the mismatched residue Ala/Thr-182 is not buried in any dimeric interface. This residue is located on the outer edge of helix α5, a portion of which is accessible to solvent in both the RSV IN CCD and RAV-1 IN CCD crystals. The A182T substitution mostly affects the side-chain orientation of the neighboring Arg-179 of the α4-α5 loop. This arginine is hydrogen bonded to the side-chain oxygen atom OG of Thr-182 in RSV IN CCD (Figure 2C), whereas a similar contact is impossible with the aliphatic Ala-182 in RAV-1 IN CCD. There, the side chain of the arginine has rotated by 130° around its CG-CD bond to mediate a crystal contact with the side-chain carboxylate in the Asp-173 of a neighboring monomer (Figure 2B). Thus, the A182T substitution influences crystal assembly via Arg-179 and, either large hexagonal crystals or tiny tetragonal microcrystals are observed for RAV-1 IN CCD_{A182T}, whereas only tetragonal crystals are obtained for RAV-1 IN CCD.

### A buried MES molecule at the dimeric interface

The novel dimeric interface buries a MES molecule (Figure 1A, left) in a canal located between helix α5 of molecule A and helix α3 of molecule B. The O1 oxygen atom of the morpholino group is oriented toward the bulk solvent, while the sulphonate group penetrates deeply into the interface. The morpholino group is further cradled by hydrophobic interactions with Tyr-194A, Phe-199A and Trp-138B. Its N4 nitrogen atom establishes a direct hydrogen bond with the hydroxyl group of Tyr-194A, while its sulphonate group is stabilized by the guanidinium group of Arg-137B. In comparison, a bound HEPES molecule is observed in the alkaline structure of RSV IN_CCD, whereas a bound citrate is observed in the acidic structure of the same fragment (38) and in the equivalent RAV-1 IN CCD_{A182T}. However, neither of these two buffer molecules is involved in the conventional interface of RSV IN CCD. The HEPES molecule, which resembles MES in its sulphonate group and a six-atom cycle, is lodged at the CCD surface along the tips of loops β1β2 and β3α1, while the citrate molecule caps the N-terminal extremity of helix α5 (Figure 1A, right).

### A novel median basic groove

As described previously by Bujacz *et al.* (1995), the conventional dimeric interface of RSV IN CCD contains a central cavity bordered by hydrophilic residues. This cavity is conserved in HIV IN and has been investigated as a target for allosteric inhibitors (43). A projection of molecular electrostatic potentials shows that this central invagination becomes a highly basic median groove in RAV1 IN CCD and strips the protein surface of the dimeric interface away from the catalytic sites (Figure 3). Inspection of the narrow groove basement reveals three small pockets arranged at regular intervals so as to accommodate a linear single-stranded nucleic acid. The distance between the central and outer pockets is 10 Å. Residues His-103A and His-103B are bridged by the central Zn²⁺ and constitute the bottom of the middle pocket.

A "blind docking" experiment was performed with a single-stranded RNA aptamer against the entire surface of our RAV-1 IN CCD dimer. All of the highest score solutions correspond to RNA fragments bound at the level of the median basic groove. According to these predictions, heterocycles of purine and pyrimidine bases could fit in the three bottom pockets of the groove with the phosphodiester chain of the RNA exposed to the solvent. The central pocket can alternatively bind to a phosphate group of the RNA backbone. In this case, the backbone adopts a linear twisted conformation, made possible by the high degree of flexibility of the chain.

### Atomic coordinates

Atomic coordinates and structure factors are as listed in the following Table:

**Table 2**

| | | | | | | | | | | | | |
|---|---|---|---|---|---|---|---|---|---|---|---|---|
| HEADER | DNA BINDING PROTEIN | | | | | | | | 27-JUL-10 3O4N | | | |
| TITLE | CRYSTAL STRUCTURE OF THE CATALYTIC CORE DOMAIN OF THE AVIAN LEUKEMIA | | | | | | | | | | | |
| TITLE | 2 AND SARCOMA VIRUS INTEGRASE WITH A NEW DIMERIC ASSOCIATION | | | | | | | | | | | |
| COMPND | MOL_ID: 1 | | | | | | | | | | | |
| COMPND | 2 MOLECULE: INTEGRASE | | | | | | | | | | | |
| COMPND | 3 CHAIN: A, B | | | | | | | | | | | |
| COMPND | 4 FRAGMENT: INRAV1 CCD, RESIDUES 625-771 | | | | | | | | | | | |
| COMPND | 5 ENGINEERED: YES | | | | | | | | | | | |
| SOURCE | MOL_ID: 1 | | | | | | | | | | | |
| SOURCE | 2 ORGANISM_SCIENTIFIC: ROUS SARCOMA VIRUS | | | | | | | | | | | |
| SOURCE | 3 ORGANISM_TAXID: 11886 | | | | | | | | | | | |
| SOURCE | 4 GENE: POL | | | | | | | | | | | |
| SOURCE | 5 EXPRESSION_SYSTEM: ESCHERICHIA COLI | | | | | | | | | | | |
| SOURCE | 6 EXPRESSION_SYSTEM_TAXID: 469008 | | | | | | | | | | | |
| SOURCE | 7 EXPRESSION_SYSTEM_STRAIN: BL21(DE3) PLYSS | | | | | | | | | | | |
| SOURCE | 8 EXPRESSION_SYSTEM_VECTOR_TYPE: PLASMID | | | | | | | | | | | |
| SOURCE | 9 EXPRESSION_SYSTEM_PLASMID: PETG10A | | | | | | | | | | | |
| KEYWDS | DNA INTEGRATION PROCESS, DNA BINDING PROTEIN | | | | | | | | | | | |
| EXPDTA | X-RAY DIFFRACTION | | | | | | | | | | | |
| AUTHOR | A.BALLANDRAS, X. ROBERT, P. GOUET | | | | | | | | | | | |
| JRNL | | AUTH A.BALLANDRAS, K.MOREAU, X.ROBERT, R.HASER, C.RONFORT, P.GOUET | | | | | | | | | | |
| JRNL | | TITL THE CRYSTAL STRUCTURE OF THE CATALYTIC CORE DOMAIN OF AN | | | | | | | | | | |
| JRNL | | TITL 2 AVIAN LEUKEMIA AND SARCOMA VIRUS INTEGRASE REVEALS A NEW | | | | | | | | | | |
| JRNL | | TITL 3 DIMERIC ASSOCIATION | | | | | | | | | | |
| JRNL | | REF TO BE PUBLISHED | | | | | | | | | | |
| JRNL | | REFN | | | | | | | | | | |
| REMARK | 2 | | | | | | | | | | | |
| | | | | | | | | | | | | |

| REMARK | 2 RESOLUTION. | | | | | 1.80 | ANGSTROMS. | | | | | |
|---|---|---|---|---|---|---|---|---|---|---|---|---|
| ATO+°M | 1 | N | GLN | A | 59 | | 38.483 | 60.607 | -14.050 | 1.00 | 36.80 | N |
| ATOM | 2 | CA | GLN | A | 59 | | 39.248 | 61.788 | -13.534 | 1.00 | 35.11 | C |
| ATOM | 3 | C | GLN | A | 59 | | 40.685 | 61.338 | -13.177 | 1.00 | 34.66 | C |
| ATOM | 4 | O | GLN | A | 59 | | 40.858 | 60.177 | -12.816 | 1.00 | 33.69 | O |
| ATOM | 5 | CB | GLN | A | 59 | | 38.593 | 62.218 | -12.198 | 1.00 | 36.56 | C |
| ATOM | 6 | CG | GLN | A | 59 | | 37.202 | 62.800 | -12.291 | 1.00 | 38.06 | C |
| ATOM | 7 | CD | GLN | A | 59 | | 36.590 | 63.098 | -10.905 | 1.00 | 42.60 | C |
| ATOM | 8 | OE1 | GLN | A | 59 | | 37.124 | 63.866 | -10.088 | 1.00 | 39.93 | O |
| ATOM | 9 | NE2 | GLN | A | 59 | | 35.439 | 62.500 | -10.661 | 1.00 | 40.79 | N |
| ATOM | 10 | N | ILE | A | 60 | | 41.667 | 62.250 | -13.265 | 1.00 | 32.29 | N |
| ATOM | 11 | CA | ILE | A | 60 | | 43.067 | 62.052 | -12.826 | 1.00 | 29.86 | C |
| ATOM | 12 | C | ILE | A | 60 | | 43.255 | 63.104 | -11.754 | 1.00 | 27.12 | C |
| ATOM | 13 | O | ILE | A | 60 | | 43.077 | 64.290 | -12.006 | 1.00 | 26.07 | O |
| ATOM | 14 | CB | ILE | A | 60 | | 44.102 | 62.283 | -13.950 | 1.00 | 31.37 | C |
| ATOM | 15 | CG1 | ILE | A | 60 | | 43.914 | 61.238 | -15.079 | 1.00 | 35.50 | C |
| ATOM | 16 | CG2 | ILE | A | 60 | | 45.536 | 62.236 | -13.353 | 1.00 | 30.95 | C |
| ATOM | 17 | CD1 | ILE | A | 60 | | 45.029 | 61.424 | -16.103 | 1.00 | 38.43 | C |
| ATOM | 18 | N | TRP | A | 61 | | 43.561 | 62.655 | -10.536 | 1.00 | 23.77 | N |
| ATOM | 19 | CA | TRP | A | 61 | | 43.749 | 63.627 | -9.439 | 1.00 | 22.97 | C |
| ATOM | 20 | C | TRP | A | 61 | | 45.236 | 63.723 | -9.075 | 1.00 | 22.27 | C |
| ATOM | 21 | O | TRP | A | 61 | | 45.970 | 62.799 | -9.374 | 1.00 | 22.72 | O |
| ATOM | 22 | CB | TRP | A | 61 | | 43.105 | 63.047 | -8.156 | 1.00 | 23.11 | C |
| ATOM | 23 | CG | TRP | A | 61 | | 41.630 | 63.016 | -8.181 | 1.00 | 24.03 | C |
| ATOM | 24 | CD1 | TRP | A | 61 | | 40.794 | 63.501 | -9.195 | 1.00 | 26.28 | C |
| ATOM | 25 | CD2 | TRP | A | 61 | | 40.776 | 62.471 | -7.151 | 1.00 | 22.74 | C |
| ATOM | 26 | NE1 | TRP | A | 61 | | 39.482 | 63.272 | -8.818 | 1.00 | 26.57 | N |
| ATOM | 27 | CE2 | TRP | A | 61 | | 39.442 | 62.646 | -7.587 | 1.00 | 27.24 | C |
| ATOM | 28 | CE3 | TRP | A | 61 | | 41.039 | 61.832 | -5.914 | 1.00 | 24.46 | C |
| ATOM | 29 | CZ2 | TRP | A | 61 | | 38.330 | 62.222 | -6.830 | 1.00 | 25.03 | C |
| ATOM | 30 | CZ3 | TRP | A | 61 | | 39.931 | 61.381 | -5.152 | 1.00 | 25.53 | C |
| ATOM | 31 | CH2 | TRP | A | 61 | | 38.587 | 61.593 | -5.613 | 1.00 | 26.45 | C |
| ATOM | 32 | N | GLN | A | 62 | | 45.646 | 64.837 | -8.461 | 1.00 | 21.79 | N |
| ATOM | 33 | CA | GLN | A | 62 | | 46.936 | 64.881 | -7.777 | 1.00 | 22.90 | C |
| ATOM | 34 | C | GLN | A | 62 | | 46.691 | 65.099 | -6.286 | 1.00 | 20.58 | C |
| ATOM | 35 | O | GLN | A | 62 | | 45.815 | 65.940 | -5.963 | 1.00 | 22.50 | O |
| ATOM | 36 | CB | GLN | A | 62 | | 47.701 | 66.085 | -8.316 | 1.00 | 24.06 | C |
| ATOM | 37 | CG | GLN | A | 62 | | 48.249 | 65.855 | -9.719 | 1.00 | 32.75 | C |
| ATOM | 38 | CD | GLN | A | 62 | | 49.672 | 65.242 | -9.662 | 1.00 | 38.33 | C |
| ATOM | 39 | OE1 | GLN | A | 62 | | 50.330 | 65.163 | -8.616 | 1.00 | 49.63 | O |
| ATOM | 40 | NE2 | GLN | A | 62 | | 50.144 | 64.828 | -10.795 | 1.00 | 45.80 | N |
| ATOM | 41 | N | THR | A | 63 | | 47.420 | 64.371 | -5.424 | 1.00 | 19.29 | N |
| ATOM | 42 | CA | THR | A | 63 | | 47.156 | 64.569 | -3.980 | 1.00 | 19.75 | C |
| ATOM | 43 | C | THR | A | 63 | | 48.509 | 64.841 | -3.279 | 1.00 | 20.04 | C |
| ATOM | 44 | O | THR | A | 63 | | 49.522 | 64.230 | -3.671 | 1.00 | 21.78 | O |
| ATOM | 45 | CB | THR | A | 63 | | 46.559 | 63.251 | -3.378 | 1.00 | 19.97 | C |
| ATOM | 46 | OG1 | THR | A | 63 | | 45.250 | 63.084 | -3.971 | 1.00 | 20.52 | O |
| ATOM | 47 | CG2 | THR | A | 63 | | 46.328 | 63.334 | -1.880 | 1.00 | 19.17 | C |
| ATOM | 48 | N | ASP | A | 64 | | 48.501 | 65.676 | -2.231 | 1.00 | 19.36 | N |
| ATOM | 49 | CA | ASP | A | 64 | | 49.738 | 65.792 | -1.407 | 1.00 | 20.50 | C |
| ATOM | 50 | C | ASP | A | 64 | | 49.345 | 66.268 | -0.022 | 1.00 | 19.38 | C |
| ATOM | 51 | O | ASP | A | 64 | | 48.210 | 66.714 | 0.235 | 1.00 | 19.94 | O |
| ATOM | 52 | CB | ASP | A | 64 | | 50.659 | 66.825 | -2.053 | 1.00 | 20.08 | C |
| ATOM | 53 | CG | ASP | A | 64 | | 52.113 | 66.740 | -1.540 | 1.00 | 20.74 | C |
| ATOM | 54 | OD1 | ASP | A | 64 | | 52.383 | 65.699 | -0.986 | 1.00 | 22.49 | O |
| ATOM | 55 | OD2 | ASP | A | 64 | | 52.914 | 67.645 | -1.762 | 1.00 | 26.57 | O |
| ATOM | 56 | N | PHE | A | 65 | | 50.273 | 66.220 | 0.926 | 1.00 | 19.70 | N |
| ATOM | 57 | CA | PHE | A | 65 | | 50.005 | 66.765 | 2.249 | 1.00 | 20.17 | C |
| ATOM | 58 | C | PHE | A | 65 | | 50.778 | 68.088 | 2.410 | 1.00 | 20.59 | C |
| ATOM | 59 | O | PHE | A | 65 | | 51.861 | 68.241 | 1.806 | 1.00 | 21.92 | O |
| ATOM | 60 | CB | PHE | A | 65 | | 50.540 | 65.829 | 3.377 | 1.00 | 19.36 | C |
| ATOM | 61 | CG | PHE | A | 65 | | 49.566 | 64.752 | 3.776 | 1.00 | 24.10 | C |
| ATOM | 62 | CD1 | PHE | A | 65 | | 48.596 | 65.011 | 4.750 | 1.00 | 22.79 | C |
| ATOM | 63 | CD2 | PHE | A | 65 | | 49.630 | 63.495 | 3.153 | 1.00 | 27.72 | C |
| ATOM | 64 | CE1 | PHE | A | 65 | | 47.709 | 63.996 | 5.153 | 1.00 | 28.29 | C |
| ATOM | 65 | CE2 | PHE | A | 65 | | 48.717 | 62.472 | 3.557 | 1.00 | 29.47 | C |
| ATOM | 66 | CZ | PHE | A | 65 | | 47.770 | 62.770 | 4.533 | 1.00 | 26.48 | C |
| ATOM | 67 | N | THR | A | 66 | | 50.232 | 68.985 | 3.228 | 1.00 | 20.08 | N |
| ATOM | 68 | CA | THR | A | 66 | | 50.900 | 70.271 | 3.554 | 1.00 | 20.68 | C |
| ATOM | 69 | C | THR | A | 66 | | 50.572 | 70.638 | 5.026 | 1.00 | 22.10 | C |
| ATOM | 70 | O | THR | A | 66 | | 49.526 | 70.272 | 5.560 | 1.00 | 21.03 | O |
| ATOM | 71 | CB | THR | A | 66 | | 50.556 | 71.414 | 2.559 | 1.00 | 22.91 | C |
| ATOM | 72 | OG1 | THR | A | 66 | | 51.414 | 72.547 | 2.819 | 1.00 | 25.14 | O |
| ATOM | 73 | CG2 | THR | A | 66 | | 49.145 | 71.878 | 2.782 | 1.00 | 24.28 | C |
| ATOM | 74 | N | LEU | A | 67 | | 51.434 | 71.397 | 5.710 | 1.00 | 20.12 | N |
| ATOM | 75 | CA | LEU | A | 67 | | 51.144 | 71.743 | 7.118 | 1.00 | 19.35 | C |
| ATOM | 76 | C | LEU | A | 67 | | 50.506 | 73.164 | 7.236 | 1.00 | 21.42 | C |
| ATOM | 77 | O | LEU | A | 67 | | 50.967 | 74.139 | 6.585 | 1.00 | 24.22 | O |
| ATOM | 78 | CB | LEU | A | 67 | | 52.489 | 71.763 | 7.939 | 1.00 | 20.09 | C |
| ATOM | 79 | CG | LEU | A | 67 | | 52.237 | 71.885 | 9.486 | 1.00 | 23.59 | C |
| ATOM | 80 | CD1 | LEU | A | 67 | | 51.739 | 70.566 | 9.981 | 1.00 | 26.67 | C |
| ATOM | 81 | CD2 | LEU | A | 67 | | 53.589 | 72.273 | 10.207 | 1.00 | 26.54 | C |
| ATOM | 82 | N | GLU | A | 68 | | 49.474 | 73.291 | 8.052 | 1.00 | 21.15 | N |
| ATOM | 83 | CA | GLU | A | 68 | | 48.834 | 74.600 | 8.320 | 1.00 | 21.46 | C |
| ATOM | 84 | C | GLU | A | 68 | | 48.742 | 74.764 | 9.820 | 1.00 | 22.35 | C |
| ATOM | 85 | O | GLU | A | 68 | | 47.797 | 74.299 | 10.488 | 1.00 | 21.21 | O |
| ATOM | 86 | CB | GLU | A | 68 | | 47.486 | 74.734 | 7.595 | 1.00 | 20.59 | C |
| ATOM | 87 | CG | GLU | A | 68 | | 46.739 | 76.055 | 7.988 | 1.00 | 23.47 | C |
| ATOM | 88 | CD | GLU | A | 68 | | 47.688 | 77.230 | 7.937 | 1.00 | 28.15 | C |
| ATOM | 89 | OE1 | GLU | A | 68 | | 47.920 | 77.705 | 6.813 | 1.00 | 35.81 | O |
| ATOM | 90 | OE2 | GLU | A | 68 | | 48.265 | 77.702 | 8.962 | 1.00 | 26.64 | O |
| ATOM | 91 | N | PRO | A | 69 | | 49.771 | 75.386 | 10.391 | 1.00 | 23.16 | N |
| ATOM | 92 | CA | PRO | A | 69 | | 49.742 | 75.461 | 11.838 | 1.00 | 24.75 | C |
| ATOM | 93 | C | PRO | A | 69 | | 48.556 | 76.225 | 12.450 | 1.00 | 23.35 | C |
| ATOM | 94 | O | PRO | A | 69 | | 48.250 | 76.040 | 13.650 | 1.00 | 24.50 | O |
| ATOM | 95 | CB | PRO | A | 69 | | 51.042 | 76.207 | 12.136 | 1.00 | 25.36 | C |
| ATOM | 96 | CG | PRO | A | 69 | | 51.944 | 75.907 | 11.058 | 1.00 | 26.96 | C |
| ATOM | 97 | CD | PRO | A | 69 | | 51.041 | 75.887 | 9.816 | 1.00 | 24.41 | C |
| ATOM | 98 | N | ARG | A | 70 | | 47.841 | 77.062 | 11.682 | 1.00 | 23.32 | N |
| ATOM | 99 | CA | ARG | A | 70 | | 46.659 | 77.707 | 12.288 | 1.00 | 23.19 | C |
| ATOM | 100 | C | ARG | A | 70 | | 45.560 | 76.686 | 12.649 | 1.00 | 23.89 | C |
| ATOM | 101 | O | ARG | A | 70 | | 44.716 | 76.972 | 13.514 | 1.00 | 23.26 | O |
| ATOM | 102 | CB | ARG | A | 70 | | 46.007 | 78.700 | 11.308 | 1.00 | 23.51 | C |
| ATOM | 103 | CG | ARG | A | 70 | | 46.925 | 79.926 | 11.047 | 1.00 | 24.99 | C |
| ATOM | 104 | CD | ARG | A | 70 | | 46.307 | 80.751 | 9.930 | 1.00 | 29.10 | C |
| ATOM | 105 | NE | ARG | A | 70 | | 47.340 | 81.639 | 9.351 | 1.00 | 35.73 | N |
| ATOM | 106 | CZ | ARG | A | 70 | | 47.706 | 82.806 | 9.848 | 1.00 | 35.05 | C |
| ATOM | 107 | NH1 | ARG | A | 70 | | 47.112 | 83.275 | 10.942 | 1.00 | 35.89 | N |
| ATOM | 108 | NH2 | ARG | A | 70 | | 48.682 | 83.497 | 9.239 | 1.00 | 34.80 | N |
| ATOM | 109 | N | MET | A | 71 | | 45.670 | 75.508 | 12.012 | 1.00 | 22.67 | N |
| ATOM | 110 | CA | MET | A | 71 | | 44.662 | 74.429 | 12.250 | 1.00 | 23.59 | C |
| ATOM | 111 | C | MET | A | 71 | | 45.092 | 73.385 | 13.277 | 1.00 | 23.52 | C |
| ATOM | 112 | O | MET | A | 71 | | 44.459 | 72.335 | 13.366 | 1.00 | 22.16 | O |
| ATOM | 113 | CB | MET | A | 71 | | 44.392 | 73.727 | 10.899 | 1.00 | 24.22 | C |
| ATOM | 114 | CG | MET | A | 71 | | 43.615 | 74.648 | 9.979 | 1.00 | 25.73 | C |
| ATOM | 115 | SD | MET | A | 71 | | 41.823 | 74.537 | 10.338 | 1.00 | 31.02 | S |
| ATOM | 116 | CE | MET | A | 71 | | 41.149 | 75.840 | 9.276 | 1.00 | 32.85 | C |
| ATOM | 117 | N | ALA | A | 72 | | 46.170 | 73.669 | 14.027 | 1.00 | 23.66 | N |
| ATOM | 118 | CA | ALA | A | 72 | | 46.641 | 72.789 | 15.095 | 1.00 | 23.99 | C |
| ATOM | 119 | C | ALA | A | 72 | | 45.575 | 72.619 | 16.173 | 1.00 | 24.10 | C |
| ATOM | 120 | O | ALA | A | 72 | | 44.747 | 73.517 | 16.404 | 1.00 | 24.87 | O |
| ATOM | 121 | CB | ALA | A | 72 | | 47.911 | 73.425 | 15.773 | 1.00 | 25.29 | C |
| ATOM | 122 | N | PRO | A | 73 | | 45.565 | 71.451 | 16.843 | 1.00 | 25.26 | N |
| ATOM | 123 | CA | PRO | A | 73 | | 46.438 | 70.324 | 16.711 | 1.00 | 25.28 | C |
| ATOM | 124 | C | PRO | A | 73 | | 46.311 | 69.452 | 15.433 | 1.00 | 25.87 | C |
| ATOM | 125 | O | PRO | A | 73 | | 47.219 | 68.665 | 15.156 | 1.00 | 25.19 | O |
| ATOM | 126 | CB | PRO | A | 73 | | 46.097 | 69.491 | 17.959 | 1.00 | 27.14 | C |
| ATOM | 127 | CG | PRO | A | 73 | | 44.695 | 69.742 | 18.226 | 1.00 | 26.51 | C |
| ATOM | 128 | CD | PRO | A | 73 | | 44.541 | 71.232 | 17.882 | 1.00 | 27.26 | C |
| ATOM | 129 | N | ARG | A | 74 | | 45.201 | 69.545 | 14.711 | 1.00 | 23.88 | N |
| ATOM | 130 | CA | ARG | A | 74 | | 45.002 | 68.767 | 13.467 | 1.00 | 22.93 | C |
| ATOM | 131 | C | ARG | A | 74 | | 45.521 | 69.608 | 12.278 | 1.00 | 21.84 | C |
| ATOM | 132 | O | ARG | A | 74 | | 44.765 | 70.000 | 11.372 | 1.00 | 21.08 | O |
| ATOM | 133 | CB | ARG | A | 74 | | 43.527 | 68.355 | 13.287 | 1.00 | 22.68 | C |
| ATOM | 134 | CG | ARG | A | 74 | | 43.157 | 67.306 | 14.389 | 1.00 | 25.22 | C |
| ATOM | 135 | CD | ARG | A | 74 | | 41.639 | 67.181 | 14.491 | 1.00 | 26.42 | C |
| ATOM | 136 | NE | ARG | A | 74 | | 41.152 | 66.476 | 13.292 | 1.00 | 25.28 | N |
| ATOM | 137 | CZ | ARG | A | 74 | | 40.037 | 66.736 | 12.605 | 1.00 | 26.18 | C |
| ATOM | 138 | NH1 | ARG | A | 74 | | 39.223 | 67.755 | 12.871 | 1.00 | 26.69 | N |
| ATOM | 139 | NH2 | ARG | A | 74 | | 39.754 | 65.947 | 11.576 | 1.00 | 24.41 | N |
| ATOM | 140 | N | SER | A | 75 | | 46.830 | 69.845 | 12.236 | 1.00 | 21.11 | N |
| ATOM | 141 | CA | SER | A | 75 | | 47.397 | 70.860 | 11.317 | 1.00 | 22.02 | C |
| ATOM | 142 | C | SER | A | 75 | | 47.875 | 70.239 | 10.011 | 1.00 | 21.11 | C |
| ATOM | 143 | O | SER | A | 75 | | 48.237 | 70.973 | 9.134 | 1.00 | 22.13 | O |
| ATOM | 144 | CB | SER | A | 75 | | 48.518 | 71.675 | 12.029 | 1.00 | 24.87 | C |
| ATOM | 145 | OG | SER | A | 75 | | 49.228 | 70.714 | 12.745 | 1.00 | 29.58 | O |
| ATOM | 146 | N | TRP | A | 76 | | 47.838 | 68.925 | 9.796 | 1.00 | 20.28 | N |
| ATOM | 147 | CA | TRP | A | 76 | | 48.258 | 68.411 | 8.497 | 1.00 | 19.31 | C |
| ATOM | 148 | C | TRP | A | 76 | | 47.060 | 68.421 | 7.545 | 1.00 | 19.75 | C |
| ATOM | 149 | O | TRP | A | 76 | | 46.041 | 67.811 | 7.858 | 1.00 | 21.23 | O |
| ATOM | 150 | CB | TRP | A | 76 | | 48.699 | 66.935 | 8.668 | 1.00 | 20.84 | C |
| ATOM | 151 | CG | TRP | A | 76 | | 50.080 | 66.851 | 9.282 | 1.00 | 21.62 | C |
| ATOM | 152 | CD1 | TRP | A | 76 | | 50.431 | 66.404 | 10.511 | 1.00 | 25.70 | C |
| ATOM | 153 | CD2 | TRP | A | 76 | | 51.272 | 67.231 | 8.616 | 1.00 | 22.71 | C |
| ATOM | 154 | NE1 | TRP | A | 76 | | 51.829 | 66.468 | 10.655 | 1.00 | 25.82 | N |
| ATOM | 155 | CE2 | TRP | A | 76 | | 52.353 | 66.960 | 9.495 | 1.00 | 24.69 | C |
| ATOM | 156 | CE3 | TRP | A | 76 | | 51.547 | 67.749 | 7.333 | 1.00 | 21.50 | C |
| ATOM | 157 | CZ2 | TRP | A | 76 | | 53.690 | 67.222 | 9.138 | 1.00 | 28.94 | C |
| ATOM | 158 | CZ3 | TRP | A | 76 | | 52.888 | 67.989 | 6.967 | 1.00 | 28.26 | C |
| ATOM | 159 | CH2 | TRP | A | 76 | | 53.939 | 67.705 | 7.870 | 1.00 | 28.55 | C |
| ATOM | 160 | N | LEU | A | 77 | | 47.185 | 69.057 | 6.381 | 1.00 | 18.59 | N |
| ATOM | 161 | CA | LEU | A | 77 | | 46.014 | 69.116 | 5.501 | 1.00 | 19.95 | C |
| ATOM | 162 | C | LEU | A | 77 | | 46.301 | 68.189 | 4.350 | 1.00 | 18.65 | C |
| ATOM | 163 | O | LEU | A | 77 | | 47.375 | 68.261 | 3.787 | 1.00 | 20.22 | O |
| ATOM | 164 | CB | LEU | A | 77 | | 45.962 | 70.544 | 4.872 | 1.00 | 20.14 | C |
| ATOM | 165 | CG | LEU | A | 77 | | 45.658 | 71.598 | 5.939 | 1.00 | 22.99 | C |
| ATOM | 166 | CD1 | LEU | A | 77 | | 45.369 | 72.957 | 5.221 | 1.00 | 24.34 | C |
| ATOM | 167 | CD2 | LEU | A | 77 | | 44.500 | 71.216 | 6.882 | 1.00 | 21.91 | C |
| ATOM | 168 | N | ALA | A | 78 | | 45.393 | 67.287 | 3.993 | 1.00 | 18.28 | N |
| ATOM | 169 | CA | ALA | A | 78 | | 45.599 | 66.485 | 2.781 | 1.00 | 17.48 | C |
| ATOM | 170 | C | ALA | A | 78 | | 44.792 | 67.238 | 1.727 | 1.00 | 17.29 | C |
| ATOM | 171 | O | ALA | A | 78 | | 43.657 | 67.612 | 2.025 | 1.00 | 17.12 | O |
| ATOM | 172 | CB | ALA | A | 78 | | 44.939 | 65.097 | 3.074 | 1.00 | 18.39 | C |
| ATOM | 173 | N | VAL | A | 79 | | 45.357 | 67.402 | 0.528 | 1.00 | 18.14 | N |
| ATOM | 174 | CA | VAL | A | 79 | | 44.717 | 68.230 | -0.520 | 1.00 | 19.14 | C |
| ATOM | 175 | C | VAL | A | 79 | | 44.706 | 67.415 | -1.799 | 1.00 | 18.54 | C |
| ATOM | 176 | O | VAL | A | 79 | | 45.768 | 66.895 | -2.188 | 1.00 | 18.09 | O |
| ATOM | 177 | CB | VAL | A | 79 | | 45.525 | 69.499 | -0.772 | 1.00 | 19.45 | C |
| ATOM | 178 | CG1 | VAL | A | 79 | | 44.961 | 70.350 | -1.946 | 1.00 | 21.00 | C |
| ATOM | 179 | CG2 | VAL | A | 79 | | 45.620 | 70.344 | 0.538 | 1.00 | 20.65 | C |
| ATOM | 180 | N | THR | A | 80 | | 43.529 | 67.311 | -2.426 | 1.00 | 18.30 | N |
| ATOM | 181 | CA | THR | A | 80 | | 43.503 | 66.673 | -3.751 | 1.00 | 19.35 | C |
| ATOM | 182 | C | THR | A | 80 | | 42.977 | 67.672 | -4.795 | 1.00 | 19.26 | C |
| ATOM | 183 | O | THR | A | 80 | | 42.075 | 68.459 | -4.461 | 1.00 | 19.23 | O |
| ATOM | 184 | CB | THR | A | 80 | | 42.507 | 65.447 | -3.732 | 1.00 | 20.79 | C |
| ATOM | 185 | OG1 | THR | A | 80 | | 43.225 | 64.342 | -3.144 | 1.00 | 20.26 | O |
| ATOM | 186 | CG2 | THR | A | 80 | | 42.073 | 65.044 | -5.149 | 1.00 | 26.77 | C |
| ATOM | 187 | N | VAL | A | 81 | | 43.520 | 67.628 | -6.020 | 1.00 | 20.22 | N |
| ATOM | 188 | CA | VAL | A | 81 | | 42.991 | 68.542 | -7.062 | 1.00 | 21.37 | C |
| ATOM | 189 | C | VAL | A | 81 | | 42.594 | 67.677 | -8.253 | 1.00 | 23.16 | C |
| ATOM | 190 | O | VAL | A | 81 | | 43.358 | 66.780 | -8.599 | 1.00 | 22.25 | O |
| ATOM | 191 | CB | VAL | A | 81 | | 44.038 | 69.586 | -7.470 | 1.00 | 23.09 | C |
| ATOM | 192 | CG1 | VAL | A | 81 | | 43.415 | 70.519 | -8.509 | 1.00 | 25.78 | C |
| ATOM | 193 | CG2 | VAL | A | 81 | | 44.493 | 70.453 | -6.261 | 1.00 | 26.41 | C |
| ATOM | 194 | N | ASP | A | 82 | | 41.425 | 67.906 | -8.864 | 1.00 | 23.40 | N |
| ATOM | 195 | CA | ASP | A | 82 | | 41.136 | 67.112 | -10.056 | 1.00 | 26.10 | C |
| ATOM | 196 | C | ASP | A | 82 | | 41.766 | 67.879 | -11.236 | 1.00 | 27.06 | C |
| ATOM | 197 | O | ASP | A | 82 | | 41.516 | 69.060 | -11.416 | 1.00 | 28.67 | O |
| ATOM | 198 | CB | ASP | A | 82 | | 39.634 | 67.039 | -10.255 | 1.00 | 26.27 | C |
| ATOM | 199 | CG | ASP | A | 82 | | 39.303 | 66.443 | -11.648 | 1.00 | 32.37 | C |
| ATOM | 200 | OD1 | ASP | A | 82 | | 39.508 | 65.262 | -11.871 | 1.00 | 38.29 | O |
| ATOM | 201 | OD2 | ASP | A | 82 | | 38.876 | 67.192 | -12.543 | 1.00 | 39.87 | O |
| ATOM | 202 | N | THR | A | 83 | | 42.580 | 67.221 | -12.027 | 1.00 | 28.98 | N |
| ATOM | 203 | CA | THR | A | 83 | | 43.441 | 67.986 | -12.951 | 1.00 | 31.21 | C |
| ATOM | 204 | C | THR | A | 83 | | 42.643 | 68.604 | -14.123 | 1.00 | 32.90 | C |
| ATOM | 205 | O | THR | A | 83 | | 43.068 | 69.607 | -14.733 | 1.00 | 34.55 | O |
| ATOM | 206 | CB | THR | A | 83 | | 44.675 | 67.219 | -13.424 | 1.00 | 31.77 | C |
| ATOM | 207 | OG1 | THR | A | 83 | | 44.302 | 65.980 | -14.016 | 1.00 | 31.94 | O |
| ATOM | 208 | CG2 | THR | A | 83 | | 45.688 | 66.946 | -12.261 | 1.00 | 33.81 | C |
| ATOM | 209 | N | ALA | A | 84 | | 41.509 | 68.002 | -14.427 | 1.00 | 32.96 | N |
| ATOM | 210 | CA | ALA | A | 84 | | 40.734 | 68.391 | -15.618 | 1.00 | 34.17 | C |
| ATOM | 211 | C | ALA | A | 84 | | 39.831 | 69.535 | -15.204 | 1.00 | 35.36 | C |
| ATOM | 212 | O | ALA | A | 84 | | 39.709 | 70.489 | -15.960 | 1.00 | 36.80 | O |
| ATOM | 213 | CB | ALA | A | 84 | | 39.971 | 67.196 | -16.176 | 1.00 | 33.70 | C |
| ATOM | 214 | N | SER | A | 85 | | 39.225 | 69.492 | -14.009 | 1.00 | 34.73 | N |
| ATOM | 215 | CA | SER | A | 85 | | 38.261 | 70.506 | -13.549 | 1.00 | 33.84 | C |
| ATOM | 216 | C | SER | A | 85 | | 38.839 | 71.542 | -12.589 | 1.00 | 33.78 | C |
| ATOM | 217 | O | SER | A | 85 | | 38.162 | 72.521 | -12.323 | 1.00 | 34.28 | O |
| ATOM | 218 | CB | SER | A | 85 | | 37.055 | 69.876 | -12.836 | 1.00 | 34.71 | C |
| ATOM | 219 | OG | SER | A | 85 | | 37.446 | 69.423 | -11.512 | 1.00 | 34.57 | O |
| ATOM | 220 | N | SER | A | 86 | | 40.034 | 71.333 | -12.028 | 1.00 | 31.96 | N |
| ATOM | 221 | CA | SER | A | 86 | | 40.532 | 72.227 | -10.957 | 1.00 | 31.52 | C |
| ATOM | 222 | C | SER | A | 86 | | 39.752 | 72.164 | -9.639 | 1.00 | 29.36 | C |
| ATOM | 223 | O | SER | A | 86 | | 40.021 | 73.006 | -8.783 | 1.00 | 28.62 | O |
| ATOM | 224 | CB | SER | A | 86 | | 40.687 | 73.681 | -11.364 | 1.00 | 33.59 | C |
| ATOM | 225 | OG | SER | A | 86 | | 41.552 | 73.681 | -12.491 | 1.00 | 38.02 | O |
| ATOM | 226 | N | ALA | A | 87 | | 38.795 | 71.238 | -9.484 | 1.00 | 27.25 | N |
| ATOM | 227 | CA | ALA | A | 87 | | 38.064 | 71.109 | -8.180 | 1.00 | 25.49 | C |
| ATOM | 228 | C | ALA | A | 87 | | 39.085 | 70.732 | -7.093 | 1.00 | 23.24 | C |
| ATOM | 229 | O | ALA | A | 87 | | 40.088 | 70.086 | -7.428 | 1.00 | 23.89 | O |
| ATOM | 230 | CB | ALA | A | 87 | | 37.014 | 69.996 | -8.259 | 1.00 | 26.53 | C |
| ATOM | 231 | N | ILE | A | 88 | | 38.836 | 71.069 | -5.825 | 1.00 | 22.30 | N |
| ATOM | 232 | CA | ILE | A | 88 | | 39.908 | 70.866 | -4.787 | 1.00 | 22.68 | C |
| ATOM | 233 | C | ILE | A | 88 | | 39.128 | 70.350 | -3.557 | 1.00 | 21.14 | C |
| ATOM | 234 | O | ILE | A | 88 | | 38.109 | 70.919 | -3.183 | 1.00 | 20.97 | O |
| ATOM | 235 | CB | ILE | A | 88 | | 40.567 | 72.204 | -4.422 | 1.00 | 23.69 | C |
| ATOM | 236 | CG1 | ILE | A | 88 | | 41.376 | 72.818 | -5.601 | 1.00 | 24.85 | C |
| ATOM | 237 | CG2 | ILE | A | 88 | | 41.402 | 72.174 | -3.045 | 1.00 | 24.97 | C |
| ATOM | 238 | CD1 | ILE | A | 88 | | 41.788 | 74.296 | -5.228 | 1.00 | 30.13 | C |
| ATOM | 239 | N | VAL | A | 89 | | 39.631 | 69.295 | -2.920 | 1.00 | 19.35 | N |
| ATOM | 240 | CA | VAL | A | 89 | | 39.053 | 68.788 | -1.673 | 1.00 | 19.59 | C |
| ATOM | 241 | C | VAL | A | 89 | | 40.184 | 68.784 | -0.637 | 1.00 | 18.88 | C |
| ATOM | 242 | O | VAL | A | 89 | | 41.253 | 68.292 | -0.978 | 1.00 | 19.58 | O |
| ATOM | 243 | CB | VAL | A | 89 | | 38.517 | 67.366 | -1.833 | 1.00 | 19.83 | C |
| ATOM | 244 | CG1 | VAL | A | 89 | | 38.005 | 66.824 | -0.476 | 1.00 | 18.84 | C |
| ATOM | 245 | CG2 | VAL | A | 89 | | 37.227 | 67.454 | -2.787 | 1.00 | 21.12 | C |
| ATOM | 246 | N | VAL | A | 90 | | 39.887 | 69.297 | 0.554 | 1.00 | 19.28 | N |
| ATOM | 247 | CA | VAL | A | 90 | | 40.907 | 69.284 | 1.619 | 1.00 | 17.79 | C |
| ATOM | 248 | C | VAL | A | 90 | | 40.295 | 68.619 | 2.842 | 1.00 | 18.12 | C |
| ATOM | 249 | O | VAL | A | 90 | | 39.081 | 68.837 | 3.108 | 1.00 | 17.11 | O |
| ATOM | 250 | CB | VAL | A | 90 | | 41.407 | 70.727 | 1.933 | 1.00 | 18.27 | C |
| ATOM | 251 | CG1 | VAL | A | 90 | | 40.250 | 71.743 | 2.312 | 1.00 | 19.50 | C |
| ATOM | 252 | CG2 | VAL | A | 90 | | 42.410 | 70.736 | 3.147 | 1.00 | 18.74 | C |
| ATOM | 253 | N | THR | A | 91 | | 41.124 | 67.821 | 3.581 | 1.00 | 17.24 | N |
| ATOM | 254 | CA | THR | A | 91 | | 40.691 | 67.413 | 4.946 | 1.00 | 17.93 | C |
| ATOM | 255 | C | THR | A | 91 | | 41.865 | 67.660 | 5.911 | 1.00 | 18.85 | C |
| ATOM | 256 | O | THR | A | 91 | | 43.033 | 67.619 | 5.462 | 1.00 | 18.85 | O |
| ATOM | 257 | CB | THR | A | 91 | | 40.341 | 65.886 | 4.983 | 1.00 | 17.90 | C |
| ATOM | 258 | OG1 | THR | A | 91 | | 41.376 | 65.147 | 4.323 | 1.00 | 19.23 | O |
| ATOM | 259 | CG2 | THR | A | 91 | | 39.027 | 65.674 | 4.173 | 1.00 | 19.32 | C |
| ATOM | 260 | N | GLN | A | 92 | | 41.522 | 67.869 | 7.185 | 1.00 | 18.89 | N |
| ATOM | 261 | CA | GLN | A | 92 | | 42.592 | 68.016 | 8.222 | 1.00 | 17.73 | C |
| ATOM | 262 | C | GLN | A | 92 | | 42.764 | 66.738 | 9.049 | 1.00 | 18.48 | C |
| ATOM | 263 | O | GLN | A | 92 | | 41.859 | 65.912 | 9.215 | 1.00 | 18.43 | O |
| ATOM | 264 | CB | GLN | A | 92 | | 42.391 | 69.260 | 9.149 | 1.00 | 18.95 | C |
| ATOM | 265 | CG | GLN | A | 92 | | 41.176 | 69.211 | 9.942 | 1.00 | 18.55 | C |
| ATOM | 266 | CD | GLN | A | 92 | | 40.950 | 70.444 | 10.795 | 1.00 | 22.83 | C |
| ATOM | 267 | OE1 | GLN | A | 92 | | 41.878 | 70.973 | 11.490 | 1.00 | 26.84 | O |
| ATOM | 268 | NE2 | GLN | A | 92 | | 39.752 | 70.914 | 10.739 | 1.00 | 17.80 | N |
| ATOM | 269 | N | HIS | A | 93 | | 43.976 | 66.595 | 9.573 | 1.00 | 17.45 | N |
| ATOM | 270 | CA | HIS | A | 93 | | 44.447 | 65.345 | 10.226 | 1.00 | 20.46 | C |
| ATOM | 271 | C | HIS | A | 93 | | 45.485 | 65.675 | 11.312 | 1.00 | 19.69 | C |
| ATOM | 272 | O | HIS | A | 93 | | 46.307 | 66.562 | 11.129 | 1.00 | 19.66 | O |
| ATOM | 273 | CB | HIS | A | 93 | | 45.079 | 64.397 | 9.208 | 1.00 | 19.35 | C |
| ATOM | 274 | CG | HIS | A | 93 | | 44.137 | 64.062 | 8.103 | 1.00 | 17.61 | C |
| ATOM | 275 | ND1 | HIS | A | 93 | | 43.159 | 63.086 | 8.229 | 1.00 | 19.06 | N |
| ATOM | 276 | CD2 | HIS | A | 93 | | 44.007 | 64.590 | 6.857 | 1.00 | 21.91 | C |
| ATOM | 277 | CE1 | HIS | A | 93 | | 42.465 | 63.047 | 7.099 | 1.00 | 20.22 | C |
| ATOM | 278 | NE2 | HIS | A | 93 | | 42.938 | 63.954 | 6.254 | 1.00 | 19.39 | N |
| ATOM | 279 | N | GLY | A | 94 | | 45.440 | 64.952 | 12.423 | 1.00 | 21.49 | N |
| ATOM | 280 | CA | GLY | A | 94 | | 46.472 | 65.172 | 13.462 | 1.00 | 22.91 | C |
| ATOM | 281 | C | GLY | A | 94 | | 47.831 | 64.582 | 13.125 | 1.00 | 23.89 | C |
| ATOM | 282 | O | GLY | A | 94 | | 48.834 | 65.150 | 13.584 | 1.00 | 24.78 | O |
| ATOM | 283 | N | ARG | A | 95 | | 47.923 | 63.526 | 12.308 | 1.00 | 21.51 | N |
| ATOM | 284 | CA | ARG | A | 95 | | 49.190 | 62.893 | 11.897 | 1.00 | 24.48 | C |
| ATOM | 285 | C | ARG | A | 95 | | 49.101 | 62.475 | 10.440 | 1.00 | 25.55 | C |
| ATOM | 286 | O | ARG | A | 95 | | 47.972 | 62.252 | 9.960 | 1.00 | 26.51 | O |
| ATOM | 287 | CB | ARG | A | 95 | | 49.407 | 61.605 | 12.731 | 1.00 | 27.16 | C |
| ATOM | 288 | CG | ARG | A | 95 | | 48.138 | 60.810 | 13.018 | 1.00 | 32.40 | C |
| ATOM | 289 | CD | ARG | A | 95 | | 47.333 | 60.239 | 11.765 | 1.00 | 42.01 | C |
| ATOM | 290 | NE | ARG | A | 95 | | 46.014 | 60.770 | 11.358 | 1.00 | 40.64 | N |
| ATOM | 291 | CZ | ARG | A | 95 | | 45.129 | 61.329 | 12.155 | 1.00 | 37.00 | C |
| ATOM | 292 | NH1 | ARG | A | 95 | | 45.444 | 61.439 | 13.424 | 1.00 | 35.12 | N |
| ATOM | 293 | NH2 | ARG | A | 95 | | 43.949 | 61.801 | 11.675 | 1.00 | 43.74 | N |
| ATOM | 294 | N | VAL | A | 96 | | 50.230 | 62.391 | 9.766 | 1.00 | 24.42 | N |
| ATOM | 295 | CA | VAL | A | 96 | | 50.296 | 61.954 | 8.365 | 1.00 | 26.63 | C |
| ATOM | 296 | C | VAL | A | 96 | | 50.507 | 60.445 | 8.368 | 1.00 | 26.71 | C |
| ATOM | 297 | O | VAL | A | 96 | | 51.622 | 59.938 | 8.577 | 1.00 | 30.38 | O |
| ATOM | 298 | CB | VAL | A | 96 | | 51.378 | 62.730 | 7.581 | 1.00 | 26.59 | C |
| ATOM | 299 | CG1 | VAL | A | 96 | | 51.613 | 62.173 | 6.158 | 1.00 | 29.04 | C |
| ATOM | 300 | CG2 | VAL | A | 96 | | 50.990 | 64.166 | 7.434 | 1.00 | 27.98 | C |
| ATOM | 301 | N | THR | A | 97 | | 49.432 | 59.698 | 8.177 | 1.00 | 25.13 | N |
| ATOM | 302 | CA | THR | A | 97 | | 49.541 | 58.256 | 8.205 | 1.00 | 24.84 | C |
| ATOM | 303 | C | THR | A | 97 | | 48.874 | 57.729 | 6.941 | 1.00 | 24.74 | C |
| ATOM | 304 | O | THR | A | 97 | | 48.170 | 58.482 | 6.278 | 1.00 | 22.23 | O |
| ATOM | 305 | CB | THR | A | 97 | | 48.767 | 57.654 | 9.359 | 1.00 | 25.56 | C |
| ATOM | 306 | OG1 | THR | A | 97 | | 47.371 | 58.036 | 9.304 | 1.00 | 25.43 | O |
| ATOM | 307 | CG2 | THR | A | 97 | | 49.458 | 58.087 | 10.739 | 1.00 | 28.01 | C |
| ATOM | 308 | N | SER | A | 98 | | 49.052 | 56.433 | 6.654 | 1.00 | 23.52 | N |
| ATOM | 309 | CA | SER | A | 98 | | 48.343 | 55.792 | 5.533 | 1.00 | 23.79 | C |
| ATOM | 310 | C | SER | A | 98 | | 46.826 | 55.934 | 5.699 | 1.00 | 23.10 | C |
| ATOM | 311 | O | SER | A | 98 | | 46.092 | 56.235 | 4.732 | 1.00 | 22.16 | O |
| ATOM | 312 | CB | SER | A | 98 | | 48.765 | 54.294 | 5.471 | 1.00 | 24.22 | C |
| ATOM | 313 | OG | SER | A | 98 | | 50.145 | 54.248 | 5.087 | 1.00 | 28.31 | O |
| ATOM | 314 | N | VAL | A | 99 | | 46.367 | 55.753 | 6.946 | 1.00 | 22.21 | N |
| ATOM | 315 | CA | VAL | A | 99 | | 44.962 | 55.880 | 7.292 | 1.00 | 22.08 | C |
| ATOM | 316 | C | VAL | A | 99 | | 44.446 | 57.293 | 6.950 | 1.00 | 21.54 | C |
| ATOM | 317 | O | VAL | A | 99 | | 43.350 | 57.455 | 6.358 | 1.00 | 19.41 | O |
| ATOM | 318 | CB | VAL | A | 99 | | 44.653 | 55.470 | 8.736 | 1.00 | 22.63 | C |
| ATOM | 319 | CG1 | VAL | A | 99 | | 43.229 | 55.832 | 9.113 | 1.00 | 24.95 | C |
| ATOM | 320 | CG2 | VAL | A | 99 | | 44.828 | 53.924 | 8.868 | 1.00 | 25.51 | C |
| ATOM | 321 | N | ALA | A | 100 | | 45.227 | 58.294 | 7.342 | 1.00 | 19.69 | N |
| ATOM | 322 | CA | ALA | A | 100 | | 44.824 | 59.642 | 7.022 | 1.00 | 20.35 | C |
| ATOM | 323 | C | ALA | A | 100 | | 44.649 | 59.821 | 5.4'/7 | 1.00 | 19.47 | C |
| ATOM | 324 | O | ALA | A | 100 | | 43.650 | 60.428 | 5.052 | 1.00 | 20.04 | O |
| ATOM | 325 | CB | ALA | A | 100 | | 45.892 | 60.678 | 7.537 | 1.00 | 19.79 | C |
| ATOM | 326 | N | ALA | A | 101 | | 45.594 | 59.330 | 4.672 | 1.00 | 19.43 | N |
| ATOM | 327 | CA | ALA | A | 101 | | 45.454 | 59.457 | 3.222 | 1.00 | 19.34 | C |
| ATOM | 328 | C | ALA | A | 101 | | 44.177 | 58.754 | 2.719 | 1.00 | 20.06 | C |
| ATOM | 329 | O | ALA | A | 101 | | 43.450 | 59.257 | 1.857 | 1.00 | 19.79 | O |
| ATOM | 330 | CB | ALA | A | 101 | | 46.704 | 58.918 | 2.501 | 1.00 | 19.58 | C |
| ATOM | 331 | N | GLN | A | 102 | | 43.923 | 57.557 | 3.254 | 1.00 | 20.94 | N |
| ATOM | 332 | CA | GLN | A | 102 | | 42.725 | 56.781 | 2.885 | 1.00 | 20.56 | C |
| ATOM | 333 | C | GLN | A | 102 | | 41.404 | 57.457 | 3.278 | 1.00 | 20.30 | C |
| ATOM | 334 | O | GLN | A | 102 | | 40.426 | 57.461 | 2.486 | 1.00 | 19.37 | O |
| ATOM | 335 | CB | GLN | A | 102 | | 42.886 | 55.392 | 3.523 | 1.00 | 20.74 | C |
| ATOM | 336 | CG | GLN | A | 102 | | 44.140 | 54.705 | 2.938 | 1.00 | 22.83 | C |
| ATOM | 337 | CD | GLN | A | 102 | | 44.594 | 53.492 | 3.727 | 1.00 | 26.56 | C |
| ATOM | 338 | OE1 | GLN | A | 102 | | 43.807 | 52.945 | 4.481 | 1.00 | 27.20 | O |
| ATOM | 339 | NE2 | GLN | A | 102 | | 45.844 | 53.096 | 3.582 | 1.00 | 25.57 | N |
| ATOM | 340 | N | HIS | A | 103 | | 41.333 | 58.057 | 4.479 | 1.00 | 17.68 | N |
| ATOM | 341 | CA | HIS | A | 103 | | 40.114 | 58.736 | 4.898 | 1.00 | 18.78 | C |
| ATOM | 342 | C | HIS | A | 103 | | 39.945 | 60.062 | 4.091 | 1.00 | 19.25 | C |
| ATOM | 343 | O | HIS | A | 103 | | 38.826 | 60.496 | 3.753 | 1.00 | 19.27 | O |
| ATOM | 344 | CB | HIS | A | 103 | | 40.101 | 58.998 | 6.440 | 1.00 | 20.33 | C |
| ATOM | 345 | CG | HIS | A | 103 | | 39.945 | 57.751 | 7.257 | 1.00 | 20.17 | C |
| ATOM | 346 | ND1 | HIS | A | 103 | | 39.851 | 57.753 | 8.640 | 1.00 | 19.67 | N |
| ATOM | 347 | CD2 | HIS | A | 103 | | 39.857 | 56.460 | 6.853 | 1.00 | 24.10 | C |
| ATOM | 348 | CE1 | HIS | A | 103 | | 39.702 | 56.496 | 9.054 | 1.00 | 20.78 | C |
| ATOM | 349 | NE2 | HIS | A | 103 | | 39.691 | 55.691 | 7.995 | 1.00 | 22.49 | N |
| ATOM | 350 | N | HIS | A | 104 | | 41.060 | 60.727 | 3.794 | 1.00 | 17.21 | N |
| ATOM | 351 | CA | HIS | A | 104 | | 40.994 | 61.861 | 2.860 | 1.00 | 17.76 | C |
| ATOM | 352 | C | HIS | A | 104 | | 40.439 | 61.466 | 1.467 | 1.00 | 17.67 | C |
| ATOM | 353 | O | HIS | A | 104 | | 39.497 | 62.119 | 1.001 | 1.00 | 18.30 | O |
| ATOM | 354 | CB | HIS | A | 104 | | 42.393 | 62.475 | 2.671 | 1.00 | 15.83 | C |
| ATOM | 355 | CG | HIS | A | 104 | | 42.433 | 63.448 | 1.539 | 1.00 | 17.66 | C |
| ATOM | 356 | ND1 | HIS | A | 104 | | 41.881 | 64.711 | 1.614 | 1.00 | 17.17 | N |
| ATOM | 357 | CD2 | HIS | A | 104 | | 42.923 | 63.307 | 0.292 | 1.00 | 19.13 | C |
| ATOM | 358 | CE1 | HIS | A | 104 | | 42.073 | 65.321 | 0.449 | 1.00 | 18.23 | C |
| ATOM | 359 | NE2 | HIS | A | 104 | | 42.675 | 64.485 | -0.376 | 1.00 | 18.11 | N |
| ATOM | 360 | N | TRP | A | 105 | | 40.967 | 60.409 | 0.882 | 1.00 | 18.68 | N |
| ATOM | 361 | CA | TRP | A | 105 | | 40.464 | 59.970 | -0.462 | 1.00 | 19.76 | C |
| ATOM | 362 | C | TRP | A | 105 | | 38.990 | 59.550 | -0.440 | 1.00 | 20.72 | C |
| ATOM | 363 | O | TRP | A | 105 | | 38.258 | 59.845 | -1.381 | 1.00 | 20.28 | O |
| ATOM | 364 | CB | TRP | A | 105 | | 41.368 | 58.885 | -1.056 | 1.00 | 18.45 | C |
| ATOM | 365 | CG | TRP | A | 105 | | 42.662 | 59.394 | -1.631 | 1.00 | 19.99 | C |
| ATOM | 366 | CD1 | TRP | A | 105 | | 42.888 | 60.612 | -2.258 | 1.00 | 18.98 | C |
| ATOM | 367 | CD2 | TRP | A | 105 | | 43.923 | 58.724 | -1.582 | 1.00 | 21.05 | C |
| ATOM | 368 | NE1 | TRP | A | 105 | | 44.208 | 60.686 | -2.609 | 1.00 | 18.75 | N |
| ATOM | 369 | CE2 | TRP | A | 105 | | 44.866 | 59.568 | -2.182 | 1.00 | 19.83 | C |
| ATOM | 370 | CE3 | TRP | A | 105 | | 44.358 | 57.525 | -1.015 | 1.00 | 21.63 | C |
| ATOM | 371 | CZ2 | TRP | A | 105 | | 46.218 | 59.231 | -2.318 | 1.00 | 22.95 | C |
| ATOM | 372 | CZ3 | TRP | A | 105 | | 45.701 | 57.185 | -1.154 | 1.00 | 24.15 | C |
| ATOM | 373 | CH2 | TRP | A | 105 | | 46.619 | 58.021 | -1.801 | 1.00 | 20.27 | C |
| ATOM | 374 | N | ALA | A | 106 | | 38.549 | 58.874 | 0.622 | 1.00 | 120.64 | N |
| ATOM | 375 | CA | ALA | A | 106 | | 37.139 | 58.534 | 0.738 | 1.00 | 19.49 | C |
| ATOM | 376 | C | ALA | A | 106 | | 36.325 | 59.804 | 0.779 | 1.00 | 20.45 | C |
| ATOM | 377 | O | ALA | A | 106 | | 35.241 | 59.864 | 0.173 | 1.00 | 20.85 | O |
| ATOM | 378 | CB | ALA | A | 106 | | 36.947 | 57.714 | 2.031 | 1.00 | 20.64 | C |
| ATOM | 379 | N | THR | A | A 107 | | 36.751 | 60.839 | 1.527 | 1.00 | 19.32 | N |
| ATOM | 380 | CA | THR | A | 107 | | 36.088 | 62.157 | 1.489 | 1.00 | 19.15 | C |
| ATOM | 381 | C | THR | A | A 107 | | 36.055 | 62.753 | 0.072 | 1.00 | 18.07 | C |
| ATOM | 382 | O | THR | A | A 107 | | 35.011 | 63.186 | -0.390 | 1.00 | 20.06 | O |
| ATOM | 383 | CB | THR | A | 107 | | 36.704 | 63.139 | 2.497 | 1.00 | 18.71 | C |
| ATOM | 384 | OG1 | THR | A | 107 | | 36.590 | 62.493 | 3.775 | 1.00 | 21.31 | O |
| ATOM | 385 | CG2 | THR | A | 107 | | 35.862 | 64.435 | 2.548 | 1.00 | 21.19 | C |
| ATOM | 386 | N | ALA | A | 108 | | 37.190 | 62.764 | -0.595 | 1.00 | 18.70 | N |
| ATOM | 387 | CA | ALA | A | 108 | | 37.269 | 63.350 | -1.954 | 1.00 | 20.04 | C |
| ATOM | 388 | C | ALA | A | 108 | | 36.329 | 62.611 | -2.948 | 1.00 | 21.17 | C |
| ATOM | 389 | O | ALA | A | 108 | | 35.675 | 63.241 | -3.853 | 1.00 | 22.68 | O |
| ATOM | 390 | CB | ALA | A | 108 | | 38.698 | 63.303 | -2.438 | 1.00 | 21.68 | C |
| ATOM | 391 | N | ILE | A | 109 | | 36.238 | 61.304 | -2.761 | 1.00 | 21.38 | N |
| ATOM | 392 | CA | ILE | A | 109 | | 35.367 | 60.468 | -3.629 | 1.00 | 21.76 | C |
| ATOM | 393 | C | ILE | A | 109 | | 33.897 | 60.777 | -3.378 | 1.00 | 23.01 | C |
| ATOM | 394 | O | ILE | A | 109 | | 33.066 | 60.917 | -4.298 | 1.00 | 23.71 | O |
| ATOM | 395 | CB | ILE | A | 109 | | 35.665 | 58.963 | -3.366 | 1.00 | 21.68 | C |
| ATOM | 396 | CG1 | ILE | A | 109 | | 37.017 | 58.610 | -3.998 | 1.00 | 20.57 | C |
| ATOM | 397 | CG2 | ILE | A | 109 | | 34.527 | 58.033 | -4.024 | 1.00 | 23.84 | C |
| ATOM | 398 | CD1 | ILE | A | 109 | | 37.549 | 57.225 | -3.474 | 1.00 | 21.26 | C |
| ATOM | 399 | N | ALA | A | 110 | | 33.569 | 60.908 | -2.086 | 1.00 | 21.91 | N |
| ATOM | 400 | CA | ALA | A | 110 | | 32.224 | 61.298 | -1.687 | 1.00 | 22.65 | C |
| ATOM | 401 | C | ALA | A | 110 | | 31.828 | 62.640 | -2.257 | 1.00 | 23.34 | C |
| ATOM | 402 | O | ALA | A | 110 | | 30.676 | 62.826 | -2.646 | 1.00 | 24.72 | O |
| ATOM | 403 | CB | ALA | A | 110 | | 32.097 | 61.357 | -0.151 | 1.00 | 21.35 | C |
| ATOM | 404 | N | VAL | A | 111 | | 32.758 | 63.570 | -2.326 | 1.00 | 23.35 | N |
| ATOM | 405 | CA | VAL | A | 111 | | 32.515 | 64.896 | -2.899 | 1.00 | 23.24 | C |
| ATOM | 406 | C | VAL | A | 111 | | 32.474 | 64.908 | -4.430 | 1.00 | 25.27 | C |
| ATOM | 407 | O | VAL | A | 111 | | 31.514 | 65.480 | -5.002 | 1.00 | 26.55 | O |
| ATOM | 408 | CB | VAL | A | 111 | | 33.515 | 65.961 | -2.354 | 1.00 | 22.91 | C |
| ATOM | 409 | CG1 | VAL | A | 111 | | 33.309 | 67.421 | -2.983 | 1.00 | 24.85 | C |
| ATOM | 410 | CG2 | VAL | A | 111 | | 33.370 | 66.146 | -0.884 | 1.00 | 20.95 | C |
| ATOM | 411 | N | LEU | A | 112 | | 33.453 | 64.306 | -5.096 | 1.00 | 25.86 | N |
| ATOM | 412 | CA | LEU | A | 112 | | 33.678 | 64.542 | -6.496 | 1.00 | 26.75 | C |
| ATOM | 413 | C | LEU | A | 112 | | 33.366 | 63.332 | -7.366 | 1.00 | 28.53 | C |
| ATOM | 414 | O | LEU | A | 112 | | 33.265 | 63.535 | -8.573 | 1.00 | 30.23 | O |
| ATOM | 415 | CB | LEU | A | 112 | | 35.150 | 64.858 | -6.749 | 1.00 | 27.73 | C |
| ATOM | 416 | CG | LEU | A | 112 | | 35.816 | 65.984 | -5.966 | 1.00 | 28.17 | C |
| ATOM | 417 | CD1 | LEU | A | 112 | | 37.357 | 65.868 | -6.184 | 1.00 | 29.71 | C |
| ATOM | 418 | CD2 | LEU | A | 112 | | 35.293 | 67.286 | -6.524 | 1.00 | 28.92 | C |
| ATOM | 419 | N | GLY | A | 113 | | 33.218 | 62.139 | -6.807 | 1.00 | 27.34 | N |
| ATOM | 420 | CA | GLY | A | 113 | | 33.137 | 60.906 | -7.585 | 1.00 | 28.48 | C |
| ATOM | 421 | C | GLY | A | 113 | | 34.447 | 60.159 | -7.596 | 1.00 | 29.10 | C |
| ATOM | 422 | O | GLY | A | 113 | | 35.504 | 60.725 | -7.282 | 1.00 | 29.57 | O |
| ATOM | 423 | N | ARG | A | 114 | | 34.395 | 58.891 | -7.975 | 1.00 | 28.13 | N |
| ATOM | 424 | CA | ARG | A | 114 | | 35.579 | 58.080 | -7.987 | 1.00 | 28.03 | C |
| ATOM | 425 | C | ARG | A | 114 | | 36.470 | 58.314 | -9.215 | 1.00 | 28.64 | C |
| ATOM | 426 | O | ARG | A | 114 | | 35.968 | 58.133 | -10.332 | 1.00 | 27.85 | O |
| ATOM | 427 | CB | ARG | A | 114 | | 35.160 | 56.601 | -7.826 | 1.00 | 28.89 | C |
| ATOM | 428 | CG | ARG | A | 114 | | 36.331 | 55.739 | -7.386 | 1.00 | 31.58 | C |
| ATOM | 429 | CD | ARG | A | 114 | | 36.009 | 54.318 | -6.989 | 1.00 | 33.17 | C |
| ATOM | 430 | NE | ARG | A | 114 | | 34.840 | 54.133 | -6.121 | 1.00 | 33.55 | N |
| ATOM | 431 | CZ | ARG | A | 114 | | 34.475 | 52.929 | -5.659 | 1.00 | 36.07 | C |
| ATOM | 432 | NH1 | ARG | A | 114 | | 35.190 | 51.852 | -5.961 | 1.00 | 35.60 | N |
| ATOM | 433 | NH2 | ARG | A | 114 | | 33.384 | 52.789 | -4.884 | 1.00 | 37.16 | N |
| ATOM | 434 | N | PRO | A | 115 | | 37.772 | 58.682 | -9.052 | 1.00 | 26.82 | N |
| ATOM | 435 | CA | PRO | A | 115 | | 38.651 | 58.972 | -10.207 | 1.00 | 27.48 | C |
| ATOM | 436 | C | PRO | A | 115 | | 39.198 | 57.684 | -10.780 | 1.00 | 27.69 | C |
| ATOM | 437 | O | PRO | A | 115 | | 39.235 | 56.645 | -10.118 | 1.00 | 27.98 | O |
| ATOM | 438 | CB | PRO | A | 115 | | 39.855 | 59.709 | -9.580 | 1.00 | 25.77 | C |
| ATOM | 439 | CG | PRO | A | 115 | | 39.992 | 58.950 | -8.195 | 1.00 | 24.13 | C |
| ATOM | 440 | CD | PRO | A | 115 | | 38.539 | 58.650 | -7.777 | 1.00 | 27.80 | C |
| ATOM | 441 | N | LYS | A | 116 | | 39.633 | 57.760 | -12.037 | 1.00 | 27.82 | N |
| ATOM | 442 | CA | LYS | A | 116 | | 40.403 | 56.682 | -12.655 | 1.00 | 28.53 | C |
| ATOM | 443 | C | LYS | A | 116 | | 41.798 | 56.451 | -12.077 | 1.00 | 26.85 | C |
| ATOM | 444 | O | LYS | A | 116 | | 42.199 | 55.298 | -11.895 | 1.00 | 25.06 | O |
| ATOM | 445 | CB | LYS | A | 116 | | 40.380 | 56.829 | -14.206 | 1.00 | 29.78 | C |
| ATOM | 446 | CG | LYS | A | 116 | | 41.410 | 55.921 | -14.917 | 1.00 | 33.34 | C |
| ATOM | 447 | CD | LYS | A | 116 | | 41.449 | 56.213 | -16.423 | 1.00 | 40.83 | C |
| ATOM | 448 | CE | LYS | A | 116 | | 42.139 | 57.515 | -16.815 | 1.00 | 45.52 | C |
| ATOM | 449 | NZ | LYS | A | 116 | | 42.317 | 57.526 | -18.316 | 1.00 | 48.69 | N |
| ATOM | 450 | N | ALA | A | 117 | | 42.531 | 57.517 | -11.721 | 1.00 | 27.07 | N |
| ATOM | 451 | CA | ALA | A | 117 | | 43.906 | 57.390 | -11.246 | 1.00 | 26.17 | C |
| ATOM | 452 | C | ALA | A | 117 | | 44.239 | 58.566 | -10.306 | 1.00 | 25.56 | C |
| ATOM | 453 | O | ALA | A | 117 | | 43.654 | 59.663 | -10.392 | 1.00 | 26.16 | O |
| ATOM | 454 | CB | ALA | A | 117 | | 44.918 | 57.354 | -12.441 | 1.00 | 28.11 | C |
| ATOM | 455 | N | ILE | A | 118 | | 45.177 | 58.318 | -9.399 | 1.00 | 25.68 | N |
| ATOM | 456 | CA | ILE | A | 118 | | 45.639 | 59.401 | -8.495 | 1.00 | 24.73 | C |
| ATOM | 457 | C | ILE | A | 118 | | 47.159 | 59.414 | -8.651 | 1.00 | 25.08 | C |
| ATOM | 458 | O | ILE | A | 118 | | 47.754 | 58.326 | -8.687 | 1.00 | 27.38 | O |
| ATOM | 459 | CB | ILE | A | 118 | | 45.259 | 58.991 | -7.022 | 1.00 | 24.91 | C |
| ATOM | 460 | CG1 | ILE | A | 118 | | 43.726 | 59.067 | -6.872 | 1.00 | 24.09 | C |
| ATOM | 461 | CG2 | ILE | A | 118 | | 45.890 | 59.975 | -6.044 | 1.00 | 24.51 | C |
| ATOM | 462 | CD1 | ILE | A | 118 | | 43.185 | 58.596 | -5.480 | 1.00 | 25.38 | C |
| ATOM | 463 | N | LYS | A | 119 | | 47.764 | 60.596 | -8.765 | 1.00 | 25.80 | N |
| ATOM | 464 | CA | LYS | A | 119 | | 49.201 | 60.744 | -8.829 | 1.00 | 26.82 | C |
| ATOM | 465 | C | LYS | A | 119 | | 49.662 | 61.409 | -7.533 | 1.00 | 25.70 | C |
| ATOM | 466 | O | LYS | A | 119 | | 49.046 | 62.381 | -7.095 | 1.00 | 25.13 | O |
| ATOM | 467 | CB | LYS | A | 119 | | 49.562 | 61.719 | -9.939 | 1.00 | 29.16 | C |
| ATOM | 468 | CG | LYS | A | 119 | | 49.000 | 61.317 | -11.311 | 1.00 | 34.93 | C |
| ATOM | 469 | CD | LYS | A | 119 | | 49.628 | 62.087 | -12.487 | 1.00 | 44.07 | C |
| ATOM | 470 | CE | LYS | A | 119 | | 49.631 | 61.100 | -13.663 | 1.00 | 48.65 | C |
| ATOM | 471 | NZ | LYS | A | 119 | | 49.903 | 61.706 | -15.017 | 1.00 | 52.40 | N |
| ATOM | 472 | N | THR | A | 120 | | 50.746 | 60.898 | -6.974 | 1.00 | 24.90 | N |
| ATOM | 473 | CA | THR | A | 120 | | 51.358 | 61.507 | -5.763 | 1.00 | 23.78 | C |
| ATOM | 474 | C | THR | A | 120 | | 52.897 | 61.495 | -5.885 | 1.00 | 25.31 | C |
| ATOM | 475 | O | THR | A | 120 | | 53.452 | 60.876 | -6.804 | 1.00 | 24.21 | O |
| ATOM | 476 | CB | THR | A | 120 | | 51.038 | 60.682 | -4.505 | 1.00 | 24.12 | C |
| ATOM | 477 | OG1 | THR | A | 120 | | 51.575 | 59.338 | -4.517 | 1.00 | 23.59 | O |
| ATOM | 478 | CG2 | THR | A | 120 | | 49.459 | 60.467 | -4.365 | 1.00 | 20.25 | C |
| ATOM | 479 | N | ASP | A | 121 | | 53.558 | 62.109 | -4.900 | 1.00 | 24.46 | N |
| ATOM | 480 | CA | ASP | A | 121 | | 54.985 | 61.897 | -4.785 | 1.00 | 26.05 | C |
| ATOM | 481 | C | ASP | A | 121 | | 55.277 | 60.590 | -4.026 | 1.00 | 27.03 | C |
| ATOM | 482 | O | ASP | A | 121 | | 54.400 | 59.729 | -3.737 | 1.00 | 25.88 | O |
| ATOM | 483 | CB | ASP | A | 121 | | 55.600 | 63.178 | -4.161 | 1.00 | 25.94 | C |
| ATOM | 484 | CG | ASP | A | 121 | | 55.081 | 63.479 | -2.738 | 1.00 | 24.70 | C |
| ATOM | 485 | OD1 | ASP | A | 121 | | 54.864 | 62.543 | -1.891 | 1.00 | 24.15 | O |
| ATOM | 486 | OD2 | ASP | A | 121 | | 54.889 | 64.683 | -2.437 | 1.00 | 25.94 | O |
| ATOM | 487 | N | ASN | A | 121 | | 56.548 | 60.433 | -3.648 | 1.00 | 26.88 | N |
| ATOM | 488 | CA | ASN | A | 122 | | 56.954 | 59.157 | -3.066 | 1.00 | 29.85 | C |
| ATOM | 489 | C | ASN | A | 122 | | 56.935 | 59.118 | -1.559 | 1.00 | 29.07 | C |
| ATOM | 490 | O | ASN | A | 122 | | 57.590 | 58.242 | -0.948 | 1.00 | 29.59 | O |
| ATOM | 491 | CB | ASN | A | 122 | | 58.375 | 58.818 | -3.570 | 1.00 | 31.90 | C |
| ATOM | 492 | CG | ASN | A | 122 | | 58.376 | 58.422 | -5.007 | 1.00 | 37.52 | C |
| ATOM | 493 | OD1 | ASN | A | 122 | | 59.089 | 59.010 | -5.827 | 1.00 | 46.60 | O |
| ATOM | 494 | ND2 | ASN | A | 122 | | 57.592 | 57.413 | -5.331 | 1.00 | 44.43 | N |
| ATOM | 495 | N | GLY | A | 123 | | 56.200 | 60.025 | -0.924 | 1.00 | 27.43 | N |
| ATOM | 496 | CA | GLY | A | 123 | | 56.052 | 60.024 | 0.527 | 1.00 | 28.99 | C |
| ATOM | 497 | C | GLY | A | 123 | | 55.576 | 58.676 | 1.059 | 1.00 | 30.40 | C |
| ATOM | 498 | O | GLY | A | 123 | | 54.781 | 57.996 | 0.402 | 1.00 | 29.96 | O |
| ATOM | 499 | N | SER | A | 124 | | 56.052 | 58.303 | 2.247 | 1.00 | 31.08 | N |
| ATOM | 500 | CA | SER | A | 124 | | 55.764 | 56.971 | 2.755 | 1.00 | 32.83 | C |
| ATOM | 501 | C | SER | A | 124 | | 54.273 | 56.686 | 3.006 | 1.00 | 32.19 | C |
| ATOM | 502 | O | SER | A | 124 | | 53.933 | 55.515 | 2.841 | 1.00 | 32.71 | O |
| ATOM | 503 | CB | SER | A | 124 | | 56.599 | 56.626 | 3.997 | 1.00 | 33.82 | C |
| ATOM | 504 | OG | SER | A | 124 | | 56.138 | 57.440 | 5.065 | 1.00 | 38.96 | O |
| ATOM | 505 | N | CYS | A | 125 | | 53.432 | 57.660 | 3.372 | 1.00 | 30.41 | N |
| ATOM | 506 | CA | CYS | A | 125 | | 51.955 | 57.543 | 3.498 | 1.00 | 31.92 | C |
| ATOM | 507 | C | CYS | A | 125 | | 51.349 | 57.065 | 2.157 | 1.00 | 30.68 | C |
| ATOM | 508 | O | CYS | A | 125 | | 50.280 | 56.439 | 2.194 | 1.00 | 31.41 | O |
| ATOM | 509 | CB | CYS | A | 125 | | 51.223 | 58.924 | 3.817 | 1.00 | 31.98 | C |
| ATOM | 510 | SG | CYS | A | 125 | | 51.890 | 60.288 | 2.762 | 1.00 | 43.12 | S |
| ATOM | 511 | N | PHE | A | 126 | | 51.984 | 57.382 | 1.030 | 1.00 | 28.93 | N |
| ATOM | 512 | CA | PHE | A | 126 | | 51.422 | 57.105 | -0.301 | 1.00 | 27.34 | C |
| ATOM | 513 | C | PHE | A | 126 | | 51.946 | 55.807 | -0.911 | 1.00 | 29.23 | C |
| ATOM | 514 | O | PHE | A | 126 | | 51.297 | 55.298 | -1.816 | 1.00 | 30.65 | O |
| ATOM | 515 | CB | PHE | A | 126 | | 51.671 | 58.260 | -1.314 | 1.00 | 25.59 | C |
| ATOM | 516 | CG | PHE | A | 126 | | 51.105 | 59.590 | -0.902 | 1.00 | 26.25 | C |
| ATOM | 517 | CD1 | PHE | A | 126 | | 49.787 | 59.691 | -0.470 | 1.00 | 26.33 | C |
| ATOM | 518 | CD2 | PHE | A | 126 | | 51.883 | 60.742 | -1.017 | 1.00 | 24.36 | C |
| ATOM | 519 | CE1 | PHE | A | 126 | | 49.285 | 60.931 | -0.085 | 1.00 | 26.60 | C |
| ATOM | 520 | CE2 | PHE | A | 126 | | 51.339 | 62.008 | -0.683 | 1.00 | 24.10 | C |
| ATOM | 521 | CZ | PHE | A | 126 | | 50.047 | 62.096 | -0.212 | 1.00 | 25.04 | C |
| ATOM | 522 | N | THR | A | 127 | | 53.086 | 55.287 | -0.446 | 1.00 | 30.13 | N |
| ATOM | 523 | CA | THR | A | 127 | | 53.708 | 54.116 | -1.083 | 1.00 | 32.56 | C |
| ATOM | 524 | C | THR | A | 127 | | 53.690 | 52.883 | -0.173 | 1.00 | 32.22 | C |
| ATOM | 525 | O | THR | A | 127 | | 54.287 | 51.826 | -0.501 | 1.00 | 32.72 | O |
| ATOM | 526 | CB | THR | A | 127 | | 55.154 | 54.442 | -1.524 | 1.00 | 34.03 | C |
| ATOM | 527 | OG1 | THR | A | 127 | | 55.876 | 54.959 | -0.382 | 1.00 | 35.28 | O |
| ATOM | 528 | CG2 | THR | A | 127 | | 55.179 | 55.502 | -2.637 | 1.00 | 35.03 | C |
| ATOM | 529 | N | SER | A | 128 | | 53.035 | 53.013 | 0.977 | 1.00 | 30.45 | N |
| ATOM | 530 | CA | SER | A | 128 | | 52.965 | 51.917 | 1.934 | 1.00 | 30.37 | C |
| ATOM | 531 | C | SER | A | 128 | | 52.147 | 50.741 | 1.364 | 1.00 | 30.11 | C |
| ATOM | 532 | O | SER | A | 128 | | 51.300 | 50.897 | 0.466 | 1.00 | 27.89 | O |
| ATOM | 533 | CB | SER | A | 128 | | 52.313 | 52.406 | 3.218 | 1.00 | 29.78 | C |
| ATOM | 534 | OG | SER | A | 128 | | 50.939 | 52.725 | 2.958 | 1.00 | 27.95 | O |
| ATOM | 535 | N | LYS | A | 129 | | 52.428 | 49.537 | 1.883 | 1.00 | 30.95 | N |
| ATOM | 536 | CA | LYS | A | 129 | | 51.575 | 48.373 | 1.550 | 1.00 | 31.89 | C |
| ATOM | 537 | C | LYS | A | 129 | | 50.059 | 48.631 | 1.826 | 1.00 | 30.20 | C |
| ATOM | 538 | O | LYS | A | 129 | | 49.169 | 48.294 | 1.003 | 1.00 | 28.68 | O |
| ATOM | 539 | CB | LYS | A | 129 | | 52.169 | 47.143 | 2.266 | 1.00 | 33.19 | C |
| ATOM | 540 | CG | LYS | A | 129 | | 51.530 | 45.823 | 1.858 | 1.00 | 39.05 | C |
| ATOM | 541 | CD | LYS | A | 129 | | 52.431 | 44.655 | 2.286 | 1.00 | 42.71 | C |
| ATOM | 542 | CE | LYS | A | 129 | | 51.666 | 43.334 | 2.146 | 1.00 | 47.88 | C |
| ATOM | 543 | NZ | LYS | A | 129 | | 52.466 | 42.156 | 2.682 | 1.00 | 49.68 | N |
| ATOM | 544 | N | SER | A | 130 | | 49.768 | 49.273 | 2.966 | 1.00 | 29.37 | N |
| ATOM | 545 | CA | SER | A | 130 | | 48.430 | 49.653 | 3.349 | 1.00 | 28.53 | C |
| ATOM | 546 | C | SER | A | 130 | | 47.742 | 50.462 | 2.238 | 1.00 | 27.00 | C |
| ATOM | 547 | O | SER | A | 130 | | 46.613 | 50.122 | 1.829 | 1.00 | 25.28 | O |
| ATOM | 548 | CB | SER | A | 130 | | 48.470 | 50.506 | 4.641 | 1.00 | 28.89 | C |
| ATOM | 549 | OG | SER | A | 130 | | 47.141 | 50.762 | 4.970 | 1.00 | 32.45 | O |
| ATOM | 550 | N | THR | A | 131 | | 48.389 | 51.538 | 1.781 | 1.00 | 26.07 | N |
| ATOM | 551 | CA | THR | A | 131 | | 47.796 | 52.408 | 0.750 | 1.00 | 26.36 | C |
| ATOM | 552 | C | THR | A | 131 | | 47.690 | 51.708 | -0.595 | 1.00 | 27.72 | C |
| ATOM | 553 | O | THR | A | 131 | | 46.714 | 51.862 | -1.344 | 1.00 | 26.38 | O |
| ATOM | 554 | CB | THR | A | 131 | | 48.546 | 53.747 | 0.703 | 1.00 | 27.16 | C |
| ATOM | 555 | OG1 | THR | A | 131 | | 48.324 | 54.373 | 1.965 | 1.00 | 27.32 | O |
| ATOM | 556 | CG2 | THR | A | 131 | | 48.018 | 54.646 | -0.395 | 1.00 | 26.65 | C |
| ATOM | 557 | N | ARG | A | 132 | | 48.724 | 50.937 | -0.939 | 1.00 | 27.42 | N |
| ATOM | 558 | CA | ARG | A | 132 | | 48.692 | 50.212 | -2.206 | 1.00 | 29.98 | C |
| ATOM | 559 | C | ARG | A | 132 | | 47.480 | 49.260 | -2.235 | 1.00 | 29.21 | C |
| ATOM | 560 | O | ARG | A | 132 | | 46.750 | 49.225 | -3.251 | 1.00 | 29.64 | O |
| ATOM | 561 | CB | ARG | A | 132 | | 50.037 | 49.458 | -2.407 | 1.00 | 30.23 | C |
| ATOM | 562 | CG | ARG | A | 132 | | 51.221 | 50.429 | -2.519 | 1.00 | 37.59 | C |
| ATOM | 563 | CD | ARG | A | 132 | | 52.517 | 49.732 | -2.984 | 1.00 | 46.26 | C |
| ATOM | 564 | NE | ARG | A | 132 | | 52.787 | 48.429 | -2.349 | 1.00 | 52.30 | N |
| ATOM | 565 | CZ | ARG | A | 132 | | 53.738 | 48.188 | -1.435 | 1.00 | 55.72 | C |
| ATOM | 566 | NH1 | ARG | A | 132 | | 54.562 | 49.150 | -1.014 | 1.00 | 58.36 | N |
| ATOM | 567 | NH2 | ARG | A | 132 | | 53.902 | 46.971 | -0.922 | 1.00 | 55.88 | N |
| ATOM | 568 | N | GLU | A | 133 | | 47.254 | 48.526 | -1.138 | 1.00 | 29.84 | N |
| ATOM | 569 | CA | GLU | A | 133 | | 46.113 | 47.622 | -0.937 | 1.00 | 30.07 | C |
| ATOM | 570 | C | GLU | A | 133 | | 44.764 | 48.307 | -1.010 | 1.00 | 29.01 | C |
| ATOM | 571 | O | GLU | A | 133 | | 43.867 | 47.834 | -1.709 | 1.00 | 27.28 | O |
| ATOM | 572 | CB | GLU | A | 133 | | 46.196 | 46.786 | 0.343 | 1.00 | 32.79 | C |
| ATOM | 573 | CG | GLU | A | 133 | | 47.380 | 45.846 | 0.212 | 1.00 | 33.48 | C |
| ATOM | 574 | CD | GLU | A | 133 | | 47.540 | 45.068 | 1.528 | 1.00 | 43.13 | C |
| ATOM | 575 | OE1 | GLU | A | A 133 | | 47.178 | 45.599 | 2.603 | 1.00 | 44.42 | O |
| ATOM | 576 | OE2 | GLU | A | 133 | | 48.054 | 43.927 | 1.486 | 1.00 | 45.20 | O |
| ATOM | 577 | N | TRP | A | 134 | | 44.664 | 49.443 | -0.325 | 1.00 | 26.33 | N |
| ATOM | 578 | CA | TRP | A | 134 | | 43.415 | 50.210 | -0.375 | 1.00 | 25.24 | C |
| ATOM | 579 | C | TRP | A | 134 | | 43.066 | 50.680 | -1.804 | 1.00 | 24.17 | C |
| ATOM | 580 | O | TRP | A | 134 | | 41.885 | 50.545 | -2.237 | 1.00 | 24.21 | O |
| ATOM | 581 | CB | TRP | A | 134 | | 43.593 | 51.423 | 0.568 | 1.00 | 25.62 | C |
| ATOM | 582 | CG | TRP | A | 134 | | 42.342 | 52.192 | 0.824 | 1.00 | 21.63 | C |
| ATOM | 583 | CD1 | TRP | A | 134 | | 41.475 | 51.983 | 1.846 | 1.00 | 23.99 | C |
| ATOM | 584 | CD2 | TRP | A | 134 | | 41.829 | 53.288 | 0.049 | 1.00 | 24.17 | C |
| ATOM | 585 | NE1 | TRP | A | 134 | | 40.440 | 52.886 | 1.756 | 1.00 | 25.44 | N |
| ATOM | 586 | CE2 | TRP | A | 134 | | 40.646 | 53.704 | 0.673 | 1.00 | 25.10 | C |
| ATOM | 587 | CE3 | TRP | A | 134 | | 42.269 | 53.945 | -1.115 | 1.00 | 23.69 | C |
| ATOM | 588 | CZ2 | TRP | A | 134 | | 39.857 | 54.734 | 0.154 | 1.00 | 25.51 | C |
| ATOM | 589 | CZ3 | TRP | A | 134 | | 41.507 | 55.032 | -1.611 | 1.00 | 24.42 | C |
| ATOM | 590 | CH2 | TRP | A | 134 | | 40.324 | 55.394 | -0.954 | 1.00 | 20.14 | C |
| ATOM | 591 | N | LEU | A | 135 | | 44.025 | 51.270 | -2.519 | 1.00 | 25.23 | N |
| ATOM | 592 | CA | LEU | A | 135 | | 43.781 | 51.837 | -3.856 | 1.00 | 25.38 | C |
| ATOM | 593 | C | LEU | A | 135 | | 43.395 | 50.674 | -4.821 | 1.00 | 26.43 | C |
| ATOM | 594 | O | LEU | A | 135 | | 42.504 | 50.834 | -5.654 | 1.00 | 26.84 | O |
| ATOM | 595 | CB | LEU | A | 135 | | 44.981 | 52.595 | -4.411 | 1.00 | 26.06 | C |
| ATOM | 596 | CG | LEU | A | 135 | | 45.257 | 53.951 | -3.737 | 1.00 | 25.45 | C |
| ATOM | 597 | CD1 | LEU | A | 135 | | 46.742 | 54.349 | -4.006 | 1.00 | 26.28 | C |
| ATOM | 598 | CD2 | LEU | A | 135 | | 44.301 | 55.003 | -4.196 | 1.00 | 25.61 | C |
| ATOM | 599 | N | ALA | A | 136 | | 44.051 | 49.519 | -4.685 | 1.00 | 27.24 | N |
| ATOM | 600 | CA | ALA | A | 136 | | 43.651 | 48.367 | -5.558 | 1.00 | 29.52 | C |
| ATOM | 601 | C | ALA | A | 136 | | 42.237 | 47.909 | -5.231 | 1.00 | 29.79 | C |
| ATOM | 602 | O | ALA | A | 136 | | 41.466 | 47.610 | -6.157 | 1.00 | 30.27 | O |
| ATOM | 603 | CB | ALA | A | 136 | | 44.620 | 47.185 | -5.351 | 1.00 | 29.95 | C |
| ATOM | 604 | N | ARG | A | 137 | | 41.878 | 47.859 | -3.951 | 1.00 | 29.48 | N |
| ATOM | 605 | CA | ARG | A | 137 | | 40.503 | 47.494 | -3.527 | 1.00 | 30.26 | C |
| ATOM | 606 | C | ARG | A | 137 | | 39.432 | 48.424 | -4.089 | 1.00 | 30.03 | C |
| ATOM | 607 | O | ARG | A | 137 | | 38.311 | 48.004 | -4.435 | 1.00 | 29.15 | O |
| ATOM | 608 | CB | ARG | A | 137 | | 40.412 | 47.413 | -1.983 | 1.00 | 30.79 | C |
| ATOM | 609 | CG | ARG | A | 137 | | 39.174 | 46.797 | -1.362 | 1.00 | 36.42 | C |
| ATOM | 610 | CD | ARG | A | 137 | | 39.275 | 46.612 | 0.182 | 1.00 | 39.46 | C |
| ATOM | 611 | NE | ARG | A | 137 | | 40.608 | 46.198 | 0.621 | 1.00 | 41.09 | N |
| ATOM | 612 | CZ | ARG | A | 137 | | 41.386 | 46.849 | 1.491 | 1.00 | 43.01 | C |
| ATOM | 613 | NH1 | ARG | A | 137 | | 40.961 | 47.974 | 2.054 | 1.00 | 46.49 | N |
| ATOM | 614 | NH2 | ARG | A | 137 | | 42.601 | 46.368 | 1.810 | 1.00 | 40.59 | N |
| ATOM | 615 | N | TRP | A | 138 | | 39.740 | 49.728 | -4.132 | 1.00 | 25.91 | N |
| ATOM | 616 | CA | TRP | A | 138 | | 38.825 | 50.692 | -4.697 | 1.00 | 26.78 | C |
| ATOM | 617 | C | TRP | A | 138 | | 38.926 | 50.822 | -6.218 | 1.00 | 26.20 | C |
| ATOM | 618 | O | TRP | A | 138 | | 38.216 | 51.635 | -6.817 | 1.00 | 28.14 | O |
| ATOM | 619 | CB | TRP | A | 138 | | 39.081 | 52.085 | -4.026 | 1.00 | 26.05 | C |
| ATOM | 620 | CG | TRP | A | 138 | | 38.583 | 52.097 | -2.585 | 1.00 | 26.92 | C |
| ATOM | 621 | CD1 | TRP | A | 138 | | 39.098 | 51.454 | -1.497 | 1.00 | 28.67 | C |
| ATOM | 622 | CD2 | TRP | A | 138 | | 37.430 | 52.799 | -2.116 | 1.00 | 29.18 | C |
| ATOM | 623 | NE1 | TRP | A | 138 | | 38.334 | 51.723 | -0.354 | 1.00 | 29.72 | N |
| ATOM | 624 | CE2 | TRP | A | 138 | | 37.317 | 52.558 | -0.727 | 1.00 | 29.75 | C |
| ATOM | 625 | CE3 | TRP | A | 138 | | 36.480 | 53.601 | -2.748 | 1.00 | 30.55 | C |
| ATOM | 626 | CZ2 | TRP | A | 138 | | 36.293 | 53.104 | 0.041 | 1.00 | 31.93 | C |
| ATOM | 627 | CZ3 | TRP | A | 138 | | 35.449 | 54.163 | -1.964 | 1.00 | 34.36 | C |
| ATOM | 628 | CH2 | TRP | A | 138 | | 35.374 | 53.901 | -0.597 | 1.00 | 33.31 | C |
| ATOM | 629 | N | GLY | A | 139 | | 39.827 | 50.085 | -6.830 | 1.00 | 27.03 | N |
| ATOM | 630 | CA | GLY | A | 139 | | 39.954 | 50.122 | -8.301 | 1.00 | 28.55 | C |
| ATOM | 631 | C | GLY | A | 139 | | 40.533 | 51.401 | -8.883 | 1.00 | 29.58 | C |
| ATOM | 632 | O | GLY | A | 139 | | 40.182 | 51.813 | -10.004 | 1.00 | 30.22 | O |
| ATOM | 633 | N | ILE | A | 140 | | 41.423 | 52.066 | -8.141 | 1.00 | 27.97 | N |
| ATOM | 634 | CA | ILE | A | 140 | | 41.965 | 53.378 | -8.590 | 1.00 | 27.20 | C |
| ATOM | 635 | C | ILE | A | 140 | | 43.432 | 53.136 | -8.903 | 1.00 | 26.87 | C |
| ATOM | 636 | O | ILE | A | 140 | | 44.194 | 52.548 | -8.081 | 1.00 | 27.38 | O |
| ATOM | 637 | CB | ILE | A | 140 | | 41.807 | 54.435 | -7.442 | 1.00 | 26.04 | C |
| ATOM | 638 | CG1 | ILE | A | 140 | | 40.307 | 54.637 | -7.191 | 1.00 | 26.21 | C |
| ATOM | 639 | CG2 | ILE | A | 140 | | 42.400 | 55.763 | -7.852 | 1.00 | 24.22 | C |
| ATOM | 640 | CD1 | ILE | A | 140 | | 40.034 | 55.430 | -5.890 | 1.00 | 26.84 | C |
| ATOM | 641 | N | ALA | A | 141 | | 43.874 | 53.563 | -10.093 | 1.00 | 27.56 | N |
| ATOM | 642 | CA | ALA | A | 141 | | 45.280 | 53.404 | -10.512 | 1.00 | 27.46 | C |
| ATOM | 643 | C | ALA | A | 141 | | 46.103 | 54.441 | -9.766 | 1.00 | 27.69 | C |
| ATOM | 644 | O | ALA | A | 141 | | 45.550 | 55.497 | -9.420 | 1.00 | 28.57 | O |
| ATOM | 645 | CB | ALA | A | 141 | | 45.439 | 53.678 | -12.026 | 1.00 | 28.92 | C |
| ATOM | 646 | N | HIS | A | 142 | | 47.381 | 54.169 | -9.557 | 1.00 | 29.58 | N |
| ATOM | 647 | CA | HIS | A | 142 | | 48.296 | 55.065 | -8.803 | 1.00 | 31.08 | C |
| ATOM | 648 | C | HIS | A | 142 | | 49.664 | 55.144 | -9.461 | 1.00 | 33.25 | C |
| ATOM | 649 | O | HIS | A | 142 | | 50.281 | 54.103 | -9.735 | 1.00 | 34.09 | O |
| ATOM | 650 | CB | HIS | A | 142 | | 48.453 | 54.537 | -7.379 | 1.00 | 30.40 | C |
| ATOM | 651 | CG | HIS | A | 142 | | 49.280 | 55.397 | -6.459 | 1.00 | 32.09 | C |
| ATOM | 652 | ND1 | HIS | A | 142 | | 50.111 | 54.853 | -5.504 | 1.00 | 35.01 | N |
| ATOM | 653 | CD2 | HIS | A | 142 | | 49.405 | 56.740 | -6.342 | 1.00 | 32.46 | C |
| ATOM | 654 | CE1 | HIS | A | 142 | | 50.712 | 55.825 | -4.835 | 1.00 | 34.69 | C |
| ATOM | 655 | NE2 | HIS | A | 142 | | 50.301 | 56.983 | -5.321 | 1.00 | 32.80 | N |
| ATOM | 656 | N | THR | A | 143 | | 50.092 | 56.378 | -9.709 | 1.00 | 33.52 | N |
| ATOM | 657 | CA | THR | A | 143 | | 51.394 | 56.717 | -10.275 | 1.00 | 36.30 | C |
| ATOM | 658 | C | THR | A | 143 | | 52.169 | 57.536 | -9.218 | 1.00 | 35.87 | C |
| ATOM | 659 | O | THR | A | 143 | | 51.527 | 58.365 | -8.543 | 1.00 | 32.29 | O |
| ATOM | 660 | CB | THR | A | 143 | | 51.166 | 57.652 | -11.463 | 1.00 | 35.97 | C |
| ATOM | 661 | OG1 | THR | A | 143 | | 50.599 | 56.844 | -12.502 | 1.00 | 42.79 | O |
| ATOM | 662 | CG2 | THR | A | 143 | | 52.509 | 58.170 | -11.995 | 1.00 | 40.45 | C |
| ATOM | 663 | N | THR | A | 144 | | 53.485 | 57.317 | -9.106 | 1.00 | 37.15 | N |
| ATOM | 664 | CA | THR | A | 144 | | 54.377 | 58.198 | -8.307 | 1.00 | 40.25 | C |
| ATOM | 665 | C | THR | A | 144 | | 55.445 | 58.872 | -9.150 | 1.00 | 41.85 | C |
| ATOM | 666 | O | THR | A | 144 | | 55.884 | 59.971 | -8.758 | 1.00 | 44.25 | O |
| ATOM | 667 | CB | THR | A | 144 | | 55.154 | 57.589 | -7.124 | 1.00 | 41.05 | C |
| ATOM | 668 | OG1 | THR | A | 144 | | 56.157 | 56.695 | -7.616 | 1.00 | 45.44 | O |
| ATOM | 669 | CG2 | THR | A | 144 | | 54.290 | 56.872 | -6.123 | 1.00 | 41.10 | C |
| ATOM | 670 | N | GLN | A | 153 | | 51.822 | 68.009 | -8.810 | 1.00 | 46.12 | N |
| ATOM | 671 | CA | GLN | A | 153 | | 52.735 | 69.090 | -8.486 | 1.00 | 43.38 | C |
| ATOM | 672 | C | GLN | A | 153 | | 52.270 | 70.532 | -8.687 | 1.00 | 41.97 | C |
| ATOM | 673 | O | GLN | A | 153 | | 51.850 | 71.127 | -7.702 | 1.00 | 40.54 | O |
| ATOM | 674 | CB | GLN | A | 153 | | 54.149 | 68.827 | -8.990 | 1.00 | 44.37 | C |
| ATOM | 675 | N | ALA | A | 154 | | 52.343 | 71.152 | -9.868 | 1.00 | 39.70 | N |
| ATOM | 676 | CA | ALA | A | 154 | | 51.987 | 72.570 | -9.913 | 1.00 | 37.60 | C |
| ATOM | 677 | C | ALA | A | 154 | | 50.543 | 72.898 | -9.511 | 1.00 | 35.44 | C |
| ATOM | 678 | O | ALA | A | 154 | | 50.307 | 73.922 | -8.865 | 1.00 | 34.24 | O |
| ATOM | 679 | CB | ALA | A | 154 | | 52.342 | 73.242 | -11.293 | 1.00 | 37.91 | C |
| ATOM | 680 | N | MET | A | 155 | | 49.581 | 72.054 | -9.879 | 1.00 | 33.24 | N |
| ATOM | 681 | CA | MET | A | 155 | | 48.187 | 72.361 | -9.525 | 1.00 | 32.59 | C |
| ATOM | 682 | C | MET | A | 155 | | 47.939 | 72.216 | -8.030 | 1.00 | 30.33 | C |
| ATOM | 683 | O | MET | A | 155 | | 47.158 | 73.001 | -7.507 | 1.00 | 128.64 | O |
| ATOM | 684 | CB | MET | A | 155 | | 47.145 | 71.531 | -10.261 | 1.00 | 32.13 | C |
| ATOM | 685 | CG | MET | A | 155 | | 47.100 | 71.950 | -11.780 | 1.00 | 37.48 | C |
| ATOM | 686 | SD | MET | A | 155 | | 45.929 | 70.929 | -12.702 | 1.00 | 40.56 | S |
| ATOM | 687 | CE | MET | A | 155 | | 44.437 | 71.689 | -12.066 | 1.00 | 37.32 | C |
| ATOM | 688 | N | VAL | A | 156 | | 48.560 | 71.208 | -7.431 | 1.00 | 32.14 | N |
| ATOM | 689 | CA | VAL | A | 156 | | 48.384 | 70.977 | -5.954 | 1.00 | 32.58 | C |
| ATOM | 690 | C | VAL | A | 156 | | 49.091 | 72.097 | -5.197 | 1.00 | 32.35 | C |
| ATOM | 691 | O | VAL | A | 156 | | 48.600 | 72.650 | -4.218 | 1.00 | 29.82 | O |
| ATOM | 692 | CB | VAL | A | 156 | | 48.730 | 69.541 | -5.478 | 1.00 | 34.30 | C |
| ATOM | 693 | CG1 | VAL | A | 156 | | 50.136 | 69.180 | -5.864 | 1.00 | 37.03 | C |
| ATOM | 694 | CG2 | VAL | A | 156 | | 48.761 | 69.535 | -3.862 | 1.00 | 35.17 | C |
| ATOM | 695 | N | GLU | A | 157 | | 50.283 | 72.484 | -5.657 | 1.00 | 32.68 | N |
| ATOM | 696 | CA | GLU | A | 157 | | 50.956 | 73.622 | -5.006 | 1.00 | 33.29 | C |
| ATOM | 697 | C | GLU | A | 157 | | 50.190 | 74.921 | -5.084 | 1.00 | 33.05 | C |
| ATOM | 698 | O | GLU | A | 157 | | 50.163 | 75.673 | -4.092 | 1.00 | 31.02 | O |
| ATOM | 699 | CB | GLU | A | 157 | | 52.378 | 73.782 | -5.544 | 1.00 | 35.14 | C |
| ATOM | 700 | CG | GLU | A | 157 | | 53.271 | 72.660 | -5.073 | 1.00 | 41.32 | C |
| ATOM | 701 | CD | GLU | A | 157 | | 54.656 | 72.611 | -5.731 | 1.00 | 49.55 | C |
| ATOM | 702 | OE1 | GLU | A | 157 | | 54.910 | 73.341 | -6.718 | 1.00 | 52.07 | O |
| ATOM | 703 | OE2 | GLU | A | 157 | | 55.484 | 71.793 | -5.253 | 1.00 | 54.15 | O |
| ATOM | 704 | N | ARG | A | 158 | | 49.604 | 75.197 | -6.248 | 1.00 | 30.64 | N |
| ATOM | 705 | CA | ARG | A | 158 | | 48.686 | 76.321 | -6.357 | 1.00 | 31.42 | C |
| ATOM | 706 | C | ARG | A | 158 | | 47.515 | 76.184 | -5.408 | 1.00 | 29.93 | C |
| ATOM | 707 | O | ARG | A | 158 | | 47.177 | 77.181 | -4.739 | 1.00 | 29.14 | O |
| ATOM | 708 | CB | ARG | A | 158 | | 48.226 | 76.553 | -7.811 | 1.00 | 32.08 | C |
| ATOM | 709 | CG | ARG | A | 158 | | 47.044 | 77.555 | -7.933 | 1.00 | 35.84 | C |
| ATOM | 710 | CD | ARG | A | 158 | | 46.697 | 77.755 | -9.414 | 1.00 | 45.84 | C |
| ATOM | 711 | NE | ARG | A | 158 | | 45.242 | 77.691 | -9.626 | 1.00 | 54.36 | N |
| ATOM | 712 | CZ | ARG | A | 158 | | 44.427 | 78.743 | -9.698 | 1.00 | 55.84 | C |
| ATOM | 713 | NH1 | ARG | A | 158 | | 44.924 | 79.972 | -9.590 | 1.00 | 58.71 | N |
| ATOM | 714 | NH2 | ARG | A | 158 | | 43.118 | 78.566 | -9.894 | 1.00 | 59.06 | N |
| ATOM | 715 | N | ALA | A | 159 | | 46.961 | 74.966 | -5.291 | 1.00 | 28.64 | N |
| ATOM | 716 | CA | ALA | A | 159 | | 45.840 | 74.803 | -4.376 | 1.00 | 27.55 | C |
| ATOM | 717 | C | ALA | A | 159 | | 46.216 | 75.052 | -2.923 | 1.00 | 26.47 | C |
| ATOM | 718 | O | ALA | A | 159 | | 45.483 | 75.684 | -2.181 | 1.00 | 25.29 | O |
| ATOM | 719 | CB | ALA | A | 159 | | 45.222 | 73.431 | -4.512 | 1.00 | 27.58 | C |
| ATOM | 720 | N | ASN | A | 160 | | 47.390 | 74.594 | -2.529 | 1.00 | 26.26 | N |
| ATOM | 721 | CA | ASN | A | 160 | | 47.847 | 74.845 | -1.165 | 1.00 | 28.25 | C |
| ATOM | 722 | C | ASN | A | 160 | | 47.961 | 76.339 | -0.839 | 1.00 | 28.69 | C |
| ATOM | 723 | O | ASN | A | 160 | | 47.591 | 76.805 | 0.242 | 1.00 | 29.08 | O |
| ATOM | 724 | CB | ASN | A | 160 | | 49.249 | 74.257 | -0.987 | 1.00 | 28.81 | C |
| ATOM | 725 | CG | ASN | A | 160 | | 49.337 | 72.747 | -1.044 | 1.00 | 28.84 | C |
| ATOM | 726 | OD1 | ASN | A | 160 | | 50.453 | 72.206 | -1.259 | 1.00 | 36.69 | O |
| ATOM | 727 | ND2 | ASN | A | 160 | | 48.254 | 72.039 | -0.853 | 1.00 | 26.54 | N |
| ATOM | 728 | N | ARG | A | 161 | | 48.566 | 77.112 | -1.739 | 1.00 | 28.06 | N |
| ATOM | 729 | CA | ARG | A | 161 | | 48.704 | 78.531 | -1.484 | 1.00 | 28.29 | C |
| ATOM | 730 | C | ARG | A | 161 | | 47.344 | 79.228 | -1.426 | 1.00 | 26.62 | C |
| ATOM | 731 | O | ARG | A | 161 | | 47.116 | 80.077 | -0.554 | 1.00 | 27.66 | O |
| ATOM | 732 | CB | ARG | A | 161 | | 49.527 | 79.176 | -2.623 | 1.00 | 29.48 | C |
| ATOM | 733 | CG | ARG | A | 161 | | 51.015 | 78.818 | -2.660 | 1.00 | 34.89 | C |
| ATOM | 734 | CD | ARG | A | 161 | | 51.850 | 79.671 | -3.686 | 1.00 | 40.28 | C |
| ATOM | 735 | NE | ARG | A | 161 | | 51.447 | 79.475 | -5.095 | 1.00 | 44.02 | N |
| ATOM | 736 | CZ | ARG | A | 161 | | 51.889 | 78.521 | -5.929 | 1.00 | 45.92 | C |
| ATOM | 737 | NH1 | ARG | A | 161 | | 52.785 | 77.623 | -5.538 | 1.00 | 47.45 | N |
| ATOM | 738 | NH2 | ARG | A | 161 | | 51.437 | 78.449 | -7.183 | 1.00 | 44.78 | N |
| ATOM | 739 | N | LEU | A | 162 | | 46.422 | 78.880 | -2.327 | 1.00 | 25.94 | N |
| ATOM | 740 | CA | LEU | A | 162 | | 45.086 | 79.503 | -2.294 | 1.00 | 25.76 | C |
| ATOM | 741 | C | LEU | A | 162 | | 44.362 | 79.132 | -1.000 | 1.00 | 24.90 | C |
| ATOM | 742 | O | LEU | A | 162 | | 43.757 | 80.017 | -0.355 | 1.00 | 25.24 | O |
| ATOM | 743 | CB | LEU | A | 162 | | 44.207 | 79.073 | -3.485 | 1.00 | 25.71 | C |
| ATOM | 744 | CG | LEU | A | 162 | | 44.695 | 79.522 | -4.864 | 1.00 | 30.31 | C |
| ATOM | 745 | CD1 | LEU | A | 162 | | 44.009 | 78.668 | -5.982 | 1.00 | 33.77 | C |
| ATOM | 746 | CD2 | LEU | A | 162 | | 44.667 | 81.053 | -5.004 | 1.00 | 33.17 | C |
| ATOM | 747 | N | LEU | A | 163 | | 44.451 | 77.848 | -0.610 | 1.00 | 24.05 | N |
| ATOM | 748 | CA | LEU | A | 163 | | 43.812 | 77.467 | 0.665 | 1.00 | 23.19 | C |
| ATOM | 749 | C | LEU | A | 163 | | 44.382 | 78.179 | 1.876 | 1.00 | 24.48 | C |
| ATOM | 750 | O | LEU | A | 163 | | 43.661 | 78.683 | 2.754 | 1.00 | 23.34 | O |
| ATOM | 751 | CB | LEU | A | 163 | | 43.912 | 75.947 | 0.929 | 1.00 | 24.22 | C |
| ATOM | 752 | CG | LEU | A | 163 | | 42.935 | 75.134 | 0.104 | 1.00 | 26.43 | C |
| ATOM | 753 | CD1 | LEU | A | 163 | | 43.453 | 73.666 | 0.133 | 1.00 | 26.46 | C |
| ATOM | 754 | CD2 | LEU | A | 163 | | 41.494 | 75.247 | 0.717 | 1.00 | 28.02 | C |
| ATOM | 755 | N | LYS | A | 164 | | 45.710 | 78.232 | 1.949 | 1.00 | 25.66 | N |
| ATOM | 756 | CA | LYS | A | 164 | | 46.338 | 78.857 | 3.117 | 1.00 | 27.09 | C |
| ATOM | 757 | C | LYS | A | 164 | | 45.992 | 80.330 | 3.215 | 1.00 | 27.49 | C |
| ATOM | 758 | O | LYS | A | 164 | | 45.832 | 80.885 | 4.305 | 1.00 | 28.63 | O |
| ATOM | 759 | CB | LYS | A | 164 | | 47.846 | 78.642 | 3.116 | 1.00 | 26.73 | C |
| ATOM | 760 | CG | LYS | A | 164 | | 48.129 | 77.150 | 3.345 | 1.00 | 28.39 | C |
| ATOM | 761 | CD | LYS | A | 164 | | 49.639 | 76.921 | 3.227 | 1.00 | 33.11 | C |
| ATOM | 762 | CE | LYS | A | 164 | | 49.975 | 75.461 | 3.446 | 1.00 | 33.94 | C |
| ATOM | 763 | NZ | LYS | A | 164 | | 51.428 | 75.203 | 3.182 | 1.00 | 34.97 | N |
| ATOM | 764 | N | ASP | A | 165 | | 45.950 | 80.956 | 2.064 | 1.00 | 28.80 | N |
| ATOM | 765 | CA | ASP | A | 165 | | 45.540 | 82.351 | 2.061 | 1.00 | 30.91 | C |
| ATOM | 766 | C | ASP | A | 165 | | 44.150 | 82.579 | 2.592 | 1.00 | 29.70 | C |
| ATOM | 767 | O | ASP | A | 165 | | 43.931 | 83.493 | 3.383 | 1.00 | 30.67 | O |
| ATOM | 768 | CB | ASP | A | 165 | | 45.500 | 82.872 | 0.650 | 1.00 | 31.86 | C |
| ATOM | 769 | CG | ASP | A | 165 | | 46.267 | 84.112 | 0.541 | 1.00 | 40.84 | C |
| ATOM | 770 | OD1 | ASP | A | 165 | | 45.633 | 85.186 | 0.369 | 1.00 | 49.93 | O |
| ATOM | 771 | OD2 | ASP | A | 166 | | 47 508 | 83.934 | 0.693 | 1.00 | 48.81 | O |
| ATOM | 772 | N | LYS | A | 166 | | 43.207 | 81.751 | 2.158 | 1.00 | 28.84 | N |
| ATOM | 773 | CA | LYS | A | 166 | | 41.837 | 81.902 | 2.607 | 1.00 | 27.62 | C |
| ATOM | 774 | C | LYS | A | 166 | | 41.780 | 81.640 | 4.088 | 1.00 | 26.29 | C |
| ATOM | 775 | O | LYS | A | 166 | | 41.111 | 82.340 | 4.846 | 1.00 | 24.90 | O |
| ATOM | 776 | CB | LYS | A | 166 | | 40.889 | 80.898 | 1.931 | 1.00 | 28.53 | C |
| ATOM | 777 | CG | LYS | A | 166 | | 40.495 | 81.206 | 0.473 | 1.00 | 32.92 | C |
| ATOM | 778 | CD | LYS | A | 166 | | 39.618 | 82.446 | 0.431 | 1.00 | 36.76 | C |
| ATOM | 779 | CE | LYS | A | 166 | | 39.123 | 82.840 | -0.994 | 1.00 | 40.11 | C |
| ATOM | 780 | NZ | LYS | A | 166 | | 40.163 | 83.740 | -1.607 | 1.00 | 39.73 | N |
| ATOM | 781 | N | ILE | A | 167 | | 42.515 | 80.623 | 4.536 | 1.00 | 25.44 | N |
| ATOM | 782 | CA | ILE | A | 167 | | 42.441 | 80.286 | 5.939 | 1.00 | 23.77 | C |
| ATOM | 783 | C | ILE | A | 167 | | 42.985 | 81.452 | 6.799 | 1.00 | 25.04 | C |
| ATOM | 784 | O | ILE | A | 167 | | 42.387 | 81.811 | 7.832 | 1.00 | 24.69 | O |
| ATOM | 785 | CB | ILE | A | 167 | | 43.133 | 78.929 | 6.265 | 1.00 | 25.08 | C |
| ATOM | 786 | CG1 | ILE | A | 167 | | 42.415 | 77.717 | 5.619 | 1.00 | 22.06 | C |
| ATOM | 787 | CG2 | ILE | A | 167 | | 43.246 | 78.726 | 7.836 | 1.00 | 24.05 | C |
| ATOM | 788 | CD1 | ILE | A | 167 | | 43.337 | 76.449 | 5.738 | 1.00 | 22.48 | C |
| ATOM | 789 | N | ARG | A | 168 | | 44.105 | 81.994 | 6.358 | 1.00 | 25.21 | N |
| ATOM | 790 | CA | ARG | A | 168 | | 44.713 | 83.131 | 7.068 | 1.00 | 27.71 | C |
| ATOM | 791 | C | ARG | A | 168 | | 43.767 | 84.315 | 7.198 | 1.00 | 27.96 | C |
| ATOM | 792 | O | ARG | A | 168 | | 43.562 | 84.802 | 8.325 | 1.00 | 28.24 | O |
| ATOM | 793 | CB | ARG | A | 168 | | 45.973 | 83.558 | 6.320 | 1.00 | 26.24 | C |
| ATOM | 794 | CG | ARG | A | 168 | | 46.638 | 84.793 | 6.990 | 1.00 | 33.68 | C |
| ATOM | 795 | CD | ARG | A | 168 | | 47.861 | 85.261 | 6.231 | 1.00 | 37.39 | C |
| ATOM | 796 | NE | ARG | A | 168 | | 47.497 | 85.857 | 4.954 | 1.00 | 44.48 | N |
| ATOM | 797 | CZ | ARG | A | 168 | | 47.744 | 85.329 | 3.755 | 1.00 | 46.60 | C |
| ATOM | 798 | NH1 | ARG | A | 168 | | 48.386 | 84.168 | 3.610 | 1.00 | 47.80 | N |
| ATOM | 799 | NH2 | ARG | A | 168 | | 47.334 | 85.980 | 2.665 | 1.00 | 49.10 | N |
| ATOM | 800 | N | VAL | A | 169 | | 43.196 | 84.749 | 6.071 | 1.00 | 27.92 | N |
| ATOM | 801 | CA | VAL | A | 169 | | 42.314 | 85.943 | 6.115 | 1.00 | 30.85 | C |
| ATOM | 802 | C | VAL | A | 169 | | 41.030 | 85.716 | 6.905 | 1.00 | 30.29 | C |
| ATOM | 803 | O | VAL | A | 169 | | 40.580 | 86.574 | 7.686 | 1.00 | 30.86 | O |
| ATOM | 804 | CB | VAL | A | 169 | | 42.095 | 86.545 | 4.699 | 1.00 | 32.04 | C |
| ATOM | 805 | CG1 | VAL | A | 169 | | 41.109 | 87.710 | 4.675 | 1.00 | 37.63 | C |
| ATOM | 806 | CG2 | VAL | A | 169 | | 43.410 | 87.045 | 4.139 | 1.00 | 33.46 | C |
| ATOM | 807 | N | LEU | A | 170 | | 40.409 | 84.548 | 6.739 | 1.00 | 28.03 | N |
| ATOM | 808 | CA | LEU | A | 170 | | 39.246 | 84.267 | 7.550 | 1.00 | 27.71 | C |
| ATOM | 809 | C | LEU | A | 170 | | 39.604 | 84.206 | 9.038 | 1.00 | 28.68 | C |
| ATOM | 810 | O | LEU | A | 170 | | 38.866 | 84.777 | 9.835 | 1.00 | 29.05 | O |
| ATOM | 811 | CB | LEU | A | 170 | | 38.577 | 82.952 | 7.138 | 1.00 | 25.75 | C |
| ATOM | 812 | CG | LEU | A | 170 | | 37.964 | 82.958 | 5.726 | 1.00 | 28.06 | C |
| ATOM | 813 | CD1 | LEU | A | 170 | | 37.695 | 81.498 | 5.227 | 1.00 | 25.77 | C |
| ATOM | 814 | CD2 | LEU | A | 170 | | 36.731 | 83.861 | 5.687 | 1.00 | 30.99 | C |
| ATOM | 815 | N | ALA | A | 171 | | 40.696 | 83.528 | 9.410 | 1.00 | 27.25 | N |
| ATOM | 816 | CA | ALA | A | 171 | | 40.995 | 83.432 | 10.851 | 1.00 | 29.05 | C |
| ATOM | 817 | C | ALA | A | 171 | | 41.331 | 84.835 | 11.446 | 1.00 | 30.14 | C |
| ATOM | 818 | O | ALA | A | 171 | | 40.816 | 85.213 | 12.505 | 1.00 | 32.02 | O |
| ATOM | 819 | CB | ALA | A | 171 | | 42.196 | 82.503 | 11.118 | 1.00 | 27.21 | C |
| ATOM | 820 | N | GLU | A | 172 | | 42.218 | 85.544 | 10.769 | 1.00 | 33.05 | N |
| ATOM | 821 | CA | GLU | A | 172 | | 42.662 | 86.862 | 11.286 | 1.00 | 35.38 | C |
| ATOM | 822 | C | GLU | A | 172 | | 41.479 | 87.838 | 11.376 | 1.00 | 36.76 | C |
| ATOM | 823 | O | GLU | A | 172 | | 41.319 | 88.587 | 12.350 | 1.00 | 37.29 | O |
| ATOM | 824 | CB | GLU | A | 172 | | 43.827 | 87.374 | 10.443 | 1.00 | 36.35 | C |
| ATOM | 825 | CG | GLU | A | 172 | | 45.079 | 86.593 | 10.773 | 1.00 | 38.80 | C |
| ATOM | 826 | CD | GLU | A | 172 | | 46.374 | 87.136 | 10.156 | 1.00 | 46.42 | C |
| ATOM | 827 | OE1 | GLU | A | 172 | | 46.325 | 88.136 | 9.409 | 1.00 | 48.97 | O |
| ATOM | 828 | OE2 | GLU | A | 172 | | 47.454 | 86.541 | 10.397 | 1.00 | 47.22 | O |
| ATOM | 829 | N | GLY | A | 173 | | 40.595 | 87.794 | 10.383 | 1.00 | 36.82 | N |
| ATOM | 830 | CA | GLY | A | 173 | | 39.355 | 88.535 | 10.456 | 1.00 | 37.48 | C |
| ATOM | 831 | C | GLY | A | 173 | | 38.497 | 88.233 | 11.667 | 1.00 | 38.35 | C |
| ATOM | 832 | O | GLY | A | 173 | | 37.848 | 89.153 | 12.131 | 1.00 | 38.87 | O |
| ATOM | 833 | N | ASP | A | 174 | | 38.423 | 87.008 | 12.189 | 1.00 | 37.85 | N |
| ATOM | 834 | CA | ASP | A | 174 | | 37.688 | 86.640 | 13.405 | 1.00 | 38.05 | C |
| ATOM | 835 | C | ASP | A | 174 | | 38.511 | 86.905 | 14.665 | 1.00 | 37.56 | C |
| ATOM | 836 | O | ASP | A | 174 | | 38.137 | 86.518 | 15.767 | 1.00 | 38.32 | O |
| ATOM | 837 | CB | ASP | A | 174 | | 37.526 | 85.121 | 13.435 | 1.00 | 40.37 | C |
| ATOM | 838 | CG | ASP | A | 174 | | 36.337 | 84.601 | 12.667 | 1.00 | 43.96 | C |
| ATOM | 839 | OD1 | ASP | A | 174 | | 35.328 | 85.326 | 12.528 | 1.00 | 49.00 | O |
| ATOM | 840 | OD2 | ASP | A | 174 | | 36.424 | 83.424 | 12.239 | 1.00 | 50.08 | O |
| ATOM | 841 | N | GLY | A | 175 | | 39.677 | 87.513 | 14.502 | 1.00 | 36.66 | N |
| ATOM | 842 | CA | GLY | A | 175 | | 40.519 | 87.824 | 15.637 | 1.00 | 36.44 | C |
| ATOM | 843 | C | GLY | A | 175 | | 41.452 | 86.728 | 16.061 | 1.00 | 36.31 | C |
| ATOM | 844 | O | GLY | A | 175 | | 42.084 | 86.854 | 17.128 | 1.00 | 37.74 | O |
| ATOM | 845 | N | PHE | A | 176 | | 41.574 | 85.643 | 15.286 | 1.00 | 34.17 | N |
| ATOM | 846 | CA | PHE | A | 176 | | 42.460 | 84.539 | 15.671 | 1.00 | 33.30 | C |
| ATOM | 847 | C | PHE | A | 176 | | 43.811 | 84.747 | 14.990 | 1.00 | 33.89 | C |
| ATOM | 848 | O | PHE | A | 176 | | 43.935 | 84.509 | 13.772 | 1.00 | 33.00 | O |
| ATOM | 849 | CB | PHE | A | 176 | | 41.859 | 83.161 | 15.295 | 1.00 | 32.99 | C |
| ATOM | 850 | CG | PHE | A | 176 | | 40.450 | 82.986 | 15.746 | 1.00 | 33.27 | C |
| ATOM | 851 | CD1 | PHE | A | 176 | | 40.115 | 83.154 | 17.106 | 1.00 | 35.48 | C |
| ATOM | 852 | CD2 | PHE | A | 176 | | 39.469 | 82.598 | 14.849 | 1.00 | 33.26 | C |
| ATOM | 853 | CE1 | PHE | A | 176 | | 38.815 | 82.986 | 17.540 | 1.00 | 37.13 | C |
| ATOM | 854 | CE2 | PHE | A | 176 | | 38.162 | 82.398 | 15.288 | 1.00 | 35.24 | C |
| ATOM | 855 | CZ | PHE | A | 176 | | 37.822 | 82.598 | 16.597 | 1.00 | 36.20 | C |
| ATOM | 856 | N | MET | A | 177 | | 44.794 | 85.257 | 15.741 | 1.00 | 33.46 | N |
| ATOM | 857 | CA | MET | A | 177 | | 46.057 | 85.571 | 15.066 | 1.00 | 35.26 | C |
| ATOM | 858 | C | MET | A | 177 | | 47.038 | 84.394 | 15.110 | 1.00 | 34.47 | C |
| ATOM | 859 | O | MET | A | 177 | | 48.068 | 84.409 | 14.408 | 1.00 | 36.30 | O |
| ATOM | 860 | CB | MET | A | 177 | | 46.738 | 86.832 | 15.670 | 1.00 | 36.12 | C |
| ATOM | 861 | CG | MET | A | 177 | | 45.806 | 88.052 | 15.745 | 1.00 | 38.84 | C |
| ATOM | 862 | SD | MET | A | 177 | | 45.493 | 88.542 | 14.032 | 1.00 | 45.88 | S |
| ATOM | 863 | CE | MET | A | 177 | | 43.753 | 88.590 | 14.310 | 1.00 | 42.93 | C |
| ATOM | 864 | N | LYS | A | 178 | | 46.781 | 83.391 | 15.930 | 1.00 | 33.62 | N |
| ATOM | 865 | CA | LYS | A | 178 | | 47.705 | 82.270 | 16.010 | 1.00 | 34.47 | C |
| ATOM | 866 | C | LYS | A | 178 | | 46.863 | 81.009 | 15.795 | 1.00 | 32.57 | C |
| ATOM | 867 | O | LYS | A | 178 | | 46.383 | 80.816 | 14.698 | 1.00 | 33.22 | O |
| ATOM | 868 | CB | LYS | A | 178 | | 48.485 | 82.301 | 17.332 | 1.00 | 34.86 | C |
| ATOM | 869 | CG | LYS | A | 178 | | 49.242 | 83.663 | 17.520 | 1.00 | 39.01 | C |
| ATOM | 870 | CD | LYS | A | 178 | | 49.973 | 83.622 | 18.881 | 1.00 | 44.38 | C |
| ATOM | 871 | CE | LYS | A | 178 | | 50.654 | 84.959 | 19.257 | 1.00 | 48.40 | C |
| ATOM | 872 | NZ | LYS | A | 178 | | 50.971 | 85.843 | 18.085 | 1.00 | 50.58 | N |
| ATOM | 873 | N | ARG | A | 179 | | 46.612 | 80.196 | 16.796 | 1.00 | 31.24 | N |
| ATOM | 874 | CA | ARG | A | 179 | | 45.863 | 78.972 | 16.530 | 1.00 | 30.17 | C |
| ATOM | 875 | C | ARG | A | 179 | | 44.361 | 79.294 | 16.472 | 1.00 | 29.72 | C |
| ATOM | 876 | O | ARG | A | 179 | | 43.823 | 80.076 | 17.258 | 1.00 | 29.29 | O |
| ATOM | 877 | CB | ARG | A | 179 | | 46.230 | 77.951 | 17.592 | 1.00 | 31.55 | C |
| ATOM | 878 | CG | ARG | A | 179 | | 45.717 | 76.548 | 17.465 | 1.00 | 32.53 | C |
| ATOM | 879 | CD | ARG | A | 179 | | 46.025 | 75.711 | 18.761 | 1.00 | 37.18 | C |
| ATOM | 880 | NE | ARG | A | 179 | | 47.424 | 75.267 | 18.756 | 1.00 | 45.57 | N |
| ATOM | 881 | CZ | ARG | A | 179 | | 47.953 | 74.100 | 19.155 | 1.00 | 46.97 | C |
| ATOM | 882 | NH1 | ARG | A | 179 | | 47.267 | 73.068 | 19.651 | 1.00 | 43.88 | N |
| ATOM | 883 | NH2 | ARG | A | 179 | | 49.266 | 73.969 | 19.038 | 1.00 | 49.38 | N |
| ATOM | 884 | N | ILE | A | 180 | | 43.643 | 78.684 | 15.541 | 1.00 | 27.57 | N |
| ATOM | 885 | CA | ILE | A | 180 | | 42.182 | 78.849 | 15.515 | 1.00 | 25.62 | C |
| ATOM | 886 | C | ILE | A | 180 | | 41.630 | 77.860 | 16.544 | 1.00 | 26.99 | C |
| ATOM | 887 | O | ILE | A | 180 | | 41.973 | 76.676 | 16.516 | 1.00 | 27.46 | O |
| ATOM | 888 | CB | ILE | A | 180 | | 41.622 | 78.427 | 14.100 | 1.00 | 24.32 | C |
| ATOM | 889 | CG1 | ILE | A | 180 | | 42.296 | 79.288 | 13.043 | 1.00 | 24.86 | C |
| ATOM | 890 | CG2 | ILE | A | 180 | | 40.070 | 78.433 | 14.053 | 1.00 | 25.76 | C |
| ATOM | 891 | CD1 | ILE | A | 180 | | 42.179 | 78.689 | 11.543 | 1.00 | 23.20 | C |
| ATOM | 892 | N | PRO | A | 181 | | 40.738 | 78.300 | 17.450 | 1.00 | 27.98 | N |
| ATOM | 893 | CA | PRO | A | 181 | | 40.103 | 77.377 | 18.396 | 1.00 | 29.29 | C |
| ATOM | 894 | C | PRO | A | 181 | | 39.451 | 76.186 | 17.686 | 1.00 | 28.56 | C |
| ATOM | 895 | O | PRO | A | 181 | | 38.851 | 76.322 | 16.618 | 1.00 | 27.60 | O |
| ATOM | 896 | CB | PRO | A | 181 | | 39.008 | 78.218 | 19.064 | 1.00 | 30.39 | C |
| ATOM | 897 | CG | PRO | A | 181 | | 39.475 | 79.588 | 18.931 | 1.00 | 31.55 | C |
| ATOM | 898 | CD | PRO | A | 181 | | 40.214 | 79.668 | 17.602 | 1.00 | 28.58 | C |
| ATOM | 899 | N | ALA | A | 182 | | 39.616 | 75.029 | 18.305 | 1.00 | 27.51 | N |
| ATOM | 900 | CA | ALA | A | 182 | | 39.170 | 73.795 | 17.636 | 1.00 | 27.83 | C |
| ATOM | 901 | C | ALA | A | 182 | | 37.673 | 73.861 | 17.298 | 1.00 | 27.64 | C |
| ATOM | 902 | O | ALA | A | 182 | | 37.283 | 73.373 | 16.257 | 1.00 | 27.83 | O |
| ATOM | 903 | CB | ALA | A | 182 | | 39.521 | 72.590 | 18.462 | 1.00 | 27.82 | C |
| ATOM | 904 | N | SER | A | 183 | | 36.855 | 74.511 | 18.120 | 1.00 | 27.64 | N |
| ATOM | 905 | CA | SER | A | 183 | | 35.426 | 74.579 | 17.850 | 1.00 | 28.36 | C |
| ATOM | 906 | C | SER | A | 183 | | 35.078 | 75.446 | 16.628 | 1.00 | 27.56 | C |
| ATOM | 907 | O | SER | A | 183 | | 33.949 | 75.383 | 16.157 | 1.00 | 28.36 | O |
| ATOM | 908 | CB | SER | A | 183 | | 34.678 | 75.001 | 19.133 | 1.00 | 30.36 | C |
| ATOM | 909 | OG | SER | A | 183 | | 34.952 | 76.379 | 19.334 | 1.00 | 32.78 | O |
| ATOM | 910 | N | LYS | A | 184 | | 36.018 | 76.225 | 16.099 | 1.00 | 24.94 | N |
| ATOM | 911 | CA | LYS | A | 184 | | 35.744 | 77.082 | 14.935 | 1.00 | 24.83 | C |
| ATOM | 912 | C | LYS | A | 184 | | 36.351 | 76.527 | 13.629 | 1.00 | 22.29 | C |
| ATOM | 913 | O | LYS | A | 184 | | 36.024 | 77.017 | 12.525 | 1.00 | 23.39 | O |
| ATOM | 914 | CB | LYS | A | 184 | | 36.288 | 78.499 | 15.170 | 1.00 | 25.65 | C |
| ATOM | 915 | CG | LYS | A | 184 | | 35.725 | 79.260 | 16.441 | 1.00 | 30.38 | C |
| ATOM | 916 | CD | LYS | A | 184 | | 34.213 | 79.233 | 16.510 | 1.00 | 33.47 | C |
| ATOM | 917 | CE | LYS | A | 184 | | 33.674 | 80.170 | 17.644 | 1.00 | 37.86 | C |
| ATOM | 918 | NZ | LYS | A | 184 | | 32.280 | 80.468 | 17.202 | 1.00 | 39.60 | N |
| ATOM | 919 | N | GLN | A | 185 | | 37.223 | 75.525 | 13.769 | 1.00 | 23.45 | N |
| ATOM | 920 | CA | GLN | A | 185 | | 37.971 | 74.978 | 12.618 | 1.00 | 22.51 | C |
| ATOM | 921 | C | GLN | A | 185 | | 37.150 | 74.317 | 11.534 | 1.00 | 21.90 | C |
| ATOM | 922 | O | GLN | A | 185 | | 37.400 | 74.495 | 10.340 | 1.00 | 22.97 | O |
| ATOM | 923 | CB | GLN | A | 185 | | 39.050 | 73.987 | 13.135 | 1.00 | 22.36 | C |
| ATOM | 924 | CG | GLN | A | 185 | | 40.150 | 74.779 | 13.939 | 1.00 | 23.72 | C |
| ATOM | 925 | CD | GLN | A | 185 | | 41.301 | 73.864 | 14.357 | 1.00 | 25.52 | C |
| ATOM | 926 | OE1 | GLN | A | 185 | | 42.256 | 74.302 | 15.038 | 1.00 | 26.14 | O |
| ATOM | 927 | NE2 | GLN | A | 185 | | 41.194 | 72.580 | 14.041 | 1.00 | 20.66 | N |
| ATOM | 928 | N | GLY | A | 186 | | 36.146 | 73.537 | 11.931 | 1.00 | 22.43 | N |
| ATOM | 929 | CA | GLY | A | 186 | | 35.396 | 72.769 | 10.925 | 1.00 | 21.90 | C |
| ATOM | 930 | C | GLY | A | 186 | | 34.657 | 73.679 | 9.977 | 1.00 | 24.23 | C |
| ATOM | 931 | O | GLY | A | 186 | | 34.716 | 73.484 | 8.770 | 1.00 | 24.35 | O |
| ATOM | 932 | N | GLU | A | 187 | | 34.043 | 74.737 | 10.495 | 1.00 | 23.36 | N |
| ATOM | 933 | CA | GLU | A | 187 | | 33.312 | 75.599 | 9.570 | 1.00 | 24.70 | C |
| ATOM | 934 | C | GLU | A | 187 | | 34.239 | 76.465 | 8.774 | 1.00 | 23.75 | C |
| ATOM | 935 | O | GLU | A | 187 | | 33.933 | 76.750 | 7.615 | 1.00 | 24.68 | O |
| ATOM | 936 | CB | GLU | A | 187 | | 32.318 | 76.498 | 10.342 | 1.00 | 25.73 | C |
| ATOM | 937 | CG | GLU | A | 187 | | 31.118 | 75.684 | 10.880 | 1.00 | 29.34 | C |
| ATOM | 938 | CD | GLU | A | 187 | | 29.980 | 75.539 | 9.855 | 1.00 | 38.82 | C |
| ATOM | 939 | OE1 | GLU | A | 187 | | 29.475 | 76.568 | 9.370 | 1.00 | 43.91 | O |
| ATOM | 940 | OE2 | GLU | A | 187 | | 29.557 | 74.415 | 9.515 | 1.00 | 40.36 | O |
| ATOM | 941 | N | LEU | A | 188 | | 35.345 | 76.880 | 9.403 | 1.00 | 23.52 | N |
| ATOM | 942 | CA | LEU | A | 188 | | 36.308 | 77.757 | 8.731 | 1.00 | 23.05 | C |
| ATOM | 943 | C | LEU | A | 188 | | 37.009 | 77.003 | 7.584 | 1.00 | 21.79 | C |
| ATOM | 944 | O | LEU | A | 188 | | 37.111 | 77.488 | 6.459 | 1.00 | 21.01 | O |
| ATOM | 945 | CB | LEU | A | 188 | | 37.334 | 78.369 | 9.707 | 1.00 | 23.43 | C |
| ATOM | 946 | CG | LEU | A | 188 | | 38.186 | 79.507 | 9.101 | 1.00 | 24.85 | C |
| ATOM | 947 | CD1 | LEU | A | 188 | | 38.729 | 80.360 | 10.294 | 1.00 | 29.24 | C |
| ATOM | 948 | CD2 | LEU | A | 188 | | 39.404 | 79.136 | 8.225 | 1.00 | 29.26 | C |
| ATOM | 949 | N | LEU | A | 189 | | 37.439 | 75.775 | 7.833 | 1.00 | 20.03 | N |
| ATOM | 950 | CA | LEU | A | 189 | | 38.045 | 74.996 | 6.707 | 1.00 | 20.77 | C |
| ATOM | 951 | C | LEU | A | 189 | | 37.053 | 74.736 | 5.564 | 1.00 | 21.26 | C |
| ATOM | 952 | O | LEU | A | 189 | | 37.410 | 74.823 | 4.371 | 1.00 | 20.50 | O |
| ATOM | 953 | CB | LEU | A | 189 | | 38.690 | 73.691 | 7.226 | 1.00 | 21.11 | C |
| ATOM | 954 | CG | LEU | A | 189 | | 39.427 | 72.854 | 6.168 | 1.00 | 20.35 | C |
| ATOM | 955 | CD1 | LEU | A | 189 | | 40.668 | 73.596 | 5.667 | 1.00 | 21.33 | C |
| ATOM | 956 | CD2 | LEU | A | 189 | | 39.787 | 71.539 | 6.941 | 1.00 | 25.03 | C |
| ATOM | 957 | N | ALA | A | 190 | | 35.792 | 74.436 | 5.923 | 1.00 | 20.89 | N |
| ATOM | 958 | CA | ALA | A | 190 | | 34.743 | 74.188 | 4.886 | 1.00 | 20.89 | C |
| ATOM | 959 | C | ALA | A | 190 | | 34.593 | 75.463 | 4.065 | 1.00 | 21.79 | C |
| ATOM | 960 | O | ALA | A | 190 | | 34.496 | 75.391 | 2.843 | 1.00 | 22.14 | O |
| ATOM | 961 | CB | ALA | A | 190 | | 33.382 | 73.693 | 5.458 | 1.00 | 20.68 | C |
| ATOM | 962 | N | LYS | A | 191 | | 34.537 | 76.621 | 4.735 | 1.00 | 22.03 | N |
| ATOM | 963 | CA | LYS | A | 191 | | 34.328 | 77.861 | 3.972 | 1.00 | 23.13 | C |
| ATOM | 964 | C | LYS | A | 191 | | 35.505 | 78.131 | 3.035 | 1.00 | 23.35 | C |
| ATOM | 965 | O | LYS | A | 191 | | 35.311 | 78.487 | 1.873 | 1.00 | 22.78 | O |
| ATOM | 966 | CB | LYS | A | 191 | | 34.091 | 79.014 | 4.946 | 1.00 | 24.83 | C |
| ATOM | 967 | CG | LYS | A | 191 | | 33.885 | 80.357 | 4.194 | 1.00 | 28.68 | C |
| ATOM | 968 | CD | LYS | A | 191 | | 33.459 | 81.362 | 5.298 | 1.00 | 32.34 | C |
| ATOM | 969 | CE | LYS | A | 191 | | 31.970 | 81.128 | 5.557 | 1.00 | 35.57 | C |
| ATOM | 970 | NZ | LYS | A | 191 | | 31.333 | 82.171 | 6.483 | 1.00 | 34.90 | N |
| ATOM | 971 | N | ALA | A | 192 | | 36.732 | 77.944 | 3.538 | 1.00 | 21.40 | N |
| ATOM | 972 | CA | ALA | A | 192 | | 37.887 | 78.074 | 2.633 | 1.00 | 22.33 | C |
| ATOM | 973 | C | ALA | A | 192 | | 37.841 | 77.174 | 1.421 | 1.00 | 21.91 | C |
| ATOM | 974 | O | ALA | A | 192 | | 38.150 | 77.556 | 0.261 | 1.00 | 23.44 | O |
| ATOM | 975 | CB | ALA | A | 192 | | 39.189 | 77.794 | 3.480 | 1.00 | 21.57 | C |
| ATOM | 976 | N | MET | A | 193 | | 37.476 | 75.901 | 1.664 | 1.00 | 22.65 | N |
| ATOM | 977 | CA | MET | A | 193 | | 37.304 | 74.948 | 0.556 | 1.00 | 21.35 | C |
| ATOM | 978 | C | MET | A | 193 | | 36.144 | 75.355 | -0.382 | 1.00 | 20.71 | C |
| ATOM | 979 | O | MET | A | 193 | | 36.284 | 75.286 | -1.616 | 1.00 | 22.72 | O |
| ATOM | 980 | CB | MET | A | 193 | | 37.013 | 73.498 | 1.037 | 1.00 | 22.23 | C |
| ATOM | 981 | CG | MET | A | 193 | | 36.783 | 72.536 | -0.138 | 1.00 | 19.94 | C |
| ATOM | 982 | SD | MET | A | 193 | | 36.720 | 70.810 | 0.574 | 1.00 | 23.73 | S |
| ATOM | 983 | CE | MET | A | 193 | | 35.372 | 70.789 | 1.727 | 1.00 | 30.91 | C |
| ATOM | 984 | N | N TYR | A | 194 | | 35.005 | 75.735 | 0.138 | 1.00 | 20.50 | N |
| ATOM | 985 | CA | TYR | A | 194 | | 33.887 | 76.061 | -0.789 | 1.00 | 21.32 | C |
| ATOM | 986 | C | TYR | A | 194 | | 34.252 | 77.287 | -1.647 | 1.00 | 23.43 | C |
| ATOM | 987 | O | TYR | A | 194 | | 33.787 | 77.428 | -2.818 | 1.00 | 24.29 | O |
| ATOM | 988 | CB | TYR | A | 194 | | 32.678 | 76.381 | 0.088 | 1.00 | 20.23 | C |
| ATOM | 989 | CG | TYR | A | 194 | | 31.822 | 75.150 | 0.444 | 1.00 | 23.76 | C |
| ATOM | 990 | CD1 | TYR | A | 194 | | 32.355 | 74.031 | 1.044 | 1.00 | 27.96 | C |
| ATOM | 991 | CD2 | TYR | A | 194 | | 30.457 | 75.115 | 0.072 | 1.00 | 29.74 | C |
| ATOM | 992 | CE1 | TYR | A | 194 | | 31.559 | 72.929 | 1.387 | 1.00 | 27.11 | C |
| ATOM | 993 | CE2 | TYR | A | 194 | | 29.620 | 73.978 | 0.377 | 1.00 | 30.28 | C |
| ATOM | 994 | CZ | TYR | A | 194 | | 30.214 | 72.890 | 1.031 | 1.00 | 30.65 | C |
| ATOM | 995 | OH | TYR | A | 194 | | 29.459 | 71.798 | 1.385 | 1.00 | 29.05 | O |
| ATOM | 996 | N | ALA | A | 195 | | 35.039 | 78.180 | -1.058 | 1.00 | 23.06 | N |
| ATOM | 997 | CA | ALA | A | 195 | | 35.419 | 79.408 | -1.826 | 1.00 | 24.85 | C |
| ATOM | 998 | C | ALA | A | 195 | | 36.248 | 79.115 | -3.057 | 1.00 | 25.78 | C |
| ATOM | 999 | O | ALA | A | 195 | | 36.376 | 79.957 | -3.967 | 1.00 | 26.42 | O |
| ATOM | 1000 | CB | ALA | A | 195 | | 36.199 | 80.340 | -0.927 | 1.00 | 24.54 | C |
| ATOM | 1001 | N | LEU | A | 196 | | 36.885 | 77.942 | -3.128 | 1.00 | 22.58 | N |
| ATOM | 1002 | CA | LEU | A | 196 | | 37.735 | 77.591 | -4.244 | 1.00 | 24.16 | C |
| ATOM | 1003 | C | LEU | A | 196 | | 37.054 | 76.641 | -5.185 | 1.00 | 23.82 | C |
| ATOM | 1004 | O | LEU | A | 196 | | 37.685 | 76.085 | -6.097 | 1.00 | 25.71 | O |
| ATOM | 1005 | CB | LEU | A | 196 | | 39.048 | 76.930 | -3.715 | 1.00 | 24.96 | C |
| ATOM | 1006 | CG | LEU | A | 196 | | 39.902 | 77.738 | -2.738 | 1.00 | 24.99 | C |
| ATOM | 1007 | CD1 | LEU | A | 196 | | 41.191 | 77.002 | -2.322 | 1.00 | 27.10 | C |
| ATOM | 1008 | CD2 | LEU | A | 196 | | 40.286 | 79.129 | -3.353 | 1.00 | 29.16 | C |
| ATOM | 1009 | N | ASN | A | 197 | | 35.761 | 76.436 | -4.963 | 1.00 | 23.36 | N |
| ATOM | 1010 | CA | ASN | A | 197 | | 35.001 | 75.418 | -5.726 | 1.00 | 23.03 | C |
| ATOM | 1011 | C | ASN | A | 197 | | 33.651 | 75.953 | -6.277 | 1.00 | 23.34 | C |
| ATOM | 1012 | O | ASN | A | 197 | | 33.350 | 77.125 | -6.082 | 1.00 | 23.78 | O |
| ATOM | 1013 | CB | ASN | A | 197 | | 34.718 | 74.227 | -4.789 | 1.00 | 23.71 | C |
| ATOM | 1014 | CG | ASN | A | 197 | | 35.927 | 73.240 | -4.793 | 1.00 | 23.32 | C |
| ATOM | 1015 | OD1 | ASN | A | 197 | | 36.187 | 72.539 | -5.777 | 1.00 | 22.01 | O |
| ATOM | 1016 | ND2 | ASN | A | 197 | | 36.619 | 73.190 | -3.685 | 1.00 | 21.16 | N |
| ATOM | 1017 | N | HIS | A | 198 | | 32.918 | 75.122 | -7.009 | 1.00 | 24.31 | N |
| ATOM | 1018 | CA | HIS | A | 198 | | 31.567 | 75.445 | -7.487 | 1.00 | 26.07 | C |
| ATOM | 1019 | C | HIS | A | 198 | | 30.591 | 74.385 | -7.022 | 1.00 | 27.76 | C |
| ATOM | 1020 | O | HIS | A | 198 | | 29.850 | 73.842 | -7.816 | 1.00 | 29.84 | O |
| ATOM | 1021 | CB | HIS | A | 198 | | 31.630 | 75.703 | -9.033 | 1.00 | 26.12 | C |
| ATOM | 1022 | CG | HIS | A | 198 | | 32.601 | 76.789 | -9.389 | 1.00 | 27.31 | C |
| ATOM | 1023 | ND1 | HIS | A | 198 | | 32.283 | 78.126 | -9.330 | 1.00 | 23.95 | N |
| ATOM | 1024 | CD2 | HIS | A | 198 | | 33.907 | 76.736 | -9.776 | 1.00 | 30.25 | C |
| ATOM | 1025 | CE1 | HIS | A | 198 | | 33.342 | 78.857 | -9.646 | 1.00 | 32.02 | C |
| ATOM | 1026 | NE2 | HIS | A | 198 | | 34.343 | 78.036 | -9.928 | 1.00 | 30.22 | N |
| ATOM | 1027 | N | PHE | A | 199 | | 30.569 | 74.052 | -5.724 | 1.00 | 30.51 | N |
| ATOM | 1028 | CA | PHE | A | 199 | | 29.611 | 73.106 | -5.126 | 1.00 | 31.68 | C |
| ATOM | 1029 | C | PHE | A | 199 | | 28.133 | 73.550 | -5.109 | 1.00 | 33.65 | C |
| ATOM | 1030 | O | PHE | A | 199 | | 27.921 | 74.787 | -4.998 | 1.00 | 33.54 | O |
| ATOM | 1031 | CB | PHE | A | 199 | | 30.027 | 72.772 | -3.664 | 1.00 | 31.44 | C |
| ATOM | 1032 | CG | PHE | A | 199 | | 31.433 | 72.198 | -3.527 | 1.00 | 30.57 | C |
| ATOM | 1033 | CD1 | PHE | A | 199 | | 31.959 | 71.392 | -4.515 | 1.00 | 29.25 | C |
| ATOM | 1034 | CD2 | PHE | A | 199 | | 32.189 | 72.419 | -2.362 | 1.00 | 27.71 | C |
| ATOM | 1035 | CE1 | PHE | A | 199 | | 33.268 | 70.821 | -4.362 | 1.00 | 31.97 | C |
| ATOM | 1036 | CE2 | PHE | A | 199 | | 33.498 | 71.860 | -2.236 | 1.00 | 25.48 | C |
| ATOM | 1037 | CZ | PHE | A | 199 | | 34.007 | 71.069 | -3.242 | 1.00 | 27.15 | C |
| ATOM | 1038 | OXT | PHE | A | 199 | | 27.184 | 72.703 | -5.151 | 1.00 | 33.12 | O |
| ATOM | 1040 | N | LEU | B | 58 | | 15.123 | 59.579 | -0.584 | 1.00 | 46.77 | N |
| ATOM | 1041 | CA | LEU | B | 58 | | 16.354 | 58.786 | -0.868 | 1.00 | 45.37 | C |
| ATOM | 1042 | C | LEU | B | 58 | | 16.712 | 57.806 | 0.280 | 1.00 | 44.02 | C |
| ATOM | 1043 | O | LEU | B | 58 | | 15.830 | 57.206 | 0.953 | 1.00 | 43.97 | O |
| ATOM | 1044 | CB | LEU | B | 58 | | 17.534 | 59.724 | -1.133 | 1.00 | 20.00 | C |
| ATOM | 1045 | CG | LEU | B | 58 | | 17.428 | 60.613 | -2.375 | 1.00 | 20.00 | C |
| ATOM | 1046 | CD1 | LEU | B | 58 | | 18.591 | 61.592 | -2.434 | 1.00 | 20.00 | C |
| ATOM | 1047 | CD2 | LEU | B | 58 | | 17.366 | 59.769 | -3.638 | 1.00 | 20.00 | C |
| ATOM | 1048 | N | GLN | B | 59 | | 18.005 | 57.615 | 0.535 | 1.00 | 41.12 | N |
| ATOM | 1049 | CA | GLN | B | 59 | | 18.315 | 56.467 | 1.378 | 1.00 | 38.50 | C |
| ATOM | 1050 | C | GLN | B | 59 | | 18.193 | 56.882 | 2.804 | 1.00 | 35.28 | C |
| ATOM | 1051 | O | GLN | B | 59 | | 18.447 | 58.033 | 3.157 | 1.00 | 35.12 | O |
| ATOM | 1052 | CB | GLN | B | 59 | | 19.744 | 55.937 | 1.229 | 1.00 | 39.17 | C |
| ATOM | 1053 | CG | GLN | B | 59 | | 20.140 | 55.387 | -0.116 | 1.00 | 41.61 | C |
| ATOM | 1054 | CD | GLN | B | 59 | | 21.645 | 55.290 | -0.197 | 1.00 | 44.55 | C |
| ATOM | 1055 | OE1 | GLN | B | 59 | | 22.280 | 54.483 | 0.491 | 1.00 | 41.07 | O |
| ATOM | 1056 | NE2 | GLN | B | 59 | | 22.224 | 56.139 | -1.044 | 1.00 | 47.34 | N |
| ATOM | 1057 | N | ILE | B | 60 | | 17.843 | 55.919 | 3.640 | 1.00 | 33.37 | N |
| ATOM | 1058 | CA | ILE | B | 60 | | 17.823 | 56.145 | 5.091 | 1.00 | 31.63 | C |
| ATOM | 1059 | C | ILE | B | 60 | | 18.789 | 55.193 | 5.764 | 1.00 | 30.35 | C |
| ATOM | 1060 | O | ILE | B | 60 | | 18.659 | 53.970 | 5.570 | 1.00 | 30.23 | O |
| ATOM | 1061 | CB | ILE | B | 60 | | 16.391 | 55.993 | 5.681 | 1.00 | 32.87 | C |
| ATOM | 1062 | CG1 | ILE | B | 60 | | 15.404 | 56.882 | 4.918 | 1.00 | 35.81 | C |
| ATOM | 1063 | CG2 | ILE | B | 60 | | 16.421 | 56.201 | 7.180 | 1.00 | 32.14 | C |
| ATOM | 1064 | CD1 | ILE | B | 60 | | 15.655 | 58.373 | 5.035 | 1.00 | 40.36 | C |
| ATOM | 1065 | N | TRP | B | 61 | | 19.781 | 55.710 | 6.520 | 1.00 | 27.48 | N |
| ATOM | 1066 | CA | TRP | B | 61 | | 20.754 | 54.794 | 7.130 | 1.00 | 25.59 | C |
| ATOM | 1067 | C | TRP | B | 61 | | 20.557 | 54.729 | 8.636 | 1.00 | 24.61 | C |
| ATOM | 1068 | O | TRP | B | 61 | | 20.024 | 55.656 | 9.257 | 1.00 | 27.37 | O |
| ATOM | 1069 | CB | TRP | B | 61 | | 22.221 | 55.329 | 6.963 | 1.00 | 26.27 | C |
| ATOM | 1070 | CG | TRP | B | 61 | | 22.702 | 55.378 | 5.531 | 1.00 | 26.60 | C |
| ATOM | 1071 | CD1 | TRP | B | 61 | | 22.127 | 54.824 | 4.394 | 1.00 | 26.26 | C |
| ATOM | 1072 | CD2 | TRP | B | 61 | | 23.910 | 55.999 | 5.097 | 1.00 | 24.76 | C |
| ATOM | 1073 | NE1 | TRP | B | 61 | | 22.892 | 55.105 | 3.294 | 1.00 | 26.38 | N |
| ATOM | 1074 | CE2 | TRP | B | 61 | | 24.008 | 55.808 | 3.704 | 1.00 | 24.88 | C |
| ATOM | 1075 | CE3 | TRP | B | 61 | | 24.905 | 56.700 | 5.793 | 1.00 | 25.14 | C |
| ATOM | 1076 | CZ2 | TRP | B | 61 | | 25.082 | 56.296 | 2.953 | 1.00 | 24.98 | C |
| ATOM | 1077 | CZ3 | TRP | B | 61 | | 25.961 | 57.195 | 5.064 | 1.00 | 25.51 | C |
| ATOM | 1078 | CH2 | TRP | B | 61 | | 26.051 | 56.987 | 3.653 | 1.00 | 22.04 | C |
| ATOM | 1079 | N | GLN | B | 62 | | 21.040 | 53.646 | 9.212 | 1.00 | 24.72 | N |
| ATOM | 1080 | CA | GLN | B | 62 | | 21.297 | 53.570 | 10.646 | 1.00 | 24.92 | C |
| ATOM | 1081 | C | GLN | B | 62 | | 22.788 | 53.378 | 10.948 | 1.00 | 24.64 | C |
| ATOM | 1082 | O | GLN | B | 62 | | 23.450 | 52.512 | 10.338 | 1.00 | 26.53 | O |
| ATOM | 1083 | CB | GLN | B | 62 | | 20.596 | 52.308 | 11.204 | 1.00 | 26.52 | C |
| ATOM | 1084 | CG | GLN | B | 62 | | 19.055 | 52.285 | 10.962 | 1.00 | 29.04 | C |
| ATOM | 1085 | CD | GLN | B | 62 | | 18.347 | 51.034 | 11.581 | 1.00 | 33.94 | C |
| ATOM | 1086 | OE1 | GLN | B | 62 | | 17.114 | 50.913 | 11.494 | 1.00 | 37.15 | O |
| ATOM | 1087 | NE2 | GLN | B | 62 | | 19.106 | 50.133 | 12.213 | 1.00 | 32.03 | N |
| ATOM | 1088 | N | THR | B | 63 | | 23.347 | 54.065 | 11.952 | 1.00 | 24.00 | N |
| ATOM | 1089 | CA | THR | B | 63 | | 24.777 | 53.903 | 12.218 | 1.00 | 23.68 | C |
| ATOM | 1090 | C | THR | B | 63 | | 24.980 | 53.742 | 13.716 | 1.00 | 23.59 | C |
| ATOM | 1091 | O | THR | B | 63 | | 24.262 | 54.391 | 14.464 | 1.00 | 23.83 | O |
| ATOM | 1092 | CB | THR | B | 63 | | 25.503 | 55.256 | 11.853 | 1.00 | 24.62 | C |
| ATOM | 1093 | OG1 | THR | B | 63 | | 25.331 | 55.432 | 10.441 | 1.00 | 25.87 | O |
| ATOM | 1094 | CG2 | THR | B | 63 | | 27.046 | 55.232 | 12.151 | 1.00 | 25.33 | C |
| ATOM | 1095 | N | ASP | B | 64 | | 25.978 | 52.956 | 14.074 | 1.00 | 23.14 | N |
| ATOM | 1096 | CA | ASP | B | 64 | | 26.380 | 52.841 | 15.494 | 1.00 | 25.41 | C |
| ATOM | 1097 | C | ASP | B | 64 | | 27.828 | 52.394 | 15.543 | 1.00 | 26.14 | C |
| ATOM | 1098 | O | ASP | B | 64 | | 28.379 | 51.935 | 14.536 | 1.00 | 26.31 | O |
| ATOM | 1099 | CB | ASP | B | 64 | | 25.481 | 51.735 | 16.113 | 1.00 | 25.11 | C |
| ATOM | 1100 | CG | ASP | B | 64 | | 25.480 | 51.805 | 17.609 | 1.00 | 27.87 | C |
| ATOM | 1101 | OD1 | ASP | B | 64 | | 25.702 | 52.945 | 18.068 | 1.00 | 26.42 | O |
| ATOM | 1102 | OD2 | ASP | B | 64 | | 25.375 | 50.794 | 18.338 | 1.00 | 29.68 | O |
| ATOM | 1103 | N | PHE | B | 65 | | 28.474 | 52.465 | 16.711 | 1.00 | 24.91 | N |
| ATOM | 1104 | CA | PHE | B | 65 | | 29.806 | 51.920 | 16.941 | 1.00 | 26.39 | C |
| ATOM | 1105 | C | PHE | B | 65 | | 29.686 | 50.649 | 17.807 | 1.00 | 28.08 | C |
| ATOM | 1106 | O | PHE | B | 65 | | 28.765 | 50.523 | 18.645 | 1.00 | 29.45 | O |
| ATOM | 1107 | CB | PHE | B | 65 | | 30.703 | 52.819 | 17.792 | 1.00 | 25.85 | C |
| ATOM | 1108 | CG | PHE | B | 65 | | 31.309 | 54.002 | 17.014 | 1.00 | 24.68 | C |
| ATOM | 1109 | CD1 | PHE | B | 65 | | 30.604 | 55.162 | 16.867 | 1.00 | 28.19 | C |
| ATOM | 1110 | CD2 | PHE | B | 65 | | 32.551 | 53.880 | 16.412 | 1.00 | 26.06 | C |
| ATOM | 1111 | CE1 | PHE | B | 65 | | 31.157 | 56.254 | 16.129 | 1.00 | 30.98 | C |
| ATOM | 1112 | CE2 | PHE | B | 65 | | 33.129 | 54.961 | 15.725 | 1.00 | 27.03 | C |
| ATOM | 1113 | CZ | PHE | B | 65 | | 32.417 | 56.116 | 15.575 | 1.00 | 24.18 | C |
| ATOM | 1114 | N | THR | B | 66 | | 30.633 | 49.762 | 17.572 | 1.00 | 28.36 | N |
| ATOM | 1115 | CA | THR | B | 66 | | 30.727 | 48.546 | 18.405 | 1.00 | 29.36 | C |
| ATOM | 1116 | C | THR | B | 66 | | 32.205 | 48.215 | 18.561 | 1.00 | 30.41 | C |
| ATOM | 1117 | O | THR | B | 66 | | 33.027 | 48.536 | 17.692 | 1.00 | 28.98 | O |
| ATOM | 1118 | CB | THR | B | 66 | | 29.904 | 47.435 | 17.769 | 1.00 | 29.09 | C |
| ATOM | 1119 | OG1 | THR | B | 66 | | 29.744 | 46.417 | 18.779 | 1.00 | 31.61 | O |
| ATOM | 1120 | CG2 | THR | B | 66 | | 30.608 | 46.894 | 16.516 | 1.00 | 29.35 | C |
| ATOM | 1121 | N | LEU | B | 67 | | 32.586 | 47.577 | 19.667 | 1.00 | 29.98 | N |
| ATOM | 1122 | CA | LEU | B | 67 | | 33.990 | 47.277 | 19.913 | 1.00 | 30.90 | C |
| ATOM | 1123 | C | LEU | B | 67 | | 34.297 | 45.842 | 19.537 | 1.00 | 31.34 | C |
| ATOM | 1124 | O | LEU | B | 67 | | 33.573 | 44.888 | 19.887 | 1.00 | 32.10 | O |
| ATOM | 1125 | CB | LEU | B | 67 | | 34.350 | 47.547 | 21.395 | 1.00 | 31.41 | C |
| ATOM | 1126 | CG | LEU | B | 67 | | 35.815 | 47.385 | 21.752 | 1.00 | 34.41 | C |
| ATOM | 1127 | CD1 | LEU | B | 67 | | 36.420 | 48.798 | 21.563 | 1.00 | 35.74 | C |
| ATOM | 1128 | CD2 | LEU | B | 67 | | 35.982 | 46.895 | 23.189 | 1.00 | 38.68 | C |
| ATOM | 1129 | N | GLU | B | 68 | | 35.400 | 45.684 | 18.829 | 1.00 | 30.64 | N |
| ATOM | 1130 | CA | GLU | B | 68 | | 35.907 | 44.389 | 18.400 | 1.00 | 30.57 | C |
| ATOM | 1131 | C | GLU | B | 68 | | 37.379 | 44.192 | 18.775 | 1.00 | 30.54 | C |
| ATOM | 1132 | O | GLU | B | 68 | | 38.302 | 44.582 | 18.069 | 1.00 | 29.83 | O |
| ATOM | 1133 | CB | GLU | B | 68 | | 35.614 | 44.135 | 16.899 | 1.00 | 29.68 | C |
| ATOM | 1134 | CG | GLU | B | 68 | | 36.205 | 42.831 | 16.378 | 1.00 | 33.75 | C |
| ATOM | 1135 | CD | GLU | B | 68 | | 35.807 | 41.638 | 17.258 | 1.00 | 41.52 | C |
| ATOM | 1136 | OE1 | GLU | B | 68 | | 34.614 | 41.231 | 17.189 | 1.00 | 44.28 | O |
| ATOM | 1137 | OE2 | GLU | B | 68 | | 36.676 | 41.122 | 18.021 | 1.00 | 38.43 | O |
| ATOM | 1138 | N | PRO | B | 69 | | 37.625 | 43.524 | 19.924 | 1.00 | 31.86 | N |
| ATOM | 1139 | CA | PRO | B | 69 | | 39.005 | 43.350 | 20.389 | 1.00 | 31.78 | C |
| ATOM | 1140 | C | PRO | B | 69 | | 39.980 | 42.617 | 19.486 | 1.00 | 31.21 | C |
| ATOM | 1141 | O | PRO | B | 69 | | 41.190 | 42.865 | 19.589 | 1.00 | 31.20 | O |
| ATOM | 1142 | CB | PRO | B | 69 | | 38.828 | 42.698 | 21.791 | 1.00 | 32.78 | C |
| ATOM | 1143 | CG | PRO | B | 69 | | 37.501 | 43.201 | 22.215 | 1.00 | 34.22 | C |
| ATOM | 1144 | CD | PRO | B | 69 | | 36.624 | 43.183 | 20.949 | 1.00 | 32.40 | C |
| ATOM | 1145 | N | ARG | B | 70 | | 39.512 | 41.757 | 18.579 | 1.00 | 29.59 | N |
| ATOM | 1146 | CA | ARG | B | 70 | | 40.388 | 41.151 | 17.642 | 1.00 | 29.97 | C |
| ATOM | 1147 | C | ARG | B | 70 | | 41.095 | 42.148 | 16.708 | 1.00 | 30.01 | C |
| ATOM | 1148 | O | ARG | B | 70 | | 42.120 | 41.814 | 16.147 | 1.00 | 29.78 | O |
| ATOM | 1149 | CB | ARG | B | 70 | | 39.658 | 40.113 | 16.791 | 1.00 | 30.77 | C |
| ATOM | 1150 | CG | ARG | B | 70 | | 39.150 | 38.934 | 17.685 | 1.00 | 31.08 | C |
| ATOM | 1151 | CD | ARG | B | 70 | | 38.383 | 37.970 | 16.807 | 1.00 | 37.33 | C |
| ATOM | 1152 | NE | ARG | B | 70 | | 37.622 | 37.106 | 17.720 | 1.00 | 40.20 | N |
| ATOM | 1153 | CZ | ARG | B | 70 | | 38.087 | 35.933 | 18.151 | 1.00 | 41.57 | C |
| ATOM | 1154 | NH1 | ARG | B | 70 | | 39.278 | 35.499 | 17.735 | 1.00 | 38.68 | N |
| ATOM | 1155 | NH2 | ARG | B | 70 | | 37.337 | 35.227 | 19.001 | 1.00 | 42.82 | N |
| ATOM | 1156 | N | MET | B | 71 | | 40.507 | 43.330 | 16.558 | 1.00 | 29.64 | N |
| ATOM | 1157 | CA | MET | B | 71 | | 41.003 | 44.368 | 15.624 | 1.00 | 29.09 | C |
| ATOM | 1158 | C | MET | B | 71 | | 41.747 | 45.466 | 16.408 | 1.00 | 29.75 | C |
| ATOM | 1159 | O | MET | B | 71 | | 41.969 | 46.583 | 15.944 | 1.00 | 28.38 | O |
| ATOM | 1160 | CB | MET | B | 71 | | 39.758 | 44.956 | 14.937 | 1.00 | 28.55 | C |
| ATOM | 1161 | CG | MET | B | 71 | | 39.092 | 44.066 | 13.838 | 1.00 | 28.69 | C |
| ATOM | 1162 | SD | MET | B | 71 | | 40.069 | 44.182 | 12.330 | 1.00 | 36.15 | S |
| ATOM | 1163 | CE | MET | B | 71 | | 39.358 | 42.850 | 11.303 | 1.00 | 35.48 | C |
| ATOM | 1164 | N | ALA | B | 72 | | 42.158 | 45.165 | 17.647 | 1.00 | 30.52 | N |
| ATOM | 1165 | CA | ALA | B | 72 | | 42.960 | 46.119 | 18.402 | 1.00 | 29.88 | C |
| ATOM | 1166 | C | ALA | B | 72 | | 44.330 | 46.388 | 17.781 | 1.00 | 30.12 | C |
| ATOM | 1167 | O | ALA | B | 72 | | 44.895 | 45.507 | 17.132 | 1.00 | 30.76 | O |
| ATOM | 1168 | CB | ALA | B | 72 | | 43.134 | 45.608 | 19.908 | 1.00 | 29.75 | C |
| ATOM | 1169 | N | PRO | B | 73 | | 44.899 | 47.594 | 17.993 | 1.00 | 30.16 | N |
| ATOM | 1170 | CA | PRO | B | 73 | | 44.421 | 48.732 | 18.765 | 1.00 | 30.85 | C |
| ATOM | 1171 | C | PRO | B | 73 | | 43.266 | 49.515 | 18.136 | 1.00 | 30.51 | C |
| ATOM | 1172 | O | PRO | B | 73 | | 42.588 | 50.217 | 18.898 | 1.00 | 29.87 | O |
| ATOM | 1173 | CB | PRO | B | 73 | | 45.651 | 49.661 | 18.876 | 1.00 | 32.85 | C |
| ATOM | 1174 | CG | PRO | B | 73 | | 46.513 | 49.324 | 17.707 | 1.00 | 30.86 | C |
| ATOM | 1175 | CD | PRO | B | 73 | | 46.212 | 47.846 | 17.353 | 1.00 | 32.72 | C |
| ATOM | 1176 | N | ARG | B | 74 | | 43.050 | 49.405 | 16.811 | 1.00 | 27.78 | N |
| ATOM | 1177 | CA | ARG | B | 74 | | 41.904 | 50.134 | 16.218 | 1.00 | 28.71 | C |
| ATOM | 1178 | C | ARG | B | 74 | | 40.611 | 49.317 | 16.324 | 1.00 | 28.02 | C |
| ATOM | 1179 | O | ARG | B | 74 | | 40.007 | 48.930 | 15.318 | 1.00 | 27.52 | O |
| ATOM | 1180 | CB | ARG | B | 74 | | 42.203 | 50.538 | 14.755 | 1.00 | 28.09 | C |
| ATOM | 1181 | CG | ARG | B | 74 | | 43.455 | 51.475 | 14.705 | 1.00 | 30.67 | C |
| ATOM | 1182 | CD | ARG | B | 74 | | 43.998 | 51.641 | 13.246 | 1.00 | 30.84 | C |
| ATOM | 1183 | NE | ARG | B | 74 | | 43.040 | 52.362 | 12.415 | 1.00 | 30.00 | N |
| ATOM | 1184 | CZ | ARG | B | 74 | | 42.730 | 52.056 | 11.147 | 1.00 | 28.47 | C |
| ATOM | 1185 | NH1 | ARG | B | 74 | | 43.265 | 51.039 | 10.477 | 1.00 | 28.84 | N |
| ATOM | 1186 | NH2 | ARG | B | 74 | | 41.874 | 52.823 | 10.485 | 1.00 | 28.02 | N |
| ATOM | 1187 | N | SER | B | 75 | | 40.144 | 49.065 | 17.553 | 1.00 | 26.48 | N |
| ATOM | 1188 | CA | SER | B | 75 | | 39.057 | 48.091 | 17.724 | 1.00 | 26.48 | C |
| ATOM | 1189 | C | SER | B | 75 | | 37.685 | 48.725 | 17.730 | 1.00 | 25.10 | C |
| ATOM | 1190 | O | SER | B | 75 | | 36.652 | 48.067 | 17.907 | 1.00 | 24.91 | O |
| ATOM | 1191 | CB | SER | B | 75 | | 39.303 | 47.271 | 19.024 | 1.00 | 28.52 | C |
| ATOM | 1192 | OG | SER | B | 75 | | 39.792 | 48.195 | 19.963 | 1.00 | 29.33 | O |
| ATOM | 1193 | N | TRP | B | 76 | | 37.617 | 50.028 | 17.513 | 1.00 | 23.24 | N |
| ATOM | 1194 | CA | TRP | B | 76 | | 36.292 | 50.566 | 17.397 | 1.00 | 24.27 | C |
| ATOM | 1195 | C | TRP | B | 76 | | 35.740 | 50.438 | 15.992 | 1.00 | 24.51 | C |
| ATOM | 1196 | O | TRP | B | 76 | | 36.286 | 51.072 | 15.095 | 1.00 | 25.35 | O |
| ATOM | 1197 | CB | TRP | B | 76 | | 36.232 | 52.024 | 17.834 | 1.00 | 23.90 | C |
| ATOM | 1198 | CG | TRP | B | 76 | | 36.239 | 52.226 | 19.315 | 1.00 | 27.11 | C |
| ATOM | 1199 | CD1 | TRP | B | 76 | | 37.255 | 52.737 | 20.110 | 1.00 | 32.19 | C |
| ATOM | 1200 | CD2 | TRP | B | 76 | | 35.141 | 51.957 | 20.181 | 1.00 | 29.88 | C |
| ATOM | 1201 | NE1 | TRP | B | 76 | | 36.819 | 52.779 | 21.437 | 1.00 | 32.88 | N |
| ATOM | 1202 | CE2 | TRP | B | 76 | | 35.534 | 52.318 | 21.498 | 1.00 | 33.31 | C |
| ATOM | 1203 | CE3 | TRP | B | 76 | | 33.864 | 51.418 | 19.969 | 1.00 | 30.98 | C |
| ATOM | 1204 | CZ2 | TRP | B | 76 | | 34.681 | 52.158 | 22.603 | 1.00 | 34.82 | C |
| ATOM | 1205 | CZ3 | TRP | B | 76 | | 33.006 | 51.273 | 21.056 | 1.00 | 35.80 | C |
| ATOM | 1206 | CH2 | TRP | B | 76 | | 33.436 | 51.645 | 22.361 | 1.00 | 36.04 | C |
| ATOM | 1207 | N | LEU | B | 77 | | 34.663 | 49.674 | 15.793 | 1.00 | 22.86 | N |
| ATOM | 1208 | CA | LEU | B | 77 | | 34.081 | 49.584 | 14.428 | 1.00 | 23.62 | C |
| ATOM | 1209 | C | LEU | B | 77 | | 32.887 | 50.496 | 14.309 | 1.00 | 23.28 | C |
| ATOM | 1210 | O | LEU | B | 77 | | 31.933 | 50.416 | 15.093 | 1.00 | 24.79 | O |
| ATOM | 1211 | CB | LEU | B | 77 | | 33.570 | 48.146 | 14.124 | 1.00 | 23.33 | C |
| ATOM | 1212 | CG | LEU | B | 77 | | 34.700 | 47.108 | 14.276 | 1.00 | 25.26 | C |
| ATOM | 1213 | CD1 | LEU | B | 77 | | 34.130 | 45.686 | 13.934 | 1.00 | 28.56 | C |
| ATOM | 1214 | CD2 | LEU | B | 77 | | 35.933 | 47.475 | 13.468 | 1.00 | 25.55 | C |
| ATOM | 1215 | N | ALA | B | 78 | | 32.884 | 51.401 | 13.322 | 1.00 | 22.52 | N |
| ATOM | 1216 | CA | ALA | B | 78 | | 31.701 | 52.206 | 13.030 | 1.00 | 21.74 | C |
| ATOM | 1217 | C | ALA | B | 78 | | 30.974 | 51.468 | 11.873 | 1.00 | 22.28 | C |
| ATOM | 1218 | O | ALA | B | 78 | | 31.575 | 51.122 | 10.850 | 1.00 | 21.32 | O |
| ATOM | 1219 | CB | ALA | B | 78 | | 32.201 | 53.603 | 12.468 | 1.00 | 21.36 | C |
| ATOM | 1220 | N | VAL | B | 79 | | 29.694 | 51.226 | 12.079 | 1.00 | 21.30 | N |
| ATOM | 1221 | CA | VAL | B | 79 | | 28.887 | 50.409 | 11.153 | 1.00 | 23.40 | C |
| ATOM | 1222 | C | VAL | B | 79 | | 27.674 | 51.174 | 10.722 | 1.00 | 21.63 | C |
| ATOM | 1223 | O | VAL | B | 79 | | 26.948 | 51.757 | 11.558 | 1.00 | 22.85 | O |
| ATOM | 1224 | CB | VAL | B | 79 | | 28.430 | 49.120 | 11.970 | 1.00 | 23.69 | C |
| ATOM | 1225 | CG1 | VAL | B | 79 | | 27.605 | 48.237 | 11.064 | 1.00 | 26.16 | C |
| ATOM | 1226 | CG2 | VAL | B | 79 | | 29.736 | 48.349 | 12.370 | 1.00 | 26.76 | C |
| ATOM | 1227 | N | THR | B | 80 | | 27.364 | 51.175 | 9.424 | 1.00 | 23.17 | N |
| ATOM | 1228 | CA | THR | B | 80 | | 26.200 | 51.836 | 8.866 | 1.00 | 22.85 | C |
| ATOM | 1229 | C | THR | B | 80 | | 25.477 | 50.769 | 8.037 | 1.00 | 24.64 | C |
| ATOM | 1230 | O | THR | B | 80 | | 26.138 | 49.965 | 7.344 | 1.00 | 25.88 | O |
| ATOM | 1231 | CB | THR | B | 80 | | 26.622 | 53.017 | 7.965 | 1.00 | 24.88 | C |
| ATOM | 1232 | OG1 | THR | B | 80 | | 26.855 | 54.173 | 8.812 | 1.00 | 25.12 | O |
| ATOM | 1233 | CG2 | THR | B | 80 | | 25.586 | 53.380 | 6.901 | 1.00 | 27.18 | C |
| ATOM | 1234 | N | VAL | B | 81 | | 24.157 | 50.748 | 8.175 | 1.00 | 25.24 | N |
| ATOM | 1235 | CA | VAL | B | 81 | | 23.330 | 49.830 | 7.371 | 1.00 | 26.84 | C |
| ATOM | 1236 | C | VAL | B | 81 | | 22.335 | 50.668 | 6.601 | 1.00 | 26.84 | C |
| ATOM | 1237 | O | VAL | B | 81 | | 21.627 | 51.496 | 7.195 | 1.00 | 26.86 | O |
| ATOM | 1238 | CB | VAL | B | 81 | | 22.511 | 48.824 | 8.255 | 1.00 | 28.12 | C |
| ATOM | 1239 | CG1 | VAL | B | 81 | | 21.882 | 47.785 | 7.277 | 1.00 | 28.56 | C |
| ATOM | 1240 | CG2 | VAL | B | 81 | | 23.435 | 48.053 | 9.153 | 1.00 | 29.38 | C |
| ATOM | 1241 | N | ASP | B | 82 | | 22.250 | 50.472 | 5.294 | 1.00 | 28.65 | N |
| ATOM | 1242 | CA | ASP | B | 82 | | 21.211 | 51.157 | 4.516 | 1.00 | 30.70 | C |
| ATOM | 1243 | C | ASP | B | 82 | | 19.888 | 50.425 | 4.742 | 1.00 | 32.16 | C |
| ATOM | 1244 | O | ASP | B | 82 | | 19.823 | 49.223 | 4.520 | 1.00 | 31.73 | O |
| ATOM | 1245 | CB | ASP | B | 82 | | 21.577 | 51.113 | 3.040 | 1.00 | 31.38 | C |
| ATOM | 1246 | CG | ASP | B | 82 | | 20.465 | 51.699 | 2.167 | 1.00 | 35.60 | C |
| ATOM | 1247 | OD1 | ASP | B | 82 | | 20.040 | 52.851 | 2.435 | 1.00 | 38.90 | O |
| ATOM | 1248 | OD2 | ASP | B | 82 | | 20.031 | 50.995 | 1.244 | 1.00 | 41.91 | O |
| ATOM | 1249 | N | THR | B | 83 | | 18.846 | 51.130 | 5.161 | 1.00 | 32.60 | N |
| ATOM | 1250 | CA | THR | B | 83 | | 17.607 | 50.431 | 5.575 | 1.00 | 34.81 | C |
| ATOM | 1251 | C | THR | B | 83 | | 16.868 | 49.781 | 4.408 | 1.00 | 35.27 | C |
| ATOM | 1252 | O | THR | B | 83 | | 16.218 | 48.752 | 4.604 | 1.00 | 37.07 | O |
| ATOM | 1253 | CB | THR | B | 83 | | 16.639 | 51.348 | 6.338 | 1.00 | 33.48 | C |
| ATOM | 1254 | OG1 | THR | B | 83 | | 16.296 | 52.473 | 5.525 | 1.00 | 34.78 | O |
| ATOM | 1255 | CG2 | THR | B | 83 | | 17.210 | 51.874 | 7.637 | 1.00 | 35.59 | C |
| ATOM | 1256 | N | ALA | B | 84 | | 16.966 | 50.323 | 3.206 | 1.00 | 36.17 | N |
| ATOM | 1257 | CA | ALA | B | 84 | | 16.235 | 49.744 | 2.060 | 1.00 | 36.96 | C |
| ATOM | 1258 | C | ALA | B | 84 | | 16.931 | 48.540 | 1.429 | 1.00 | 37.79 | C |
| ATOM | 1259 | O | ALA | B | 84 | | 16.276 | 47.630 | 0.909 | 1.00 | 38.97 | O |
| ATOM | 1260 | CB | ALA | B | 84 | | 16.046 | 50.789 | 0.982 | 1.00 | 36.47 | C |
| ATOM | 1261 | N | SER | B | 85 | | 18.259 | 48.524 | 1.436 | 1.00 | 36.43 | N |
| ATOM | 1262 | CA | SER | B | 85 | | 18.959 | 47.480 | 0.727 | 1.00 | 35.64 | C |
| ATOM | 1263 | C | SER | B | 85 | | 19.598 | 46.506 | 1.686 | 1.00 | 34.73 | C |
| ATOM | 1264 | O | SER | B | 85 | | 20.069 | 45.471 | 1.210 | 1.00 | 34.63 | O |
| ATOM | 1265 | CB | SER | B | 85 | | 20.004 | 48.124 | -0.184 | 1.00 | 35.41 | C |
| ATOM | 1266 | OG | SER | B | 85 | | 21.092 | 48.609 | 0.642 | 1.00 | 39.33 | O |
| ATOM | 1267 | N | SER | B | 86 | | 19.613 | 46.799 | 2.998 | 1.00 | 33.56 | N |
| ATOM | 1268 | CA | SER | B | 86 | | 20.440 | 46.086 | 3.992 | 1.00 | 31.82 | C |
| ATOM | 1269 | C | SER | B | 86 | | 21.980 | 46.085 | 3.800 | 1.00 | 31.10 | C |
| ATOM | 1270 | O | SER | B | 86 | | 22.669 | 45.331 | 4.499 | 1.00 | 29.72 | O |
| ATOM | 1271 | CB | SER | B | 86 | | 20.003 | 44.611 | 4.187 | 1.00 | 33.90 | C |
| ATOM | 1272 | OG | SER | B | 86 | | 18.703 | 44.626 | 4.780 | 1.00 | 34.95 | O |
| ATOM | 1273 | N | ALA | B | 87 | | 22.518 | 46.888 | 2.878 | 1.00 | 29.90 | N |
| ATOM | 1274 | CA | ALA | B | 87 | | 23.968 | 46.902 | 2.641 | 1.00 | 28.58 | C |
| ATOM | 1275 | C | ALA | B | 87 | | 24.673 | 47.474 | 3.884 | 1.00 | 26.88 | C |
| ATOM | 1276 | O | ALA | B | 87 | | 24.107 | 48.363 | 4.503 | 1.00 | 27.55 | O |
| ATOM | 1277 | CB | ALA | B | 87 | | 24.256 | 47.729 | 1.391 | 1.00 | 29.76 | C |
| ATOM | 1278 | N | ILE | B | 88 | | 25.862 | 46.978 | 4.208 | 1.00 | 26.41 | N |
| ATOM | 1279 | CA | ILE | B | 88 | | 26.628 | 47.390 | 5.396 | 1.00 | 26.89 | C |
| ATOM | 1280 | C | ILE | B | 88 | | 27.918 | 48.065 | 4.943 | 1.00 | 24.50 | C |
| ATOM | 1281 | O | ILE | B | 88 | | 28.558 | 47.629 | 3.975 | 1.00 | 24.10 | O |
| ATOM | 1282 | CB | ILE | B | 88 | | 27.053 | 46.113 | 6.173 | 1.00 | 27.78 | C |
| ATOM | 1283 | CG1 | ILE | B | 88 | | 25.799 | 45.314 | 6.548 | 1.00 | 30.96 | C |
| ATOM | 1284 | CG2 | ILE | B | 88 | | 27.972 | 46.405 | 7.324 | 1.00 | 30.55 | C |
| ATOM | 1285 | CD1 | ILE | B | 88 | | 26.127 | 43.948 | 7.242 | 1.00 | 33.93 | C |
| ATOM | 1286 | N | VAL | B | 89 | | 28.325 | 49.122 | 5.650 | 1.00 | 23.84 | N |
| ATOM | 1287 | CA | VAL | B | 89 | | 29.656 | 49.678 | 5.441 | 1.00 | 22.78 | C |
| ATOM | 1288 | C | VAL | B | 89 | | 30.277 | 49.732 | 6.852 | 1.00 | 21.00 | C |
| ATOM | 1289 | O | VAL | B | 89 | | 29.611 | 50.178 | 7.779 | 1.00 | 21.51 | O |
| ATOM | 1290 | CB | VAL | B | 89 | | 29.618 | 51.147 | 4.867 | 1.00 | 22.55 | C |
| ATOM | 1291 | CG1 | VAL | B | 89 | | 31.004 | 51.766 | 4.828 | 1.00 | 22.15 | C |
| ATOM | 1292 | CG2 | VAL | B | 89 | | 29.147 | 51.123 | 3.401 | 1.00 | 25.80 | C |
| ATOM | 1293 | N | VAL | B | 90 | | 31.514 | 49.269 | 6.948 | 1.00 | 20.89 | N |
| ATOM | 1294 | CA | VAL | B | 90 | | 32.187 | 49.274 | 8.248 | 1.00 | 21.06 | C |
| ATOM | 1295 | C | VAL | B | 90 | | 33.572 | 49.907 | 8.087 | 1.00 | 21.34 | C |
| ATOM | 1296 | O | VAL | B | 90 | | 34.297 | 49.626 | 7.099 | 1.00 | 22.15 | O |
| ATOM | 1297 | CB | VAL | B | 90 | | 32.296 | 47.819 | 8.837 | 1.00 | 20.57 | C |
| ATOM | 1298 | CG1 | VAL | B | 90 | | 33.027 | 46.850 | 7.925 | 1.00 | 23.85 | C |
| ATOM | 1299 | CG2 | VAL | B | 90 | | 32.999 | 47.871 | 10.236 | 1.00 | 20.49 | C |
| ATOM | 1300 | N | THR | B | 91 | | 33.956 | 50.772 | 9.036 | 1.00 | 20.80 | N |
| ATOM | 1301 | CA | THR | B | 91 | | 35.333 | 51.212 | 9.088 | 1.00 | 21.25 | C |
| ATOM | 1302 | C | THR | B | 91 | | 35.880 | 51.047 | 10.501 | 1.00 | 21.05 | C |
| ATOM | 1303 | O | THR | B | 91 | | 35.063 | 51.076 | 11.446 | 1.00 | 22.65 | O |
| ATOM | 1304 | CB | THR | B | 91 | | 35.437 | 52.716 | 8.736 | 1.00 | 21.00 | C |
| ATOM | 1305 | OG1 | THR | B | 91 | | 34.511 | 53.494 | 9.518 | 1.00 | 20.99 | O |
| ATOM | 1306 | CG2 | THR | B | 91 | | 35.033 | 52.909 | 7.273 | 1.00 | 22.87 | C |
| ATOM | 1307 | N | GLN | B | 92 | | 37.197 | 50.979 | 10.655 | 1.00 | 20.89 | N |
| ATOM | 1308 | CA | GLN | B | 92 | | 37.771 | 50.778 | 12.006 | 1.00 | 22.12 | C |
| ATOM | 1309 | C | GLN | B | 92 | | 38.561 | 52.045 | 12.441 | 1.00 | 22.00 | C |
| ATOM | 1310 | O | GLN | B | 92 | | 39.027 | 52.832 | 11.592 | 1.00 | 21.32 | O |
| ATOM | 1311 | CB | GLN | B | 92 | | 38.619 | 49.484 | 12.116 | 1.00 | 23.27 | C |
| ATOM | 1312 | CG | GLN | B | 92 | | 39.874 | 49.501 | 11.280 | 1.00 | 25.26 | C |
| ATOM | 1313 | CD | GLN | B | 92 | | 40.801 | 48.271 | 11.390 | 1.00 | 23.33 | C |
| ATOM | 1314 | OE1 | GLN | B | 92 | | 41.271 | 47.785 | 10.362 | 1.00 | 24.67 | O |
| ATOM | 1315 | NE2 | GLN | B | 92 | | 41.157 | 47.872 | 12.628 | 1.00 | 23.62 | N |
| ATOM | 1316 | N | HIS | B | 93 | | 38.606 | 52.257 | 13.773 | 1.00 | 21.70 | N |
| ATOM | 1317 | CA | HIS | B | 93 | | 39.086 | 53.501 | 14.370 | 1.00 | 22.93 | C |
| ATOM | 1318 | C | HIS | B | 93 | | 39.734 | 53.179 | 15.725 | 1.00 | 24.74 | C |
| ATOM | 1319 | O | HIS | B | 93 | | 39.281 | 52.286 | 16.424 | 1.00 | 24.81 | O |
| ATOM | 1320 | CB | HIS | B | 93 | | 37.919 | 54.459 | 14.581 | 1.00 | 20.94 | C |
| ATOM | 1321 | CG | HIS | B | 93 | | 37.171 | 54.743 | 13.318 | 1.00 | 23.30 | C |
| ATOM | 1322 | ND1 | HIS | B | 93 | | 37.592 | 55.712 | 12.418 | 1.00 | 23.71 | N |
| ATOM | 1323 | CD2 | HIS | B | 93 | | 36.072 | 54.155 | 12.778 | 1.00 | 21.52 | C |
| ATOM | 1324 | CE1 | HIS | B | 93 | | 36.770 | 55.710 | 11.378 | 1.00 | 23.58 | C |
| ATOM | 1325 | NE2 | HIS | B | 93 | | 35.864 | 54.754 | 11.554 | 1.00 | 22.91 | N |
| ATOM | 1326 | N | GLY | B | 94 | | 40.789 | 53.929 | 16.039 | 1.00 | 25.62 | N |
| ATOM | 1327 | CA | GLY | B | 94 | | 41.381 | 53.804 | 17.375 | 1.00 | 27.16 | C |
| ATOM | 1328 | C | GLY | B | 94 | | 40.623 | 54.456 | 18.506 | 1.00 | 28.68 | C |
| ATOM | 1329 | O | GLY | B | 94 | | 40.874 | 54.054 | 19.653 | 1.00 | 30.92 | O |
| ATOM | 1330 | N | ARG | B | 95 | | 39.792 | 55.476 | 18.266 | 1.00 | 28.37 | N |
| ATOM | 1331 | CA | ARG | B | 95 | | 39.053 | 56.138 | 19.334 | 1.00 | 30.25 | C |
| ATOM | 1332 | C | ARG | B | 95 | | 37.681 | 56.453 | 18.806 | 1.00 | 30.13 | C |
| ATOM | 1333 | O | ARG | B | 95 | | 37.510 | 56.542 | 17.588 | 1.00 | 30.78 | O |
| ATOM | 1334 | CB | ARG | B | 95 | | 39.679 | 57.489 | 19.746 | 1.00 | 31.84 | C |
| ATOM | 1335 | CG | ARG | B | 95 | | 40.949 | 57.338 | 20.596 | 1.00 | 37.47 | C |
| ATOM | 1336 | CD | ARG | B | 95 | | 41.405 | 58.731 | 21.095 | 1.00 | 47.31 | C |
| ATOM | 1337 | NE | ARG | B | 95 | | 42.300 | 58.601 | 22.247 | 1.00 | 55.70 | N |
| ATOM | 1338 | CZ | ARG | B | 95 | | 43.625 | 58.752 | 22.234 | 1.00 | 58.29 | C |
| ATOM | 1339 | NH1 | ARG | B | 95 | | 44.286 | 59.066 | 21.124 | 1.00 | 60.00 | N |
| ATOM | 1340 | NH2 | ARG | B | 95 | | 44.293 | 58.590 | 23.367 | 1.00 | 59.70 | N |
| ATOM | 1341 | N | VAL | B | 96 | | 36.704 | 56.575 | 19.690 | 1.00 | 29.26 | N |
| ATOM | 1342 | CA | VAL | B | 96 | | 35.365 | 56.877 | 19.241 | 1.00 | 30.59 | C |
| ATOM | 1343 | C | VAL | B | 96 | | 35.193 | 58.373 | 19.376 | 1.00 | 30.80 | C |
| ATOM | 1344 | O | VAL | B | 96 | | 34.936 | 58.878 | 20.483 | 1.00 | 33.20 | O |
| ATOM | 1345 | CB | VAL | B | 96 | | 34.252 | 56.170 | 20.029 | 1.00 | 32.16 | C |
| ATOM | 1346 | CG1 | VAL | B | 96 | | 34.117 | 54.786 | 19.529 | 1.00 | 34.99 | C |
| ATOM | 1347 | CG2 | VAL | B | 96 | | 34.669 | 56.033 | 21.453 | 1.00 | 31.60 | C |
| ATOM | 1348 | N | THR | B | 97 | | 35.419 | 59.109 | 18.300 | 1.00 | 28.27 | N |
| ATOM | 1349 | CA | THR | B | 97 | | 35.287 | 60.570 | 18.361 | 1.00 | 26.48 | C |
| ATOM | 1350 | C | THR | B | 97 | | 34.365 | 61.094 | 17.262 | 1.00 | 24.89 | C |
| ATOM | 1351 | O | THR | B | 97 | | 33.942 | 60.335 | 16.388 | 1.00 | 22.12 | O |
| ATOM | 1352 | CB | THR | B | 97 | | 36.616 | 61.258 | 18.103 | 1.00 | 26.89 | C |
| ATOM | 1353 | OG1 | THR | B | 97 | | 37.086 | 60.856 | 16.799 | 1.00 | 27.31 | O |
| ATOM | 1354 | CG2 | THR | B | 97 | | 37.676 | 60.780 | 19.137 | 1.00 | 30.67 | C |
| ATOM | 1355 | N | SER | B | 98 | | 34.056 | 62.394 | 17.349 | 1.00 | 22.20 | N |
| ATOM | 1356 | CA | SER | B | 98 | | 33.164 | 62.986 | 16.363 | 1.00 | 23.56 | C |
| ATOM | 1357 | C | SER | B | 98 | | 33.837 | 62.845 | 14.996 | 1.00 | 22.48 | C |
| ATOM | 1358 | O | SER | B | 98 | | 33.158 | 62.551 | 13.998 | 1.00 | 23.19 | O |
| ATOM | 1359 | CB | SER | B | 98 | | 32.946 | 64.471 | 16.704 | 1.00 | 24.77 | C |
| ATOM | 1360 | OG | SER | B | 98 | | 32.146 | 64.581 | 17.882 | 1.00 | 28.14 | O |
| ATOM | 1361 | N | VAL | B | 99 | | 35.140 | 63.055 | 14.939 | 1.00 | 22.53 | N |
| ATOM | 1362 | CA | VAL | B | 99 | | 35.831 | 62.925 | 13.629 | 1.00 | 22.26 | C |
| ATOM | 1363 | C | VAL | B | 99 | | 35.782 | 61.517 | 13.099 | 1.00 | 22.56 | C |
| ATOM | 1364 | O | VAL | B | 99 | | 35.551 | 61.312 | 11.887 | 1.00 | 20.63 | O |
| ATOM | 1365 | CB | VAL | B | 99 | | 37.266 | 63.429 | 13.773 | 1.00 | 24.71 | C |
| ATOM | 1366 | CG1 | VAL | B | 99 | | 38.105 | 63.082 | 12.556 | 1.00 | 23.53 | C |
| ATOM | 1367 | CG2 | VAL | B | 99 | | 37.188 | 64.933 | 13.956 | 1.00 | 26.48 | C |
| ATOM | 1368 | N | ALA | B | 100 | | 35.965 | 60.517 | 13.977 | 1.00 | 21.38 | N |
| ATOM | 1369 | CA | ALA | B | 100 | | 35.894 | 59.118 | 13.477 | 1.00 | 21.43 | C |
| ATOM | 1370 | C | ALA | B | 100 | | 34.501 | 58.894 | 12.867 | 1.00 | 21.14 | C |
| ATOM | 1371 | O | ALA | B | 100 | | 34.409 | 58.257 | 11.778 | 1.00 | 20.24 | O |
| ATOM | 1372 | CB | ALA | B | 100 | | 36.118 | 58.109 | 14.630 | 1.00 | 22.68 | C |
| ATOM | 1373 | N | ALA | B | 101 | | 33.423 | 59.381 | 13.479 | 1.00 | 19.59 | N |
| ATOM | 1374 | CA | ALA | B | 101 | | 32.093 | 59.170 | 12.870 | 1.00 | 20.32 | C |
| ATOM | 1375 | C | ALA | B | 101 | | 31.974 | 59.870 | 11.515 | 1.00 | 21.45 | C |
| ATOM | 1376 | O | ALA | B | 101 | | 31.330 | 59.409 | 10.575 | 1.00 | 21.60 | O |
| ATOM | 1377 | CB | ALA | B | 101 | | 30.927 | 59.816 | 13.713 | 1.00 | 21.05 | C |
| ATOM | 1378 | N | GLN | B | 102 | | 32.502 | 61.091 | 11.453 | 1.00 | 19.92 | N |
| ATOM | 1379 | CA | GLN | B | 102 | | 32.450 | 61.818 | 10.172 | 1.00 | 19.58 | C |
| ATOM | 1380 | C | GLN | B | 102 | | 33.289 | 61.178 | 9.074 | 1.00 | 18.59 | C |
| ATOM | 1381 | O | GLN | B | 102 | | 32.893 | 61.196 | 7.904 | 1.00 | 20.46 | O |
| ATOM | 1382 | CB | GLN | B | 102 | | 33.037 | 63.254 | 10.438 | 1.00 | 17.86 | C |
| ATOM | 1383 | CG | GLN | B | 102 | | 32.030 | 63.943 | 11.370 | 1.00 | 20.17 | C |
| ATOM | 1384 | CD | GLN | B | 102 | | 32.587 | 65.173 | 12.041 | 1.00 | 25.97 | C |
| ATOM | 1385 | OE1 | GLN | B | 102 | | 33.520 | 65.802 | 11.518 | 1.00 | 23.19 | O |
| ATOM | 1386 | NE2 | GLN | B | 102 | | 32.098 | 65.455 | 13.258 | 1.00 | 27.05 | N |
| ATOM | 1387 | N | HIS | B | 103 | | 34.465 | 60.622 | 9.385 | 1.00 | 18.86 | N |
| ATOM | 1388 | CA | HIS | B | 103 | | 35.218 | 59.955 | 8.331 | 1.00 | 17.64 | C |
| ATOM | 1389 | C | HIS | B | 103 | | 34.577 | 58.622 | 7.920 | 1.00 | 20.69 | C |
| ATOM | 1390 | O | HIS | B | 103 | | 34.679 | 58.244 | 6.745 | 1.00 | 19.76 | O |
| ATOM | 1391 | CB | HIS | B | 103 | | 36.649 | 59.726 | 8.838 | 1.00 | 19.17 | C |
| ATOM | 1392 | CG | HIS | B | 103 | | 37.480 | 60.985 | 8.894 | 1.00 | 19.09 | C |
| ATOM | 1393 | ND1 | HIS | B | 103 | | 38.817 | 61.004 | 9.254 | 1.00 | 19.36 | N |
| ATOM | 1394 | CD2 | HIS | B | 103 | | 37.148 | 62.259 | 8.582 | 1.00 | 21.78 | C |
| ATOM | 1395 | CE1 | HIS | B | 103 | | 39.263 | 62.266 | 9.219 | 1.00 | 19.96 | C |
| ATOM | 1396 | NE2 | HIS | B | 103 | | 38.261 | 63.054 | 8.820 | 1.00 | 19.02 | N |
| ATOM | 1397 | N | HIS | B | 104 | | 33.968 | 57.961 | 8.901 | 1.00 | 20.19 | N |
| ATOM | 1398 | CA | HIS | B | 104 | | 33.160 | 56.722 | 8.585 | 1.00 | 19.22 | C |
| ATOM | 1399 | C | HIS | B | 104 | | 32.066 | 57.111 | 7.584 | 1.00 | 20.09 | C |
| ATOM | 1400 | O | HIS | B | 104 | | 31.906 | 56.441 | 6.524 | 1.00 | 19.87 | O |
| ATOM | 1401 | CB | HIS | B | 104 | | 32.496 | 56.252 | 9.893 | 1.00 | 18.67 | C |
| ATOM | 1402 | CG | HIS | B | 104 | | 31.385 | 55.262 | 9.631 | 1.00 | 18.43 | C |
| ATOM | 1403 | ND1 | HIS | B | 104 | | 31.682 | 53.997 | 9.170 | 1.00 | 21.09 | N |
| ATOM | 1404 | CD2 | HIS | B | 104 | | 30.041 | 55.404 | 9.571 | 1.00 | 22.01 | C |
| ATOM | 1405 | CE1 | HIS | B | 104 | | 30.542 | 53.364 | 8.943 | 1.00 | 21.80 | C |
| ATOM | 1406 | NE2 | HIS | B | 104 | | 29.536 | 54.186 | 9.193 | 1.00 | 21.50 | N |
| ATOM | 1407 | N | TRP | B | 105 | | 31.284 | 58.151 | 7.886 | 1.00 | 18.81 | N |
| ATOM | 1408 | CA | TRP | B | 105 | | 30.194 | 58.532 | 7.003 | 1.00 | 20.10 | C |
| ATOM | 1409 | C | TRP | B | 105 | | 30.689 | 58.975 | 5.628 | 1.00 | 21.40 | C |
| ATOM | 1410 | O | TRP | B | 105 | | 30.017 | 58.684 | 4.639 | 1.00 | 21.13 | O |
| ATOM | 1411 | CB | TRP | B | 105 | | 29.229 | 59.560 | 7.567 | 1.00 | 20.56 | C |
| ATOM | 1412 | CG | TRP | B | 105 | | 28.343 | 59.076 | 8.679 | 1.00 | 19.00 | C |
| ATOM | 1413 | CD1 | TRP | B | 105 | | 27.638 | 57.872 | 8.708 | 1.00 | 22.04 | C |
| ATOM | 1414 | CD2 | TRP | B | 105 | | 27.986 | 59.800 | 9.855 | 1.00 | 20.22 | C |
| ATOM | 1415 | NE1 | TRP | B | 105 | | 26.923 | 57.804 | 9.867 | 1.00 | 22.51 | N |
| ATOM | 1416 | CE2 | TRP | B | 105 | | 27.096 | 58.973 | 10.581 | 1.00 | 23.01 | C |
| ATOM | 1417 | CE3 | TRP | B | 105 | | 28.332 | 61.061 | 10.374 | 1.00 | 22.66 | C |
| ATOM | 1418 | CZ2 | TRP | B | 105 | | 26.579 | 59.362 | 11.816 | 1.00 | 25.94 | C |
| ATOM | 1419 | CZ3 | TRP | B | 105 | | 27.801 | 61.463 | 11.607 | 1.00 | 26.71 | C |
| ATOM | 1420 | CH2 | TRP | B | 105 | | 26.917 | 60.618 | 12.292 | 1.00 | 25.59 | C |
| ATOM | 1421 | N | ALA | B | 106 | | 31.821 | 59.679 | 5.559 | 1.00 | 19.82 | N |
| ATOM | 1422 | CA | ALA | B | 106 | | 32.342 | 59.991 | 4.235 | 1.00 | 21.82 | C |
| ATOM | 1423 | C | ALA | B | 106 | | 32.677 | 58.736 | 3.448 | 1.00 | 21.48 | C |
| ATOM | 1424 | O | ALA | B | 106 | | 32.439 | 58.678 | 2.215 | 1.00 | 21.15 | O |
| ATOM | 1425 | CB | ALA | B | 106 | | 33.663 | 60.863 | 4.329 | 1.00 | 20.46 | C |
| ATOM | 1426 | N | THR | B | 107 | | 33.227 | 57.720 | 4.114 | 1.00 | 20.38 | N |
| ATOM | 1427 | CA | THR | B | 107 | | 33.468 | 56.438 | 3.466 | 1.00 | 20.73 | C |
| ATOM | 1428 | C | THR | B | 107 | | 32.149 | 55.817 | 2.994 | 1.00 | 19.78 | C |
| ATOM | 1429 | O | THR | B | 107 | | 32.095 | 55.311 | 1.824 | 1.00 | 22.30 | O |
| ATOM | 1430 | CB | THR | B | 107 | | 34.220 | 55.454 | 4.399 | 1.00 | 21.99 | C |
| ATOM | 1431 | OG1 | THR | B | 107 | | 35.476 | 56.080 | 4.768 | 1.00 | 22.08 | O |
| ATOM | 1432 | CG2 | THR | B | 107 | | 34.629 | 54.135 | 3.690 | 1.00 | 23.60 | C |
| ATOM | 1433 | N | ALA | B | 108 | | 31.158 | 55.797 | 3.877 | 1.00 | 19.68 | N |
| ATOM | 1434 | CA | ALA | B | 108 | | 29.877 | 55.174 | 3.439 | 1.00 | 20.77 | C |
| ATOM | 1435 | C | ALA | B | 108 | | 29.243 | 55.951 | 2.280 | 1.00 | 21.36 | C |
| ATOM | 1436 | O | ALA | B | 108 | | 28.628 | 55.318 | 1.387 | 1.00 | 23.62 | O |
| ATOM | 1437 | CB | ALA | B | 108 | | 28.962 | 54.942 | 4.661 | 1.00 | 21.64 | C |
| ATOM | 1438 | N | ILE | B | 109 | | 29.403 | 57.289 | 2.251 | 1.00 | 20.98 | N |
| ATOM | 1439 | CA | ILE | B | 109 | | 28.883 | 58.059 | 1.100 | 1.00 | 21.28 | C |
| ATOM | 1440 | C | ILE | B | 109 | | 29.608 | 57.670 | -0.173 | 1.00 | 22.53 | C |
| ATOM | 1441 | O | ILE | B | 109 | | 28.970 | 57.527 | -1.276 | 1.00 | 24.80 | O |
| ATOM | 1442 | CB | ILE | B | 109 | | 28.923 | 59.611 | 1.354 | 1.00 | 21.06 | C |
| ATOM | 1443 | CG1 | ILE | B | 109 | | 28.002 | 60.070 | 2.511 | 1.00 | 21.85 | C |
| ATOM | 1444 | CG2 | ILE | B | 109 | | 28.584 | 60.351 | -0.020 | 1.00 | 21.68 | C |
| ATOM | 1445 | CD1 | ILE | B | 109 | | 28.249 | 61.546 | 2.964 | 1.00 | 22.00 | C |
| ATOM | 1446 | N | ALA | B | 110 | | 30.941 | 57.551 | -0.065 | 1.00 | 21.98 | N |
| ATOM | 1447 | CA | ALA | B | 110 | | 31.743 | 57.068 | -1.205 | 1.00 | 22.85 | C |
| ATOM | 1448 | C | ALA | B | 110 | | 31.306 | 55.696 | -1.729 | 1.00 | 24.75 | C |
| ATOM | 1449 | O | ALA | B | 110 | | 31.351 | 55.461 | -2.941 | 1.00 | 25.82 | O |
| ATOM | 1450 | CB | ALA | B | 110 | | 33.249 | 57.109 | -0.913 | 1.00 | 22.37 | C |
| ATOM | 1451 | N | VAL | B | 111 | | 30.876 | 54.793 | -0.852 | 1.00 | 25.47 | N |
| ATOM | 1452 | CA | VAL | B | 111 | | 30.499 | 53.439 | -1.300 | 1.00 | 26.90 | C |
| ATOM | 1453 | C | VAL | B | 111 | | 29.034 | 53.421 | -1.792 | 1.00 | 27.16 | C |
| ATOM | 1454 | O | VAL | B | 111 | | 28.727 | 52.813 | -2.838 | 1.00 | 29.74 | O |
| ATOM | 1455 | CB | VAL | B | 111 | | 30.622 | 52.470 | -0.085 | 1.00 | 27.18 | C |
| ATOM | 1456 | CG1 | VAL | B | 111 | | 29.983 | 51.033 | -0.338 | 1.00 | 28.93 | C |
| ATOM | 1457 | CG2 | VAL | B | 111 | | 32.120 | 52.355 | 0.296 | 1.00 | 27.28 | C |
| ATOM | 1458 | N | LEU | B | 112 | | 28.130 | 54.007 | -1.019 | 1.00 | 26.37 | N |
| ATOM | 1459 | CA | LEU | B | 112 | | 26.648 | 53.942 | -1.161 | 1.00 | 27.44 | C |
| ATOM | 1460 | C | LEU | B | 112 | | 25.986 | 55.098 | -1.911 | 1.00 | 28.92 | C |
| ATOM | 1461 | O | LEU | B | 112 | | 24.929 | 54.883 | -2.501 | 1.00 | 29.01 | O |
| ATOM | 1462 | CB | LEU | B | 112 | | 25.956 | 53.766 | 0.208 | 1.00 | 27.69 | C |
| ATOM | 1463 | CG | LEU | B | 112 | | 26.365 | 52.528 | 1.053 | 1.00 | 29.77 | C |
| ATOM | 1464 | CD1 | LEU | B | 112 | | 25.680 | 52.513 | 2.458 | 1.00 | 30.29 | C |
| ATOM | 1465 | CD2 | LEU | B | 112 | | 26.204 | 51.157 | 0.287 | 1.00 | 34.84 | C |
| ATOM | 1466 | N | GLY | B | 113 | | 26.560 | 56.303 | -1.861 | 1.00 | 27.29 | N |
| ATOM | 1467 | CA | GLY | B | 113 | | 25.923 | 57.524 | -2.347 | 1.00 | 28.34 | C |
| ATOM | 1468 | C | GLY | B | 113 | | 25.393 | 58.308 | -1.164 | 1.00 | 27.74 | C |
| ATOM | 1469 | O | GLY | B | 113 | | 25.359 | 57.786 | -0.031 | 1.00 | 28.10 | O |
| ATOM | 1470 | N | ARG | B | 114 | | 24.976 | 59.561 | -1.356 | 1.00 | 28.72 | N |
| ATOM | 1471 | CA | ARG | B | 114 | | 24.564 | 60.396 | -0.203 | 1.00 | 29.39 | C |
| ATOM | 1472 | C | ARG | B | 114 | | 23.173 | 60.053 | 0.305 | 1.00 | 29.81 | C |
| ATOM | 1473 | O | ARG | B | 114 | | 22.262 | 59.895 | -0.498 | 1.00 | 30.08 | O |
| ATOM | 1474 | CB | ARG | B | 114 | | 24.471 | 61.875 | -0.543 | 1.00 | 31.33 | C |
| ATOM | 1475 | CG | ARG | B | 114 | | 25.649 | 62.763 | -0.300 | 1.00 | 39.16 | C |
| ATOM | 1476 | CD | ARG | B | 114 | | 25.690 | 63.740 | -1.476 | 1.00 | 44.75 | C |
| ATOM | 1477 | NE | ARG | B | 114 | | 25.892 | 65.157 | -1.198 | 1.00 | 50.94 | N |
| ATOM | 1478 | CZ | ARG | B | 114 | | 24.890 | 65.941 | -0.819 | 1.00 | 53.54 | C |
| ATOM | 1479 | NH1 | ARG | B | 114 | | 23.693 | 65.378 | -0.662 | 1.00 | 54.94 | N |
| ATOM | 1480 | NH2 | ARG | B | 114 | | 25.075 | 67.236 | -0.581 | 1.00 | 50.99 | N |
| ATOM | 1481 | N | PRO | B | 115 | | 22.983 | 59.937 | 1.627 | 1.00 | 28.77 | N |
| ATOM | 1482 | CA | PRO | B | 115 | | 21.650 | 59.542 | 2.085 | 1.00 | 28.51 | C |
| ATOM | 1483 | C | PRO | B | 115 | | 20.794 | 60.766 | 2.304 | 1.00 | 29.28 | C |
| ATOM | 1484 | O | PRO | B | 115 | | 21.301 | 61.886 | 2.378 | 1.00 | 29.05 | O |
| ATOM | 1485 | CB | PRO | B | 115 | | 21.948 | 58.795 | 3.399 | 1.00 | 28.55 | C |
| ATOM | 1486 | CG | PRO | B | 115 | | 23.219 | 59.622 | 3.976 | 1.00 | 26.68 | C |
| ATOM | 1487 | CD | PRO | B | 115 | | 23.998 | 59.970 | 2.700 | 1.00 | 27.88 | C |
| ATOM | 1488 | N | LYS | B | 116 | | 19.487 | 60.578 | 2.449 | 1.00 | 28.85 | N |
| ATOM | 1489 | CA | LYS | B | 116 | | 18.622 | 61.664 | 2.907 | 1.00 | 29.63 | C |
| ATOM | 1490 | C | LYS | B | 116 | | 18.661 | 61.880 | 4.416 | 1.00 | 28.09 | C |
| ATOM | 1491 | O | LYS | B | 116 | | 18.526 | 63.032 | 4.905 | 1.00 | 27.74 | O |
| ATOM | 1492 | CB | LYS | B | 116 | | 17.179 | 61.254 | 2.530 | 1.00 | 31.29 | C |
| ATOM | 1493 | CG | LYS | B | 116 | | 16.121 | 62.036 | 3.301 | 1.00 | 36.28 | C |
| ATOM | 1494 | CD | LYS | B | 116 | | 14.738 | 61.684 | 2.721 | 1.00 | 44.11 | C |
| ATOM | 1495 | CE | LYS | B | 116 | | 13.874 | 62.934 | 2.795 | 1.00 | 47.55 | C |
| ATOM | 1496 | NZ | LYS | B | 116 | | 12.600 | 62.837 | 2.060 | 1.00 | 49.82 | N |
| ATOM | 1497 | N | ALA | B | 117 | | 18.834 | 60.784 | 5.154 | 1.00 | 26.92 | N |
| ATOM | 1498 | CA | ALA | B | 117 | | 18.748 | 60.832 | 6.625 | 1.00 | 28.00 | C |
| ATOM | 1499 | C | ALA | B | 117 | | 19.579 | 59.726 | 7.271 | 1.00 | 27.17 | C |
| ATOM | 1500 | O | ALA | B | 117 | | 19.774 | 58.690 | 6.639 | 1.00 | 26.94 | O |
| ATOM | 1501 | CB | ALA | B | 117 | | 17.250 | 60.726 | 7.148 | 1.00 | 27.62 | C |
| ATOM | 1502 | N | ILE | B | 118 | | 20.102 | 59.995 | 8.474 | 1.00 | 25.54 | N |
| ATOM | 1503 | CA | ILE | B | 118 | | 20.889 | 59.034 | 9.241 | 1.00 | 24.85 | C |
| ATOM | 1504 | C | ILE | B | 118 | | 20.256 | 59.021 | 10.632 | 1.00 | 26.01 | C |
| ATOM | 1505 | O | ILE | B | 118 | | 20.073 | 60.074 | 11.278 | 1.00 | 27.20 | O |
| ATOM | 1506 | CB | ILE | B | 118 | | 22.390 | 59.444 | 9.364 | 1.00 | 23.89 | C |
| ATOM | 1507 | CG1 | ILE | B | 118 | | 23.070 | 59.364 | 8.001 | 1.00 | 24.09 | C |
| ATOM | 1508 | CG2 | ILE | B | 118 | | 23.146 | 58.511 | 10.338 | 1.00 | 27.19 | C |
| ATOM | 1509 | CD1 | ILE | B | 118 | | 24.582 | 59.872 | 7.991 | 1.00 | 23.63 | C |
| ATOM | 1510 | N | LYS | B | 119 | | 19.967 | 57.811 | 11.100 | 1.00 | 26.80 | N |
| ATOM | 1511 | CA | LYS | B | 119 | | 19.428 | 57.570 | 12.454 | 1.00 | 28.18 | C |
| ATOM | 1512 | C | LYS | B | 119 | | 20.525 | 57.012 | 13.349 | 1.00 | 27.54 | C |
| ATOM | 1513 | O | LYS | B | 119 | | 21.214 | 56.049 | 12.992 | 1.00 | 26.72 | O |
| ATOM | 1514 | CB | LYS | B | 119 | | 18.270 | 56.554 | 12.388 | 1.00 | 30.28 | C |
| ATOM | 1515 | CG | LYS | B | 119 | | 17.233 | 56.985 | 11.338 | 1.00 | 34.66 | C |
| ATOM | 1516 | CD | LYS | B | 119 | | 16.023 | 56.049 | 11.306 | 1.00 | 41.62 | C |
| ATOM | 1517 | CE | LYS | B | 119 | | 14.697 | 56.769 | 11.001 | 1.00 | 48.23 | C |
| ATOM | 1518 | NZ | LYS | B | 119 | | 14.789 | 57.884 | 9.973 | 1.00 | 50.82 | N |
| ATOM | 1519 | N | THR | B | 120 | | 20.715 | 57.622 | 14.519 | 1.00 | 27.39 | N |
| ATOM | 1520 | CA | THR | B | 120 | | 21.687 | 57.106 | 15.469 | 1.00 | 27.32 | C |
| ATOM | 1521 | C | THR | B | 120 | | 21.109 | 57.172 | 16.892 | 1.00 | 27.65 | C |
| ATOM | 1522 | O | THR | B | 120 | | 20.023 | 57.685 | 17.096 | 1.00 | 28.61 | O |
| ATOM | 1523 | CB | THR | B | 120 | | 23.015 | 57.958 | 15.535 | 1.00 | 27.36 | C |
| ATOM | 1524 | OG1 | THR | B | 120 | | 22.673 | 59.230 | 16.114 | 1.00 | 27.02 | O |
| ATOM | 1525 | CG2 | THR | B | 120 | | 23.656 | 58.185 | 14.120 | 1.00 | 27.96 | C |
| ATOM | 1526 | N | ASP | B | 121 | | 21.847 | 56.654 | 17.858 | 1.00 | 29.14 | N |
| ATOM | 1527 | CA | ASP | B | 121 | | 21.502 | 56.791 | 19.272 | 1.00 | 30.42 | C |
| ATOM | 1528 | C | ASP | B | 121 | | 22.040 | 58.135 | 19.807 | 1.00 | 32.23 | C |
| ATOM | 1529 | O | ASP | B | 121 | | 22.468 | 58.988 | 19.024 | 1.00 | 32.42 | O |
| ATOM | 1530 | CB | ASP | B | 121 | | 21.953 | 55.560 | 20.082 | 1.00 | 31.19 | C |
| ATOM | 1531 | CG | ASP | B | 121 | | 23.458 | 55.298 | 20.080 | 1.00 | 28.97 | C |
| ATOM | 1532 | OD1 | ASP | B | 121 | | 24.232 | 56.303 | 20.181 | 1.00 | 31.58 | O |
| ATOM | 1533 | OD2 | ASP | B | 121 | | 23.994 | 54.109 | 20.069 | 1.00 | 28.62 | O |
| ATOM | 1534 | N | ASN | B | 122 | | 22.024 | 58.354 | 21.114 | 1.00 | 32.10 | N |
| ATOM | 1535 | CA | ASN | B | 122 | | 22.472 | 59.629 | 21.668 | 1.00 | 34.12 | C |
| ATOM | 1536 | C | ASN | B | 122 | | 23.887 | 59.651 | 22.166 | 1.00 | 32.63 | C |
| ATOM | 1537 | O | ASN | B | 122 | | 24.210 | 60.458 | 23.034 | 1.00 | 32.85 | O |
| ATOM | 1538 | CB | ASN | B | 122 | | 21.511 | 60.120 | 22.778 | 1.00 | 35.82 | C |
| ATOM | 1539 | CG | ASN | B | 122 | | 20.156 | 60.451 | 22.222 | 1.00 | 42.15 | C |
| ATOM | 1540 | OD1 | ASN | B | 122 | | 19.167 | 59.766 | 22.500 | 1.00 | 49.86 | O |
| ATOM | 1541 | ND2 | ASN | B | 122 | | 20.089 | 61.500 | 21.397 | 1.00 | 50.21 | N |
| ATOM | 1542 | N | GLY | B | 123 | | 24.750 | 58.790 | 21.631 | 1.00 | 32.18 | N |
| ATOM | 1543 | CA | GLY | B | 123 | | 26.152 | 58.815 | 22.028 | 1.00 | 31.96 | C |
| ATOM | 1544 | C | GLY | B | 123 | | 26.712 | 60.213 | 21.748 | 1.00 | 32.39 | C |
| ATOM | 1545 | O | GLY | B | 123 | | 26.256 | 60.858 | 20.804 | 1.00 | 32.32 | O |
| ATOM | 1546 | N | SER | B | 124 | | 27.682 | 60.663 | 22.527 | 1.00 | 32.85 | N |
| ATOM | 1547 | CA | SER | B | 124 | | 28.076 | 62.065 | 22.447 | 1.00 | 36.07 | C |
| ATOM | 1548 | C | SER | B | 124 | | 28.734 | 62.377 | 21.109 | 1.00 | 35.68 | C |
| ATOM | 1549 | O | SER | B | 124 | | 28.609 | 63.495 | 20.632 | 1.00 | 36.75 | O |
| ATOM | 1550 | CB | SER | B | 124 | | 28.958 | 62.461 | 23.620 | 1.00 | 37.13 | C |
| ATOM | 1551 | OG | SER | B | 124 | | 30.151 | 61.721 | 23.438 | 1.00 | 42.48 | O |
| ATOM | 1552 | N | CYS | B | 125 | | 29.349 | 61.389 | 20.469 | 1.00 | 34.89 | N |
| ATOM | 1553 | CA | CYS | B | 125 | | 30.014 | 61.676 | 19.172 | 1.00 | 35.40 | C |
| ATOM | 1554 | C | CYS | B | 125 | | 29.015 | 61.838 | 18.008 | 1.00 | 33.42 | C |
| ATOM | 1555 | O | CYS | B | 125 | | 29.368 | 62.372 | 16.927 | 1.00 | 32.51 | O |
| ATOM | 1556 | CB | CYS | B | 125 | | 31.144 | 60.671 | 18.928 | 1.00 | 36.72 | C |
| ATOM | 1557 | SG | CYS | B | 125 | | 30.595 | 59.215 | 18.085 | 1.00 | 44.67 | S |
| ATOM | 1558 | N | PHE | B | 126 | | 27.767 | 61.414 | 18.238 | 1.00 | 30.08 | N |
| ATOM | 1559 | CA | PHE | B | 126 | | 26.689 | 61.630 | 17.295 | 1.00 | 29.31 | C |
| ATOM | 1560 | C | PHE | B | 126 | | 25.888 | 62.894 | 17.581 | 1.00 | 28.85 | C |
| ATOM | 1561 | O | PHE | B | 126 | | 25.229 | 63.418 | 16.708 | 1.00 | 29.52 | O |
| ATOM | 1562 | CB | PHE | B | 126 | | 25.686 | 60.469 | 17.290 | 1.00 | 28.25 | C |
| ATOM | 1563 | CG | PHE | B | 126 | | 26.254 | 59.188 | 16.882 | 1.00 | 28.27 | C |
| ATOM | 1564 | CD1 | PHE | B | 126 | | 27.036 | 59.094 | 15.742 | 1.00 | 27.22 | C |
| ATOM | 1565 | CD2 | PHE | B | 126 | | 25.986 | 58.032 | 17.651 | 1.00 | 28.72 | C |
| ATOM | 1566 | CE1 | PHE | B | 126 | | 27.578 | 57.874 | 15.353 | 1.00 | 29.30 | C |
| ATOM | 1567 | CE2 | PHE | B | 126 | | 26.506 | 56.785 | 17.281 | 1.00 | 28.21 | C |
| ATOM | 1568 | CZ | PHE | B | 126 | | 27.321 | 56.680 | 16.109 | 1.00 | 31.39 | C |
| ATOM | 1569 | N | THR | B | 127 | | 25.938 | 63.403 | 18.787 | 1.00 | 29.29 | N |
| ATOM | 1570 | CA | THR | B | 127 | | 25.120 | 64.571 | 19.055 | 1.00 | 31.18 | C |
| ATOM | 1571 | C | THR | B | 127 | | 25.978 | 65.823 | 19.325 | 1.00 | 30.26 | C |
| ATOM | 1572 | O | THR | B | 127 | | 25.418 | 66.829 | 19.751 | 1.00 | 31.77 | O |
| ATOM | 1573 | CB | THR | B | 127 | | 24.209 | 64.354 | 20.292 | 1.00 | 31.43 | C |
| ATOM | 1574 | OG1 | THR | B | 127 | | 25.030 | 64.293 | 21.453 | 1.00 | 35.73 | O |
| ATOM | 1575 | CG2 | THR | B | 127 | | 23.327 | 63.108 | 20.135 | 1.00 | 34.92 | C |
| ATOM | 1576 | N | SER | B | 128 | | 27.296 | 65.765 | 19.144 | 1.00 | 29.27 | N |
| ATOM | 1577 | CA | SER | B | 128 | | 28.224 | 66.908 | 19.320 | 1.00 | 29.17 | C |
| ATOM | 1578 | C | SER | B | 128 | | 27.918 | 68.046 | 18.344 | 1.00 | 27.78 | C |
| ATOM | 1579 | O | SER | B | 128 | | 27.278 | 67.874 | 17.285 | 1.00 | 26.60 | O |
| ATOM | 1580 | CB | SER | B | 128 | | 29.700 | 66.482 | 19.156 | 1.00 | 29.31 | C |
| ATOM | 1581 | OG | SER | B | 128 | | 29.875 | 66.065 | 17.777 | 1.00 | 28.49 | O |
| ATOM | 1582 | N | LYS | B | 129 | | 28.343 | 69.252 | 18.742 | 1.00 | 27.09 | N |
| ATOM | 1583 | CA | LYS | B | 129 | | 28.265 | 70.381 | 17.800 | 1.00 | 27.38 | C |
| ATOM | 1584 | C | LYS | B | 129 | | 29.010 | 70.092 | 16.490 | 1.00 | 26.49 | C |
| ATOM | 1585 | O | LYS | B | 129 | | 28.471 | 70.441 | 15.433 | 1.00 | 27.43 | O |
| ATOM | 1586 | CB | LYS | B | 129 | | 28.868 | 71.666 | 18.398 | 1.00 | 28.22 | C |
| ATOM | 1587 | CG | LYS | B | 129 | | 28.548 | 72.908 | 17.534 | 1.00 | 32.76 | C |
| ATOM | 1588 | CD | LYS | B | 129 | | 29.525 | 74.043 | 17.943 | 1.00 | 41.27 | C |
| ATOM | 1589 | CE | LYS | B | 129 | | 29.243 | 74.748 | 19.265 | 1.00 | 48.12 | C |
| ATOM | 1590 | NZ | LYS | B | 129 | | 30.448 | 75.634 | 19.632 | 1.00 | 48.30 | N |
| ATOM | 1591 | N | SER | B | 130 | | 30.188 | 69.471 | 16.557 | 1.00 | 26.01 | N |
| ATOM | 1592 | CA | SER | B | 130 | | 31.050 | 69.195 | 15.390 | 1.00 | 25.25 | C |
| ATOM | 1593 | C | SER | B | 130 | | 30.262 | 68.238 | 14.459 | 1.00 | 25.51 | C |
| ATOM | 1594 | O | SER | B | 130 | | 30.246 | 68.440 | 13.231 | 1.00 | 24.03 | O |
| ATOM | 1595 | CB | SER | B | 130 | | 32.342 | 68.493 | 15.813 | 1.00 | 27.92 | C |
| ATOM | 1596 | OG | SER | B | 130 | | 32.965 | 67.872 | 14.689 | 1.00 | 30.62 | O |
| ATOM | 1597 | N | THR | B | 131 | | 29.646 | 67.191 | 15.024 | 1.00 | 23.95 | N |
| ATOM | 1598 | CA | THR | B | 131 | | 28.817 | 66.296 | 14.174 | 1.00 | 24.51 | C |
| ATOM | 1599 | C | THR | B | 131 | | 27.552 | 66.924 | 13.611 | 1.00 | 24.23 | C |
| ATOM | 1600 | O | THR | B | 131 | | 27.248 | 66.728 | 12.427 | 1.00 | 23.32 | O |
| ATOM | 1601 | CB | THR | B | 131 | | 28.558 | 64.961 | 14.945 | 1.00 | 23.62 | C |
| ATOM | 1602 | OG1 | THR | B | 131 | | 29.804 | 64.319 | 15.074 | 1.00 | 23.96 | O |
| ATOM | 1603 | CG2 | THR | B | 131 | | 27.628 | 64.073 | 14.125 | 1.00 | 26.13 | C |
| ATOM | 1604 | N | ARG | B | 132 | | 26.784 | 67.707 | 14.400 | 1.00 | 24.79 | N |
| ATOM | 1605 | CA | ARG | B | 132 | | 25.647 | 68.488 | 13.919 | 1.00 | 26.68 | C |
| ATOM | 1606 | C | ARG | B | 132 | | 25.965 | 69.413 | 12.728 | 1.00 | 26.08 | C |
| ATOM | 1607 | O | ARG | B | 132 | | 25.260 | 69.382 | 11.722 | 1.00 | 25.46 | O |
| ATOM | 1608 | CB | ARG | B | 132 | | 24.969 | 69.257 | 15.103 | 1.00 | 28.27 | C |
| ATOM | 1609 | CG | ARG | B | 132 | | 23.674 | 70.099 | 14.880 | 1.00 | 37.64 | C |
| ATOM | 1610 | CD | ARG | B | 132 | | 23.351 | 71.112 | 16.062 | 1.00 | 47.23 | C |
| ATOM | 1611 | NE | ARG | B | 132 | | 24.328 | 72.199 | 16.318 | 1.00 | 53.55 | N |
| ATOM | 1612 | CZ | ARG | B | 132 | | 24.664 | 73.169 | 15.452 | 1.00 | 58.18 | C |
| ATOM | 1613 | NH1 | ARG | B | 132 | | 24.136 | 73.227 | 14.230 | 1.00 | 58.82 | N |
| ATOM | 1614 | NH2 | ARG | B | 132 | | 25.554 | 74.107 | 15.773 | 1.00 | 59.65 | N |
| ATOM | 1615 | N | GLU | B | 132 | | 27.067 | 70.142 | 12.840 | 1.00 | 25.73 | N |
| ATOM | 1616 | CA | GLU | B | 133 | | 27.592 | 71.012 | 11.774 | 1.00 | 25.52 | C |
| ATOM | 1617 | C | GLU | B | 133 | | 27.968 | 70.227 | 10.534 | 1.00 | 24.16 | C |
| ATOM | 1618 | O | GLU | B | 133 | | 27.626 | 70.631 | 9.440 | 1.00 | 23.55 | O |
| ATOM | 1619 | CB | GLU | B | 133 | | 28.780 | 71.850 | 12.253 | 1.00 | 25.81 | C |
| ATOM | 1620 | CG | GLU | B | 133 | | 28.349 | 72.927 | 13.218 | 1.00 | 29.77 | C |
| ATOM | 1621 | CD | GLU | B | 133 | | 29.552 | 73.639 | 13.797 | 1.00 | 35.21 | C |
| ATOM | 1622 | OE1 | GLU | B | 133 | | 30.698 | 73.113 | 13.818 | 1.00 | 37.67 | O |
| ATOM | 1623 | OE2 | GLU | B | 133 | | 29.339 | 74.772 | 14.258 | 1.00 | 40.45 | O |
| ATOM | 1624 | N | TRP | B | 134 | | 28.661 | 69.100 | 10.698 | 1.00 | 23.38 | N |
| ATOM | 1625 | CA | TRP | B | 134 | | 29.036 | 68.289 | 9.536 | 1.00 | 23.08 | C |
| ATOM | 1626 | C | TRP | B | 134 | | 27.809 | 67.762 | 8.755 | 1.00 | 24.01 | C |
| ATOM | 1627 | O | TRP | B | 134 | | 27.728 | 67.869 | 7.503 | 1.00 | 24.25 | O |
| ATOM | 1628 | CB | TRP | B | 134 | | 29.976 | 67.153 | 10.020 | 1.00 | 21.31 | C |
| ATOM | 1629 | CG | TRP | B | 134 | | 30.621 | 66.309 | 8.905 | 1.00 | 21.61 | C |
| ATOM | 1630 | CD1 | TRP | B | 134 | | 31.862 | 66.440 | 8.442 | 1.00 | 24.16 | C |
| ATOM | 1631 | CD2 | TRP | B | 134 | | 30.029 | 65.174 | 8.235 | 1.00 | 23.56 | C |
| ATOM | 1632 | NE1 | TRP | B | 134 | | 32.101 | 65.474 | 7.471 | 1.00 | 24.07 | N |
| ATOM | 1633 | CE2 | TRP | B | 134 | | 30.989 | 64.701 | 7.308 | 1.00 | 22.25 | C |
| ATOM | 1634 | CE3 | TRP | B | 134 | | 28.778 | 64.541 | 8.326 | 1.00 | 21.96 | C |
| ATOM | 1635 | CZ2 | TRP | B | 134 | | 30.754 | 63.616 | 6.469 | 1.00 | 27.42 | C |
| ATOM | 1636 | CZ3 | TRP | B | 134 | | 28.536 | 63.420 | 7.501 | 1.00 | 26.30 | C |
| ATOM | 1637 | CH2 | TRP | B | 134 | | 29.524 | 62.997 | 6.579 | 1.00 | 25.16 | C |
| ATOM | 1638 | N | LEU | B | 135 | | 26.870 | 67.199 | 9.520 | 1.00 | 23.97 | N |
| ATOM | 1639 | CA | LEU | B | 135 | | 25.647 | 66.708 | 8.894 | 1.00 | 24.01 | C |
| ATOM | 1640 | C | LEU | B | 135 | | 24.862 | 67.776 | 8.139 | 1.00 | 24.92 | C |
| ATOM | 1641 | O | LEU | B | 135 | | 24.358 | 67.508 | 7.053 | 1.00 | 23.90 | O |
| ATOM | 1642 | CB | LEU | B | 135 | | 24.790 | 65.919 | 9.895 | 1.00 | 25.28 | C |
| ATOM | 1643 | CG | LEU | B | 135 | | 25.433 | 64.597 | 10.387 | 1.00 | 23.86 | C |
| ATOM | 1644 | CD1 | LEU | B | 135 | | 24.693 | 64.208 | 11.727 | 1.00 | 27.20 | C |
| ATOM | 1645 | CD2 | LEU | B | 135 | | 25.223 | 63.498 | 9.336 | 1.00 | 26.10 | C |
| ATOM | 1646 | N | ALA | B | 136 | | 24.849 | 68.988 | 8.673 | 1.00 | 24.79 | N |
| ATOM | 1647 | CA | ALA | B | 136 | | 24.175 | 70.115 | 8.054 | 1.00 | 26.27 | C |
| ATOM | 1648 | C | ALA | B | 136 | | 24.882 | 70.538 | 6.759 | 1.00 | 26.61 | C |
| ATOM | 1649 | O | ALA | B | 136 | | 24.218 | 70.813 | 5.734 | 1.00 | 27.03 | O |
| ATOM | 1650 | CB | ALA | B | 136 | | 24.098 | 71.247 | 9.090 | 1.00 | 27.24 | C |
| ATOM | 1651 | N | ARG | B | 137 | | 26.214 | 70.538 | 6.775 | 1.00 | 25.23 | N |
| ATOM | 1652 | CA | ARG | B | 137 | | 26.966 | 70.920 | 5.563 | 1.00 | 27.39 | C |
| ATOM | 1653 | C | ARG | B | 137 | | 26.713 | 69.924 | 4.437 | 1.00 | 26.77 | C |
| ATOM | 1654 | O | ARG | B | 137 | | 26.769 | 70.271 | 3.245 | 1.00 | 26.78 | O |
| ATOM | 1655 | CB | ARG | B | 137 | | 28.519 | 70.997 | 5.807 | 1.00 | 25.95 | C |
| ATOM | 1656 | CG | ARG | B | 137 | | 28.993 | 72.209 | 6.631 | 1.00 | 30.39 | C |
| ATOM | 1657 | CD | ARG | B | 137 | | 30.534 | 72.368 | 6.624 | 1.00 | 28.57 | C |
| ATOM | 1658 | NE | ARG | B | 137 | | 31.284 | 71.293 | 7.272 | 1.00 | 29.54 | N |
| ATOM | 1659 | CZ | ARG | B | 137 | | 31.541 | 71.226 | 8.574 | 1.00 | 28.73 | C |
| ATOM | 1660 | NH1 | ARG | B | 137 | | 31.097 | 72.156 | 9.416 | 1.00 | 30.49 | N |
| ATOM | 1661 | NH2 | ARG | B | 137 | | 32.259 | 70.200 | 9.034 | 1.00 | 29.03 | N |
| ATOM | 1662 | N | TRP | B | 138 | | 26.490 | 68.653 | 4.780 | 1.00 | 25.57 | N |
| ATOM | 1663 | CA | TRP | B | 138 | | 26.222 | 67.628 | 3.775 | 1.00 | 25.38 | C |
| ATOM | 1664 | C | TRP | B | 138 | | 24.720 | 67.578 | 3.430 | 1.00 | 26.43 | C |
| ATOM | 1665 | O | TRP | B | 138 | | 24.371 | 66.841 | 2.546 | 1.00 | 26.44 | O |
| ATOM | 1666 | CB | TRP | B | 138 | | 26.706 | 66.233 | 4.240 | 1.00 | 24.51 | C |
| ATOM | 1667 | CG | TRP | B | 138 | | 28.183 | 66.040 | 4.048 | 1.00 | 22.69 | C |
| ATOM | 1668 | CD1 | TRP | B | 138 | | 29.213 | 66.374 | 4.882 | 1.00 | 20.83 | C |
| ATOM | 1669 | CD2 | TRP | B | 138 | | 28.753 | 65.519 | 2.852 | 1.00 | 22.99 | C |
| ATOM | 1670 | NE1 | TRP | B | 138 | | 30.432 | 66.032 | 4.260 | 1.00 | 22.57 | N |
| ATOM | 1671 | CE2 | TRP | B | 138 | | 30.141 | 65.495 | 3.025 | 1.00 | 24.95 | C |
| ATOM | 1672 | CE3 | TRP | B | 138 | | 28.179 | 65.041 | 1.656 | 1.00 | 25.37 | C |
| ATOM | 1673 | CZ2 | TRP | B | 138 | | 30.989 | 65.029 | 2.026 | 1.00 | 28.31 | C |
| ATOM | 1674 | CZ3 | TRP | B | 138 | | 29.015 | 64.568 | 0.662 | 1.00 | 27.38 | C |
| ATOM | 1675 | CH2 | TRP | B | 138 | | 30.399 | 64.550 | 0.873 | 1.00 | 27.80 | C |
| ATOM | 1676 | N | GLY | B | 139 | | 23.874 | 68.294 | 4.155 | 1.00 | 25.84 | N |
| ATOM | 1677 | CA | GLY | B | 139 | | 22.439 | 68.290 | 3.908 | 1.00 | 28.16 | C |
| ATOM | 1678 | C | GLY | B | 139 | | 21.765 | 66.989 | 4.262 | 1.00 | 29.34 | C |
| ATOM | 1679 | O | GLY | B | 139 | | 20.815 | 66.582 | 3.536 | 1.00 | 29.95 | O |
| ATOM | 1680 | N | ILE | B | 140 | | 22.226 | 66.377 | 5.353 | 1.00 | 28.00 | N |
| ATOM | 1681 | CA | ILE | B | 140 | | 21.696 | 65.100 | 5.811 | 1.00 | 28.47 | C |
| ATOM | 1682 | C | ILE | B | 140 | | 20.924 | 65.310 | 7.091 | 1.00 | 28.43 | C |
| ATOM | 1683 | O | ILE | B | 140 | | 21.408 | 65.833 | 8.102 | 1.00 | 28.97 | O |
| ATOM | 1684 | CB | ILE | B | 140 | | 22.822 | 64.043 | 6.070 | 1.00 | 28.34 | C |
| ATOM | 1685 | CG1 | ILE | B | 140 | | 23.596 | 63.767 | 4.778 | 1.00 | 25.46 | C |
| ATOM | 1686 | CG2 | ILE | B | 140 | | 22.196 | 62.734 | 6.646 | 1.00 | 27.73 | C |
| ATOM | 1687 | CD1 | ILE | B | 140 | | 24.939 | 62.969 | 4.970 | 1.00 | 26.31 | C |
| ATOM | 1688 | N | ALA | B | 141 | | 19.663 | 64.877 | 7.064 | 1.00 | 29.40 | N |
| ATOM | 1689 | CA | ALA | B | 141 | | 18.832 | 64.972 | 8.264 | 1.00 | 29.87 | C |
| ATOM | 1690 | C | ALA | B | 141 | | 19.235 | 63.911 | 9.285 | 1.00 | 31.14 | C |
| ATOM | 1691 | O | ALA | B | 141 | | 19.654 | 62.817 | 8.899 | 1.00 | 31.34 | O |
| ATOM | 1692 | CB | ALA | B | 141 | | 17.342 | 64.868 | 7.913 | 1.00 | 30.17 | C |
| ATOM | 1693 | N | HIS | B | 142 | | 19.130 | 64.224 | 10.569 | 1.00 | 31.41 | N |
| ATOM | 1694 | CA | HIS | B | 142 | | 19.592 | 63.311 | 11.615 | 1.00 | 34.07 | C |
| ATOM | 1695 | C | HIS | B | 142 | | 18.498 | 63.116 | 12.656 | 1.00 | 37.42 | C |
| ATOM | 1696 | O | HIS | B | 142 | | 17.983 | 64.104 | 13.187 | 1.00 | 37.54 | O |
| ATOM | 1697 | CB | HIS | B | 142 | | 20.881 | 63.878 | 12.210 | 1.00 | 32.71 | C |
| ATOM | 1698 | CG | HIS | B | 142 | | 21.530 | 63.080 | 13.303 | 1.00 | 30.93 | C |
| ATOM | 1699 | ND1 | HIS | B | 142 | | 22.158 | 63.714 | 14.356 | 1.00 | 30.54 | N |
| ATOM | 1700 | CD2 | HIS | B | 142 | | 21.700 | 61.745 | 13.515 | 1.00 | 29.51 | C |
| ATOM | 1701 | CE1 | HIS | B | 142 | | 22.656 | 62.806 | 15.174 | 1.00 | 30.76 | C |
| ATOM | 1702 | NE2 | HIS | B | 142 | | 22.374 | 61.608 | 14.704 | 1.00 | 27.49 | N |
| ATOM | 1703 | N | THR | B | 143 | | 18.154 | 61.857 | 12.964 | 1.00 | 40.62 | N |
| ATOM | 1704 | CA | THR | B | 143 | | 17.144 | 61.623 | 14.002 | 1.00 | 43.48 | C |
| ATOM | 1705 | C | THR | B | 143 | | 17.794 | 60.786 | 15.093 | 1.00 | 44.40 | C |
| ATOM | 1706 | O | THR | B | 143 | | 18.562 | 59.831 | 14.812 | 1.00 | 43.48 | O |
| ATOM | 1707 | CB | THR | B | 143 | | 15.884 | 60.886 | 13.478 | 1.00 | 43.90 | C |
| ATOM | 1708 | OG1 | THR | B | 143 | | 16.202 | 59.517 | 13.240 | 1.00 | 48.35 | O |
| ATOM | 1709 | CG2 | THR | B | 143 | | 15.392 | 61.418 | 12.141 | 1.00 | 45.58 | C |
| ATOM | 1710 | N | THR | B | 144 | | 17.499 | 61.146 | 16.339 | 1.00 | 45.58 | N |
| ATOM | 1711 | CA | THR | B | 144 | | 18.002 | 60.347 | 17.469 | 1.00 | 47.28 | C |
| ATOM | 1712 | C | THR | B | 144 | | 16.823 | 59.718 | 18.220 | 1.00 | 48.25 | C |
| ATOM | 1713 | O | THR | B | 144 | | 16.973 | 59.283 | 19.365 | 1.00 | 49.64 | O |
| ATOM | 1714 | CB | THR | B | 144 | | 18.958 | 61.119 | 18.419 | 1.00 | 46.93 | C |
| ATOM | 1715 | OG1 | THR | B | 144 | | 18.290 | 62.273 | 18.947 | 1.00 | 49.78 | O |
| ATOM | 1716 | CG2 | THR | B | 144 | | 20.227 | 61.622 | 17.724 | 1.00 | 46.48 | C |
| ATOM | 1717 | N | SER | B | 150 | | 16.415 | 56.107 | 25.239 | 1.00 | 60.00 | N |
| ATOM | 1718 | CA | SER | B | 150 | | 17.613 | 55.320 | 24.938 | 1.00 | 60.00 | C |
| ATOM | 1719 | C | SER | B | 150 | | 17.266 | 53.980 | 24.284 | 1.00 | 60.00 | C |
| ATOM | 1720 | O | SER | B | 150 | | 17.807 | 52.968 | 24.701 | 1.00 | 60.00 | O |
| ATOM | 1721 | CB | SER | B | 150 | | 18.488 | 55.099 | 26.223 | 1.00 | 60.00 | C |
| ATOM | 1722 | N | GLN | B | 151 | | 16.391 | 53.937 | 23.280 | 1.00 | 60.00 | N |
| ATOM | 1723 | CA | GLN | B | 151 | | 16.032 | 52.664 | 22.634 | 1.00 | 60.00 | C |
| ATOM | 1724 | C | GLN | B | 151 | | 17.226 | 52.214 | 21.792 | 1.00 | 60.00 | C |
| ATOM | 1725 | O | GLN | B | 151 | | 18.299 | 52.817 | 21.879 | 1.00 | 60.00 | O |
| ATOM | 1726 | CB | GLN | B | 151 | | 14.757 | 52.833 | 21.778 | 1.00 | 60.00 | C |
| ATOM | 1727 | N | GLY | B | 152 | | 17.086 | 51.181 | 20.966 | 1.00 | 60.00 | N |
| ATOM | 1728 | CA | GLY | B | 152 | | 18.169 | 50.857 | 20.020 | 1.00 | 59.49 | C |
| ATOM | 1729 | C | GLY | B | 152 | | 17.610 | 50.715 | 18.599 | 1.00 | 58.03 | C |
| ATOM | 1730 | O | GLY | B | 152 | | 16.422 | 50.964 | 18.394 | 1.00 | 58.67 | O |
| ATOM | 1731 | N | GLN | B | 153 | | 18.426 | 50.313 | 17.622 | 1.00 | 55.83 | N |
| ATOM | 1732 | CA | GLN | B | 153 | | 17.890 | 49.976 | 16.296 | 1.00 | 53.07 | C |
| ATOM | 1733 | C | GLN | B | 153 | | 18.276 | 48.556 | 15.855 | 1.00 | 51.34 | C |
| ATOM | 1734 | O | GLN | B | 153 | | 19.460 | 48.240 | 15.695 | 1.00 | 50.79 | O |
| ATOM | 1735 | CB | GLN | B | 153 | | 18.292 | 51.014 | 15.241 | 1.00 | 53.56 | C |
| ATOM | 1736 | N | ALA | B | 154 | | 17.261 | 47.705 | 15.666 | 1.00 | 48.02 | N |
| ATOM | 1737 | CA | ALA | B | 154 | | 17.447 | 46.328 | 15.201 | 1.00 | 45.39 | C |
| ATOM | 1738 | C | ALA | B | 154 | | 18.408 | 46.009 | 14.046 | 1.00 | 43.43 | C |
| ATOM | 1739 | O | ALA | B | 154 | | 19.229 | 45.091 | 14.179 | 1.00 | 41.71 | O |
| ATOM | 1740 | CB | ALA | B | 154 | | 16.056 | 45.677 | 14.925 | 1.00 | 45.74 | C |
| ATOM | 1741 | N | MET | B | 155 | | 18.340 | 46.746 | 12.931 | 1.00 | 41.58 | N |
| ATOM | 1742 | CA | MET | B | 155 | | 19.137 | 46.364 | 11.774 | 1.00 | 39.80 | C |
| ATOM | 1743 | C | MET | B | 155 | | 20.635 | 46.552 | 12.025 | 1.00 | 37.77 | C |
| ATOM | 1744 | O | MET | B | 155 | | 21.428 | 45.700 | 11.631 | 1.00 | 34.33 | O |
| ATOM | 1745 | CB | MET | B | 155 | | 18.730 | 47.099 | 10.507 | 1.00 | 40.51 | C |
| ATOM | 1746 | CG | MET | B | 155 | | 17.369 | 46.720 | 9.985 | 1.00 | 44.73 | C |
| ATOM | 1747 | SD | MET | B | 155 | | 17.094 | 47.605 | 8.426 | 1.00 | 49.52 | S |
| ATOM | 1748 | CE | MET | B | 155 | | 18.279 | 46.847 | 7.322 | 1.00 | 45.25 | C |
| ATOM | 1749 | N | VAL | B | 156 | | 21.016 | 47.672 | 12.652 | 1.00 | 37.49 | N |
| ATOM | 1750 | CA | VAL | B | 156 | | 22.435 | 47.911 | 12.936 | 1.00 | 37.14 | C |
| ATOM | 1751 | C | VAL | B | 156 | | 22.976 | 46.935 | 14.011 | 1.00 | 37.53 | C |
| ATOM | 1752 | O | VAL | B | 156 | | 24.079 | 46.384 | 13.894 | 1.00 | 37.31 | O |
| ATOM | 1753 | CB | VAL | B | 156 | | 22.764 | 49.442 | 13.090 | 1.00 | 37.63 | C |
| ATOM | 1754 | CG1 | VAL | B | 156 | | 22.243 | 50.035 | 14.409 | 1.00 | 37.88 | C |
| ATOM | 1755 | CG2 | VAL | B | 156 | | 24.225 | 49.669 | 12.918 | 1.00 | 37.26 | C |
| ATOM | 1756 | N | GLU | B | 157 | | 22.192 | 46.658 | 15.055 | 1.00 | 39.16 | N |
| ATOM | 1757 | CA | GLU | B | 157 | | 22.581 | 45.660 | 16.085 | 1.00 | 41.58 | C |
| ATOM | 1758 | C | GLU | B | 157 | | 22.796 | 44.282 | 15.464 | 1.00 | 40.53 | C |
| ATOM | 1759 | O | GLU | B | 157 | | 23.714 | 43.576 | 15.849 | 1.00 | 41.12 | O |
| ATOM | 1760 | CB | GLU | B | 157 | | 21.636 | 45.615 | 17.327 | 1.00 | 42.59 | C |
| ATOM | 1761 | CG | GLU | B | 157 | | 21.669 | 46.945 | 18.085 | 1.00 | 48.90 | C |
| ATOM | 1762 | CD | GLU | B | 157 | | 21.247 | 47.021 | 19.589 | 1.00 | 55.47 | C |
| ATOM | 1763 | OE1 | GLU | B | 157 | | 22.101 | 47.274 | 20.483 | 1.00 | 57.13 | O |
| ATOM | 1764 | OE2 | GLU | B | 157 | | 20.021 | 46.896 | 19.877 | 1.00 | 57.91 | O |
| ATOM | 1765 | N | ARG | B | 158 | | 21.956 | 43.898 | 14.506 | 1.00 | 40.40 | N |
| ATOM | 1766 | CA | ARG | B | 158 | | 22.096 | 42.650 | 13.759 | 1.00 | 39.61 | C |
| ATOM | 1767 | C | ARG | B | 158 | | 23.358 | 42.649 | 12.886 | 1.00 | 38.57 | C |
| ATOM | 1768 | O | ARG | B | 158 | | 24.075 | 41.664 | 12.848 | 1.00 | 36.72 | O |
| ATOM | 1769 | CB | ARG | B | 158 | | 20.866 | 42.449 | 12.877 | 1.00 | 40.64 | C |
| ATOM | 1770 | CG | ARG | B | 158 | | 21.004 | 41.231 | 11.983 | 1.00 | 43.83 | C |
| ATOM | 1771 | CD | ARG | B | 158 | | 19.686 | 40.772 | 11.377 | 1.00 | 52.08 | C |
| ATOM | 1772 | NE | ARG | B | 158 | | 19.897 | 39.711 | 10.383 | 1.00 | 58.03 | N |
| ATOM | 1773 | CZ | ARG | B | 158 | | 20.200 | 38.439 | 10.663 | 1.00 | 60.00 | C |
| ATOM | 1774 | NH1 | ARG | B | 158 | | 20.349 | 38.033 | 11.923 | 1.00 | 60.00 | N |
| ATOM | 1775 | NH2 | ARG | B | 158 | | 20.358 | 37.547 | 9.682 | 1.00 | 60.00 | N |
| ATOM | 1776 | N | ALA | B | 159 | | 23.631 | 43.759 | 12.183 | 1.00 | 36.71 | N |
| ATOM | 1777 | CA | ALA | B | 159 | | 24.877 | 43.852 | 11.406 | 1.00 | 36.72 | C |
| ATOM | 1778 | C | ALA | B | 159 | | 26.104 | 43.718 | 12.338 | 1.00 | 36.26 | C |
| ATOM | 1779 | O | ALA | B | 159 | | 27.070 | 43.020 | 11.983 | 1.00 | 35.36 | O |
| ATOM | 1780 | CB | ALA | B | 159 | | 24.913 | 45.178 | 10.646 | 1.00 | 35.58 | C |
| ATOM | 1781 | N | ASN | B | 160 | | 26.058 | 44.383 | 13.494 | 1.00 | 37.33 | N |
| ATOM | 1782 | CA | ASN | B | 160 | | 27.133 | 44.294 | 14.500 | 1.00 | 38.54 | C |
| ATOM | 1783 | C | ASN | B | 160 | | 27.417 | 42.829 | 14.821 | 1.00 | 18.79 | C |
| ATOM | 1784 | O | ASN | B | 160 | | 28.553 | 42.389 | 14.822 | 1.00 | 38.94 | O |
| ATOM | 1785 | CB | ASN | B | 160 | | 26.828 | 45.020 | 15.818 | 1.00 | 38.42 | C |
| ATOM | 1786 | CG | ASN | B | 160 | | 26.840 | 46.543 | 15.742 | 1.00 | 40.72 | C |
| ATOM | 1787 | OD1 | ASN | B | 160 | | 26.379 | 47.200 | 16.692 | 1.00 | 43.33 | O |
| ATOM | 1788 | ND2 | ASN | B | 160 | | 27.347 | 47.122 | 14.667 | 1.00 | 39.70 | N |
| ATOM | 1789 | N | ARG | B | 161 | | 26.378 | 42.037 | 15.108 | 1.00 | 40.14 | N |
| ATOM | 1790 | CA | ARG | B | 161 | | 26.546 | 40.598 | 15.318 | 1.00 | 39.68 | C |
| ATOM | 1791 | C | ARG | B | 161 | | 27.055 | 39.788 | 14.157 | 1.00 | 39.08 | C |
| ATOM | 1792 | O | ARG | B | 161 | | 27.918 | 38.946 | 14.368 | 1.00 | 37.24 | O |
| ATOM | 1793 | CB | ARG | B | 161 | | 25.214 | 39.933 | 15.757 | 1.00 | 42.67 | C |
| ATOM | 1794 | CG | ARG | B | 161 | | 24.908 | 39.923 | 17.253 | 1.00 | 46.17 | C |
| ATOM | 1795 | CD | ARG | B | 161 | | 25.615 | 38.763 | 18.001 | 1.00 | 54.32 | C |
| ATOM | 1796 | NE | ARG | B | 161 | | 26.836 | 39.136 | 18.736 | 1.00 | 60.00 | N |
| ATOM | 1797 | CZ | ARG | B | 161 | | 28.077 | 38.768 | 18.414 | 1.00 | 60.00 | C |
| ATOM | 1798 | NH1 | ARG | B | 161 | | 28.305 | 37.983 | 17.353 | 1.00 | 60.00 | N |
| ATOM | 1799 | NH2 | ARG | B | 161 | | 29.093 | 39.180 | 19.170 | 1.00 | 60.00 | N |
| ATOM | 1800 | N | LEU | B | 162 | | 26.523 | 40.023 | 12.952 | 1.00 | 37.86 | N |
| ATOM | 1801 | CA | LEU | B | 162 | | 27.037 | 39.336 | 11.770 | 1.00 | 36.71 | C |
| ATOM | 1802 | C | LEU | B | 162 | | 28.498 | 39.650 | 11.527 | 1.00 | 35.85 | C |
| ATOM | 1803 | O | LEU | B | 162 | | 29.328 | 38.790 | 11.204 | 1.00 | 34.67 | O |
| ATOM | 1804 | CB | LEU | B | 162 | | 26.232 | 39.766 | 10.540 | 1.00 | 36.96 | C |
| ATOM | 1805 | CG | LEU | B | 162 | | 24.843 | 39.123 | 10.404 | 1.00 | 37.75 | C |
| ATOM | 1806 | CD1 | LEU | B | 162 | | 24.017 | 39.858 | 9.373 | 1.00 | 39.23 | C |
| ATOM | 1807 | CD2 | LEU | B | 162 | | 24.990 | 37.656 | 9.998 | 1.00 | 36.87 | C |
| ATOM | 1808 | N | LEU | B | 163 | | 28.812 | 40.934 | 11.648 | 1.00 | 35.81 | N |
| ATOM | 1809 | CA | LEU | B | 163 | | 30.188 | 41.321 | 11.390 | 1.00 | 36.25 | C |
| ATOM | 1810 | C | LEU | B | 163 | | 31.135 | 40.685 | 12.413 | 1.00 | 36.05 | C |
| ATOM | 1811 | O | LEU | B | 163 | | 32.150 | 40.115 | 12.055 | 1.00 | 35.65 | O |
| ATOM | 1812 | CB | LEU | B | 163 | | 30.200 | 42.852 | 11.431 | 1.00 | 36.29 | C |
| ATOM | 1813 | CG | LEU | B | 163 | | 31.174 | 43.733 | 10.676 | 1.00 | 38.41 | C |
| ATOM | 1814 | CD1 | LEU | B | 163 | | 31.500 | 43.246 | 9.298 | 1.00 | 35.17 | C |
| ATOM | 1815 | CD2 | LEU | B | 163 | | 30.431 | 45.104 | 10.708 | 1.00 | 37.84 | C |
| ATOM | 1816 | N | LYS | B | 164 | | 30.845 | 40.764 | 13.704 | 1.00 | 37.52 | N |
| ATOM | 1817 | CA | LYS | B | 164 | | 31.792 | 40.107 | 14.619 | 1.00 | 39.08 | C |
| ATOM | 1818 | C | LYS | B | 164 | | 31.981 | 38.601 | 14.386 | 1.00 | 39.18 | C |
| ATOM | 1819 | O | LYS | B | 164 | | 33.121 | 38.121 | 14.438 | 1.00 | 39.05 | O |
| ATOM | 1820 | CB | LYS | B | 164 | | 31.496 | 40.399 | 16.093 | 1.00 | 39.78 | C |
| ATOM | 1821 | CG | LYS | B | 164 | | 31.597 | 41.842 | 16.489 | 1.00 | 41.49 | C |
| ATOM | 1822 | CD | LYS | B | 164 | | 30.894 | 41.999 | 17.830 | 1.00 | 45.50 | C |
| ATOM | 1823 | CE | LYS | B | 164 | | 31.220 | 43.356 | 18.425 | 1.00 | 46.34 | C |
| ATOM | 1824 | NZ | LYS | B | 164 | | 30.288 | 43.712 | 19.518 | 1.00 | 45.15 | N |
| ATOM | 1825 | N | ASP | B | 165 | | 30.903 | 37.852 | 14.121 | 1.00 | 40.15 | N |
| ATOM | 1826 | CA | ASP | B | 165 | | 31.010 | 36.428 | 13.764 | 1.00 | 40.74 | C |
| ATOM | 1827 | C | ASP | B | 165 | | 31.876 | 36.201 | 12.554 | 1.00 | 40.09 | C |
| ATOM | 1828 | O | ASP | B | 165 | | 32.749 | 35.341 | 12.558 | 1.00 | 39.80 | O |
| ATOM | 1829 | CB | ASP | B | 165 | | 29.656 | 35.807 | 13.384 | 1.00 | 42.81 | C |
| ATOM | 1830 | CG | ASP | B | 165 | | 28.757 | 35.496 | 14.589 | 1.00 | 46.69 | C |
| ATOM | 1831 | OD1 | ASP | B | 165 | | 28.931 | 36.024 | 15.720 | 1.00 | 53.01 | O |
| ATOM | 1832 | OD2 | ASP | B | 165 | | 27.822 | 34.679 | 14.382 | 1.00 | 53.12 | O |
| ATOM | 1833 | N | LYS | B | 166 | | 31.620 | 36.993 | 11.509 | 1.00 | 38.55 | N |
| ATOM | 1834 | CA | LYS | B | 166 | | 32.398 | 36.882 | 10.298 | 1.00 | 37.11 | C |
| ATOM | 1835 | C | LYS | B | 166 | | 33.885 | 37.170 | 10.517 | 1.00 | 35.91 | C |
| ATOM | 1836 | O | LYS | B | 166 | | 34.741 | 36.425 | 10.010 | 1.00 | 34.39 | O |
| ATOM | 1837 | CB | LYS | B | 166 | | 31.774 | 37.709 | 9.162 | 1.00 | 37.44 | C |
| ATOM | 1838 | CG | LYS | B | 166 | | 32.391 | 37.470 | 7.795 | 1.00 | 39.15 | C |
| ATOM | 1839 | CD | LYS | B | 166 | | 32.322 | 36.028 | 7.260 | 1.00 | 43.03 | C |
| ATOM | 1840 | CE | LYS | B | 166 | | 31.012 | 35.710 | 6.559 | 1.00 | 45.01 | C |
| ATOM | 1841 | NZ | LYS | B | 166 | | 31.197 | 34.453 | 5.728 | 1.00 | 48.16 | N |
| ATOM | 1842 | N | ILE | B | 167 | | 34.208 | 38.255 | 11.230 | 1.00 | 35.83 | N |
| ATOM | 1843 | CA | ILE | B | 167 | | 35.602 | 38.570 | 11.569 | 1.00 | 35.66 | C |
| ATOM | 1844 | C | ILE | B | 167 | | 36.337 | 37.387 | 12.243 | 1.00 | 37.19 | C |
| ATOM | 1845 | O | ILE | B | 167 | | 37.450 | 37.031 | 11.828 | 1.00 | 36.40 | O |
| ATOM | 1846 | CB | ILE | B | 167 | | 35.665 | 39.827 | 12.450 | 1.00 | 36.12 | C |
| ATOM | 1847 | CG1 | ILE | B | 167 | | 35.303 | 41.030 | 11.543 | 1.00 | 35.49 | C |
| ATOM | 1848 | CG2 | ILE | B | 167 | | 37.059 | 39.964 | 13.057 | 1.00 | 33.83 | C |
| ATOM | 1849 | CD1 | ILE | B | 167 | | 35.170 | 42.385 | 12.280 | 1.00 | 34.65 | C |
| ATOM | 1850 | N | ARG | B | 168 | | 35.684 | 36.818 | 13.247 | 1.00 | 39.31 | N |
| ATOM | 1851 | CA | ARG | B | 168 | | 36.194 | 35.613 | 13.964 | 1.00 | 42.61 | C |
| ATOM | 1852 | C | ARG | B | 168 | | 36.497 | 34.446 | 12.986 | 1.00 | 42.67 | C |
| ATOM | 1853 | O | ARG | B | 168 | | 37.613 | 33.915 | 12.949 | 1.00 | 43.41 | O |
| ATOM | 1854 | CB | ARG | B | 168 | | 35.246 | 35.243 | 15.125 | 1.00 | 42.71 | C |
| ATOM | 1855 | CG | ARG | B | 168 | | 35.649 | 34.016 | 16.012 | 1.00 | 47.56 | C |
| ATOM | 1856 | CD | ARG | B | 168 | | 34.495 | 33.520 | 16.924 | 1.00 | 52.54 | C |
| ATOM | 1857 | NE | ARG | B | 168 | | 33.280 | 33.173 | 16.161 | 1.00 | 56.20 | N |
| ATOM | 1858 | CZ | ARG | B | 168 | | 33.056 | 32.001 | 15.541 | 1.00 | 59.96 | C |
| ATOM | 1859 | NH1 | ARG | B | 168 | | 33.943 | 31.006 | 15.546 | 1.00 | 60.00 | N |
| ATOM | 1860 | NH2 | ARG | B | 168 | | 31.921 | 31.795 | 14.875 | 1.00 | 60.00 | N |
| ATOM | 1861 | N | VAL | B | 169 | | 35.538 | 34.089 | 12.130 | 1.00 | 44.14 | N |
| ATOM | 1862 | CA | VAL | B | 169 | | 35.696 | 32.984 | 11.175 | 1.00 | 44.88 | C |
| ATOM | 1863 | C | VAL | B | 169 | | 36.878 | 33.210 | 10.238 | 1.00 | 45.22 | C |
| ATOM | 1864 | O | VAL | B | 169 | | 37.714 | 32.330 | 10.016 | 1.00 | 44.73 | O |
| ATOM | 1865 | CB | VAL | B | 169 | | 34.393 | 32.732 | 10.362 | 1.00 | 45.12 | C |
| ATOM | 1866 | CG1 | VAL | B | 169 | | 34.635 | 31.732 | 9.230 | 1.00 | 44.89 | C |
| ATOM | 1867 | CG2 | VAL | B | 169 | | 33.255 | 32.283 | 11.268 | 1.00 | 46.42 | C |
| ATOM | 1868 | N | LEU | B | 170 | | 36.971 | 34.418 | 9.675 | 1.00 | 45.26 | N |
| ATOM | 1869 | CA | LEU | B | 170 | | 38.043 | 34.700 | 8.732 | 1.00 | 44.96 | C |
| ATOM | 1870 | C | LEU | B | 170 | | 39.416 | 34.681 | 9.398 | 1.00 | 45.07 | C |
| ATOM | 1871 | O | LEU | B | 170 | | 40.365 | 34.111 | 8.845 | 1.00 | 45.19 | O |
| ATOM | 1872 | CB | LEU | B | 170 | | 37.814 | 36.031 | 8.011 | 1.00 | 45.66 | C |
| ATOM | 1873 | CG | LEU | B | 170 | | 36.568 | 36.085 | 7.125 | 1.00 | 45.38 | C |
| ATOM | 1874 | CD1 | LEU | B | 170 | | 36.423 | 37.444 | 6.466 | 1.00 | 44.12 | C |
| ATOM | 1875 | CD2 | LEU | B | 170 | | 36.612 | 34.991 | 6.096 | 1.00 | 46.62 | C |
| ATOM | 1876 | N | ALA | B | 171 | | 39.513 | 35.312 | 10.565 | 1.00 | 45.23 | N |
| ATOM | 1877 | CA | ALA | B | 171 | | 40.774 | 35.419 | 11.301 | 1.00 | 46.12 | C |
| ATOM | 1878 | C | ALA | B | 171 | | 41.231 | 34.000 | 11.640 | 1.00 | 47.31 | C |
| ATOM | 1879 | O | ALA | B | 171 | | 42.393 | 33.627 | 11.438 | 1.00 | 46.96 | O |
| ATOM | 1880 | CB | ALA | B | 171 | | 40.587 | 36.181 | 12.583 | 1.00 | 45.25 | C |
| ATOM | 1881 | N | GLU | B | 172 | | 40.288 | 33.242 | 12.182 | 1.00 | 48.79 | N |
| ATOM | 1882 | CA | GLU | B | 172 | | 40.591 | 31.865 | 12.591 | 1.00 | 50.33 | C |
| ATOM | 1883 | C | GLU | B | 172 | | 41.006 | 30.946 | 11.446 | 1.00 | 51.21 | C |
| ATOM | 1884 | O | GLU | B | 172 | | 41.743 | 30.011 | 11.717 | 1.00 | 52.29 | O |
| ATOM | 1885 | CB | GLU | B | 172 | | 39.546 | 31.311 | 13.576 | 1.00 | 50.93 | C |
| ATOM | 1886 | CG | GLU | B | 172 | | 39.819 | 31.927 | 14.929 | 1.00 | 51.91 | C |
| ATOM | 1887 | CD | GLU | B | 172 | | 38.829 | 31.606 | 16.024 | 1.00 | 55.28 | C |
| ATOM | 1888 | OE1 | GLU | B | 172 | | 37.725 | 31.106 | 15.748 | 1.00 | 58.14 | O |
| ATOM | 1889 | OE2 | GLU | B | 172 | | 39.186 | 31.875 | 17.191 | 1.00 | 58.02 | O |
| ATOM | 1890 | N | GLY | B | 173 | | 40.605 | 31.209 | 10.203 | 1.00 | 51.19 | N |
| ATOM | 1891 | CA | GLY | B | 173 | | 41.016 | 30.402 | 9.067 | 1.00 | 51.89 | C |
| ATOM | 1892 | C | GLY | B | 173 | | 42.432 | 30.749 | 8.653 | 1.00 | 52.57 | C |
| ATOM | 1893 | O | GLY | B | 173 | | 43.112 | 29.936 | 8.040 | 1.00 | 52.32 | O |
| ATOM | 1894 | N | ASP | B | 174 | | 42.896 | 31.954 | 8.996 | 1.00 | 52.50 | N |
| ATOM | 1895 | CA | ASP | B | 174 | | 44.237 | 32.409 | 8.644 | 1.00 | 53.04 | C |
| ATOM | 1896 | C | ASP | B | 174 | | 45.213 | 32.146 | 9.785 | 1.00 | 52.35 | C |
| ATOM | 1897 | O | ASP | B | 174 | | 46.386 | 32.483 | 9.694 | 1.00 | 52.31 | O |
| ATOM | 1898 | CB | ASP | B | 174 | | 44.226 | 33.893 | 8.234 | 1.00 | 53.27 | C |
| ATOM | 1899 | CG | ASP | B | 174 | | 43.370 | 34.143 | 7.000 | 1.00 | 56.37 | C |
| ATOM | 1900 | OD1 | ASP | B | 174 | | 42.716 | 33.166 | 6.543 | 1.00 | 56.66 | O |
| ATOM | 1901 | OD2 | ASP | B | 174 | | 43.339 | 35.295 | 6.479 | 1.00 | 58.29 | O |
| ATOM | 1902 | N | GLY | B | 175 | | 44.725 | 31.555 | 10.865 | 1.00 | 51.89 | N |
| ATOM | 1903 | CA | GLY | B | 175 | | 45.608 | 31.164 | 11.947 | 1.00 | 51.47 | C |
| ATOM | 1904 | C | GLY | B | 175 | | 45.767 | 32.187 | 13.052 | 1.00 | 51.32 | C |
| ATOM | 1905 | O | GLY | B | 175 | | 46.586 | 31.998 | 13.958 | 1.00 | 50.58 | O |
| ATOM | 1906 | N | PHE | B | 176 | | 45.000 | 33.276 | 12.975 | 1.00 | 50.07 | N |
| ATOM | 1907 | CA | PHE | B | 176 | | 45.042 | 34.283 | 14.032 | 1.00 | 49.52 | C |
| ATOM | 1908 | C | PHE | B | 176 | | 43.963 | 33.879 | 15.022 | 1.00 | 48.76 | C |
| ATOM | 1909 | O | PHE | B | 176 | | 42.792 | 33.935 | 14.714 | 1.00 | 48.54 | O |
| ATOM | 1910 | CB | PHE | B | 176 | | 44.888 | 35.697 | 13.434 | 1.00 | 48.41 | C |
| ATOM | 1911 | CG | PHE | B | 176 | | 45.838 | 35.943 | 12.293 | 1.00 | 49.29 | C |
| ATOM | 1912 | CD1 | PHE | B | 176 | | 47.211 | 35.811 | 12.495 | 1.00 | 49.18 | C |
| ATOM | 1913 | CD2 | PHE | B | 176 | | 45.370 | 36.286 | 11.022 | 1.00 | 49.18 | C |
| ATOM | 1914 | CE1 | PHE | B | 176 | | 48.115 | 36.024 | 11.461 | 1.00 | 50.19 | C |
| ATOM | 1915 | CE2 | PHE | B | 176 | | 46.262 | 36.500 | 9.967 | 1.00 | 49.87 | C |
| ATOM | 1916 | CZ | PHE | B | 176 | | 47.641 | 36.367 | 10.189 | 1.00 | 50.87 | C |
| ATOM | 1917 | N | MET | B | 177 | | 44.353 | 33.471 | 16.223 | 1.00 | 49.15 | N |
| ATOM | 1918 | CA | MET | B | 177 | | 43.393 | 32.939 | 17.195 | 1.00 | 49.14 | C |
| ATOM | 1919 | C | MET | B | 177 | | 42.900 | 33.973 | 18.198 | 1.00 | 48.59 | C |
| ATOM | 1920 | O | MET | B | 177 | | 41.818 | 33.825 | 18.769 | 1.00 | 48.53 | O |
| ATOM | 1921 | CB | MET | B | 177 | | 43.996 | 31.723 | 17.929 | 1.00 | 50.32 | C |
| ATOM | 1922 | CG | MET | B | 177 | | 44.242 | 30.545 | 16.980 | 1.00 | 52.23 | C |
| ATOM | 1923 | SD | MET | B | 177 | | 42.645 | 30.035 | 16.316 | 1.00 | 59.41 | S |
| ATOM | 1924 | CE | MET | B | 177 | | 43.084 | 29.672 | 14.627 | 1.00 | 57.19 | C |
| ATOM | 1925 | N | LYS | B | 178 | | 43.690 | 35.025 | 18.406 | 1.00 | 47.59 | N |
| ATOM | 1926 | CA | LYS | B | 178 | | 43.349 | 36.091 | 19.338 | 1.00 | 46.97 | C |
| ATOM | 1927 | C | LYS | B | 178 | | 43.426 | 37.434 | 18.572 | 1.00 | 46.10 | C |
| ATOM | 1928 | O | LYS | B | 178 | | 42.572 | 37.682 | 17.720 | 1.00 | 45.89 | O |
| ATOM | 1929 | CB | LYS | B | 178 | | 44.215 | 36.043 | 20.605 | 1.00 | 46.80 | C |
| ATOM | 1930 | CG | LYS | B | 178 | | 43.984 | 34.822 | 21.544 | 1.00 | 49.14 | C |
| ATOM | 1931 | CD | LYS | B | 178 | | 43.681 | 35.258 | 23.003 | 1.00 | 53.47 | C |
| ATOM | 1932 | CE | LYS | B | 178 | | 43.769 | 34.121 | 24.043 | 1.00 | 56.46 | C |
| ATOM | 1933 | NZ | LYS | B | 178 | | 45.109 | 34.099 | 24.696 | 1.00 | 57.07 | N |
| ATOM | 1934 | N | ARG | B | 179 | | 44.429 | 38.284 | 18.805 | 1.00 | 44.79 | N |
| ATOM | 1935 | CA | ARG | B | 179 | | 44.503 | 39.564 | 18.091 | 1.00 | 42.37 | C |
| ATOM | 1936 | C | ARG | B | 179 | | 44.970 | 39.363 | 16.652 | 1.00 | 40.81 | C |
| ATOM | 1937 | O | ARG | B | 179 | | 45.912 | 38.594 | 16.395 | 1.00 | 39.77 | O |
| ATOM | 1938 | CB | ARG | B | 179 | | 45.434 | 40.541 | 18.840 | 1.00 | 43.70 | C |
| ATOM | 1939 | CG | ARG | B | 179 | | 45.275 | 42.023 | 18.474 | 1.00 | 45.61 | C |
| ATOM | 1940 | CD | ARG | B | 179 | | 46.165 | 42.929 | 19.342 | 1.00 | 48.78 | C |
| ATOM | 1941 | NE | ARG | B | 179 | | 47.588 | 42.954 | 18.983 | 1.00 | 53.02 | N |
| ATOM | 1942 | CZ | ARG | B | 179 | | 48.129 | 43.520 | 17.889 | 1.00 | 57.00 | C |
| ATOM | 1943 | NH1 | ARG | B | 179 | | 47.402 | 44.140 | 16.929 | 1.00 | 55.74 | N |
| ATOM | 1944 | NH2 | ARG | B | 179 | | 49.453 | 43.460 | 17.747 | 1.00 | 53.85 | N |
| ATOM | 1945 | N | ILE | B | 180 | | 44.327 | 40.052 | 15.698 | 1.00 | 37.32 | N |
| ATOM | 1946 | CA | ILE | B | 180 | | 44.794 | 40.008 | 14.317 | 1.00 | 36.06 | C |
| ATOM | 1947 | C | ILE | B | 180 | | 45.980 | 40.972 | 14.169 | 1.00 | 36.49 | C |
| ATOM | 1948 | O | ILE | B | 180 | | 45.850 | 42.127 | 14.603 | 1.00 | 36.10 | O |
| ATOM | 1949 | CB | ILE | B | 180 | | 43.653 | 40.465 | 13.371 | 1.00 | 34.59 | C |
| ATOM | 1950 | CG1 | ILE | B | 180 | | 42.458 | 39.538 | 13.544 | 1.00 | 34.81 | C |
| ATOM | 1951 | CG2 | ILE | B | 180 | | 44.096 | 40.403 | 11.917 | 1.00 | 34.33 | C |
| ATOM | 1952 | CD1 | ILE | B | 180 | | 41.097 | 40.102 | 13.273 | 1.00 | 35.90 | C |
| ATOM | 1953 | N | PRO | B | 181 | | 47.102 | 40.541 | 13.543 | 1.00 | 37.19 | N |
| ATOM | 1954 | CA | PRO | B | 181 | | 48.259 | 41.431 | 13.356 | 1.00 | 36.56 | C |
| ATOM | 1955 | C | PRO | B | 181 | | 47.809 | 42.643 | 12.549 | 1.00 | 36.30 | C |
| ATOM | 1956 | O | PRO | B | 181 | | 47.085 | 42.515 | 11.550 | 1.00 | 33.84 | O |
| ATOM | 1957 | CB | PRO | B | 181 | | 49.276 | 40.599 | 12.573 | 1.00 | 37.19 | C |
| ATOM | 1958 | CG | PRO | B | 181 | | 48.893 | 39.180 | 12.924 | 1.00 | 37.77 | C |
| ATOM | 1959 | CD | PRO | B | 181 | | 47.382 | 39.180 | 13.029 | 1.00 | 37.52 | C |
| ATOM | 1960 | N | ALA | B | 182 | | 48.220 | 43.804 | 13.031 | 1.00 | 36.18 | N |
| ATOM | 1961 | CA | ALA | B | 182 | | 47.792 | 45.046 | 12.394 | 1.00 | 35.73 | C |
| ATOM | 1962 | C | ALA | B | 182 | | 47.928 | 45.045 | 10.865 | 1.00 | 35.64 | C |
| ATOM | 1963 | O | ALA | B | 182 | | 47.047 | 45.586 | 10.169 | 1.00 | 35.59 | O |
| ATOM | 1964 | CB | ALA | B | 182 | | 48.517 | 46.230 | 13.075 | 1.00 | 36.52 | C |
| ATOM | 1965 | N | SER | B | 183 | | 48.981 | 44.439 | 10.308 | 1.00 | 35.57 | N |
| ATOM | 1966 | CA | SER | B | 183 | | 49.210 | 44.418 | 8.845 | 1.00 | 36.40 | C |
| ATOM | 1967 | C | SER | B | 183 | | 48.197 | 43.566 | 8.057 | 1.00 | 35.96 | C |
| ATOM | 1968 | O | SER | B | 183 | | 48.152 | 43.644 | 6.807 | 1.00 | 37.13 | O |
| ATOM | 1969 | CB | SER | B | 183 | | 50.639 | 43.929 | 8.507 | 1.00 | 37.57 | C |
| ATOM | 1970 | OG | SER | B | 183 | | 50.728 | 42.527 | 8.708 | 1.00 | 38.04 | O |
| ATOM | 1971 | N | LYS | B | 184 | | 47.380 | 42.791 | 8.771 | 1.00 | 34.56 | N |
| ATOM | 1972 | CA | LYS | B | 184 | | 46.382 | 41.880 | 8.183 | 1.00 | 33.82 | C |
| ATOM | 1973 | C | LYS | B | 184 | | 44.953 | 42.367 | 8.316 | 1.00 | 33.07 | C |
| ATOM | 1974 | O | LYS | B | 184 | | 44.047 | 41.821 | 7.669 | 1.00 | 33.52 | O |
| ATOM | 1975 | CB | LYS | B | 184 | | 46.518 | 40.491 | 8.823 | 1.00 | 34.45 | C |
| ATOM | 1976 | CG | LYS | B | 184 | | 47.887 | 39.848 | 8.499 | 1.00 | 36.89 | C |
| ATOM | 1977 | CD | LYS | B | 184 | | 47.910 | 39.237 | 7.098 | 1.00 | 42.80 | C |
| ATOM | 1978 | CE | LYS | B | 184 | | 49.338 | 38.985 | 6.622 | 1.00 | 43.92 | C |
| ATOM | 1979 | NZ | LYS | B | 184 | | 49.983 | 38.361 | 7.817 | 1.00 | 50.41 | N |
| ATOM | 1980 | N | GLN | B | 185 | | 44.765 | 43.380 | 9.175 | 1.00 | 31.04 | N |
| ATOM | 1981 | CA | GLN | B | 185 | | 43.435 | 43.755 | 9.621 | 1.00 | 30.36 | C |
| ATOM | 1982 | C | GLN | B | 185 | | 42.678 | 44.449 | 8.490 | 1.00 | 29.74 | C |
| ATOM | 1983 | O | GLN | B | 185 | | 41.478 | 44.254 | 8.380 | 1.00 | 31.26 | O |
| ATOM | 1984 | CB | GLN | B | 185 | | 43.517 | 44.731 | 10.795 | 1.00 | 29.72 | C |
| ATOM | 1985 | CG | GLN | B | 185 | | 43.890 | 44.050 | 12.115 | 1.00 | 28.77 | C |
| ATOM | 1986 | CD | GLN | B | 185 | | 43.949 | 45.035 | 13.254 | 1.00 | 30.42 | C |
| ATOM | 1987 | OE1 | GLN | B | 185 | | 43.714 | 46.231 | 13.069 | 1.00 | 28.50 | O |
| ATOM | 1988 | NE2 | GLN | B | 185 | | 44.304 | 44.556 | 14.436 | 1.00 | 31.08 | N |
| ATOM | 1989 | N | GLY | B | 186 | | 43.338 | 45.278 | 7.679 | 1.00 | 30.84 | N |
| ATOM | 1990 | CA | GLY | B | 186 | | 42.595 | 45.982 | 6.621 | 1.00 | 30.40 | C |
| ATOM | 1991 | G | GLY | B | 186 | | 41.947 | 45.019 | 5.622 | 1.00 | 30.30 | C |
| ATOM | 1992 | O | GLY | B | 187 | | 40.755 | 45.174 | 5.289 | 1.00 | 29.33 | O |
| ATOM | 1993 | N | GLU | B | 187 | | 42.729 | 44.053 | 5.142 | 1.00 | 30.55 | N |
| ATOM | 1994 | CA | GLU | B | 187 | | 42.206 | 43.121 | 4.112 | 1.00 | 31.76 | C |
| ATOM | 1995 | C | GLU | B | 187 | | 41.121 | 42.253 | 4.727 | 1.00 | 31.28 | C |
| ATOM | 1996 | O | GLU | B | 187 | | 40.082 | 41.935 | 4.094 | 1.00 | 31.17 | O |
| ATOM | 1997 | CB | GLU | B | 187 | | 43.354 | 42.298 | 3.498 | 1.00 | 33.44 | C |
| ATOM | 1998 | CG | GLU | B | 187 | | 44.266 | 43.073 | 2.531 | 1.00 | 36.43 | C |
| ATOM | 1999 | CD | GLU | B | 187 | | 43.805 | 42.951 | 1.068 | 1.00 | 45.46 | C |
| ATOM | 2000 | OE1 | GLU | B | 187 | | 43.931 | 41.789 | 0.578 | 1.00 | 49.71 | O |
| ATOM | 2001 | OE2 | GLU | B | 187 | | 43.343 | 43.941 | 0.411 | 1.00 | 42.25 | O |
| ATOM | 2002 | N | LEU | B | 188 | | 41.297 | 41.885 | 5.996 | 1.00 | 30.25 | N |
| ATOM | 2003 | CA | LEU | B | 188 | | 40.358 | 40.990 | 6.657 | 1.00 | 29.93 | C |
| ATOM | 2004 | C | LEU | B | 188 | | 39.028 | 41.682 | 6.951 | 1.00 | 28.41 | C |
| ATOM | 2005 | O | LEU | B | 188 | | 37.974 | 41.102 | 6.746 | 1.00 | 27.38 | O |
| ATOM | 2006 | CB | LEU | B | 188 | | 41.028 | 40.378 | 7.913 | 1.00 | 30.89 | C |
| ATOM | 2007 | CG | LEU | B | 188 | | 40.470 | 39.101 | 8.532 | 1.00 | 34.66 | C |
| ATOM | 2008 | CD1 | LEU | B | 188 | | 41.618 | 38.441 | 9.291 | 1.00 | 40.39 | C |
| ATOM | 2009 | CD2 | LEU | B | 188 | | 39.325 | 39.524 | 9.491 | 1.00 | 36.37 | C |
| ATOM | 2010 | N | LEU | B | 189 | | 39.064 | 42.952 | 7.393 | 1.00 | 26.07 | N |
| ATOM | 2011 | CA | LEU | B | 189 | | 37.824 | 43.655 | 7.655 | 1.00 | 25.69 | C |
| ATOM | 2012 | C | LEU | B | 189 | | 37.067 | 43.863 | 6.321 | 1.00 | 25.35 | C |
| ATOM | 2013 | O | LEU | B | 189 | | 35.838 | 43.763 | 6.295 | 1.00 | 25.58 | O |
| ATOM | 2014 | CB | LEU | B | 189 | | 38.107 | 45.031 | 8.350 | 1.00 | 25.26 | C |
| ATOM | 2015 | CG | LEU | B | 189 | | 36.862 | 45.862 | 8.615 | 1.00 | 24.16 | C |
| ATOM | 2016 | CD1 | LEU | B | 189 | | 35.894 | 45.301 | 9.682 | 1.00 | 25.25 | C |
| ATOM | 2017 | CD2 | LEU | B | 189 | | 37.331 | 47.282 | 9.081 | 1.00 | 28.18 | C |
| ATOM | 2018 | N | ALA | B | 190 | | 37.790 | 44.158 | 5.249 | 1.00 | 26.96 | N |
| ATOM | 2019 | CA | ALA | B | 190 | | 37.174 | 44.404 | 3.926 | 1.00 | 27.57 | C |
| ATOM | 2020 | C | ALA | B | 190 | | 36.535 | 43.100 | 3.400 | 1.00 | 28.47 | C |
| ATOM | 2021 | O | ALA | B | 190 | | 35.448 | 43.099 | 2.777 | 1.00 | 27.77 | O |
| ATOM | 2022 | CB | ALA | B | 190 | | 38.276 | 44.806 | 2.947 | 1.00 | 28.68 | C |
| ATOM | 2023 | N | LYS | B | 191 | | 37.242 | 41.994 | 3.628 | 1.00 | 28.95 | N |
| ATOM | 2024 | CA | LYS | B | 191 | | 36.737 | 40.676 | 3.203 | 1.00 | 30.09 | C |
| ATOM | 2025 | C | LYS | B | 191 | | 35.486 | 40.303 | 3.984 | 1.00 | 29.22 | C |
| ATOM | 2026 | O | LYS | B | 191 | | 34.550 | 39.708 | 3.407 | 1.00 | 28.59 | O |
| ATOM | 2027 | CB | LYS | B | 191 | | 37.937 | 39.694 | 3.329 | 1.00 | 30.74 | C |
| ATOM | 2028 | CG | LYS | B | 191 | | 37.741 | 38.196 | 3.128 | 1.00 | 40.26 | C |
| ATOM | 2029 | CD | LYS | B | 191 | | 39.115 | 37.448 | 2.956 | 1.00 | 46.40 | C |
| ATOM | 2030 | CE | LYS | B | 191 | | 39.129 | 36.124 | 3.776 | 1.00 | 51.43 | C |
| ATOM | 2031 | NZ | LYS | B | 191 | | 40.376 | 35.290 | 3.958 | 1.00 | 55.38 | N |
| ATOM | 2032 | N | ALA | B | 192 | | 35.429 | 40.632 | 5.286 | 1.00 | 28.05 | N |
| ATOM | 2033 | CA | ALA | B | 192 | | 34.242 | 40.378 | 6.094 | 1.00 | 27.99 | C |
| ATOM | 2034 | C | ALA | B | 192 | | 33.044 | 41.215 | 5.649 | 1.00 | 28.87 | C |
| ATOM | 2035 | O | ALA | B | 192 | | 31.906 | 40.710 | 5.533 | 1.00 | 26.74 | O |
| ATOM | 2036 | CB | ALA | B | 192 | | 34.504 | 40.570 | 7.611 | 1.00 | 28.95 | C |
| ATOM | 2037 | N | MET | B | 193 | | 33.313 | 42.504 | 5.360 | 1.00 | 25.79 | N |
| ATOM | 2038 | CA | MET | B | 193 | | 32.236 | 43.373 | 4.957 | 1.00 | 25.82 | C |
| ATOM | 2039 | C | MET | B | 193 | | 31.731 | 42.864 | 3.564 | 1.00 | 24.87 | C |
| ATOM | 2040 | O | MET | B | 193 | | 30.514 | 42.835 | 3.389 | 1.00 | 27.83 | O |
| ATOM | 2041 | CB | MET | B | 193 | | 32.800 | 44.791 | 4.769 | 1.00 | 24.38 | C |
| ATOM | 2042 | CG | MET | B | 193 | | 31.877 | 45.759 | 4.104 | 1.00 | 27.72 | C |
| ATOM | 2043 | SD | MET | B | 193 | | 32.544 | 47.457 | 4.092 | 1.00 | 27.48 | S |
| ATOM | 2044 | CE | MET | B | 193 | | 31.708 | 48.167 | 2.655 | 1.00 | 29.25 | C |
| ATOM | 2045 | N | TYR | B | 194 | | 32.632 | 42.501 | 2.662 | 1.00 | 25.33 | N |
| ATOM | 2046 | CA | TYR | B | 194 | | 32.238 | 42.044 | 1.291 | 1.00 | 25.87 | C |
| ATOM | 2047 | C | TYR | B | 194 | | 31.385 | 40.757 | 1.389 | 1.00 | 28.05 | C |
| ATOM | 2048 | O | TYR | B | 194 | | 30.291 | 40.696 | 0.812 | 1.00 | 26.97 | O |
| ATOM | 2049 | CB | TYR | B | 194 | | 33.452 | 41.861 | 0.401 | 1.00 | 25.59 | C |
| ATOM | 2050 | CG | TYR | B | 194 | | 33.042 | 41.630 | -1.053 | 1.00 | 27.10 | C |
| ATOM | 2051 | CD1 | TYR | B | 194 | | 32.673 | 40.344 | -1.483 | 1.00 | 28.74 | C |
| ATOM | 2052 | CD2 | TYR | B | 194 | | 33.004 | 42.701 | -1.964 | 1.00 | 29.26 | C |
| ATOM | 2053 | CE1 | TYR | B | 194 | | 32.275 | 40.144 | -2.796 | 1.00 | 28.59 | C |
| ATOM | 2054 | CE2 | TYR | B | 194 | | 32.600 | 42.492 | -3.314 | 1.00 | 27.72 | C |
| ATOM | 2055 | CZ | TYR | B | 194 | | 32.224 | 41.185 | -3.696 | 1.00 | 29.91 | C |
| ATOM | 2056 | OH | TYR | B | 194 | | 31.792 | 40.921 | -5.023 | 1.00 | 30.34 | O |
| ATOM | 2057 | N | ALA | B | 195 | | 31.892 | 39.786 | 2.162 | 1.00 | 29.47 | N |
| ATOM | 2058 | CA | ALA | B | 195 | | 31.136 | 38.531 | 2.404 | 1.00 | 30.54 | C |
| ATOM | 2059 | C | ALA | B | 195 | | 29.690 | 38.721 | 2.906 | 1.00 | 31.33 | C |
| ATOM | 2060 | O | ALA | B | 195 | | 28.745 | 38.095 | 2.386 | 1.00 | 32.32 | O |
| ATOM | 2061 | CB | ALA | B | 195 | | 31.973 | 37.600 | 3.320 | 1.00 | 30.66 | C |
| ATOM | 2062 | N | LEU | B | 196 | | 29.461 | 39.587 | 3.888 | 1.00 | 30.72 | N |
| ATOM | 2063 | CA | LEU | B | 196 | | 28.096 | 39.896 | 4.292 | 1.00 | 31.19 | C |
| ATOM | 2064 | C | LEU | B | 196 | | 27.238 | 40.586 | 3.238 | 1.00 | 31.94 | C |
| ATOM | 2065 | O | LEU | B | 196 | | 26.021 | 40.288 | 3.164 | 1.00 | 33.05 | O |
| ATOM | 2066 | CB | LEU | B | 196 | | 28.060 | 40.727 | 5.578 | 1.00 | 30.84 | C |
| ATOM | 2067 | CG | LEU | B | 196 | | 28.786 | 40.054 | 6.763 | 1.00 | 32.67 | C |
| ATOM | 2068 | CD1 | LEU | B | 196 | | 28.673 | 41.004 | 7.935 | 1.00 | 35.62 | C |
| ATOM | 2069 | CD2 | LEU | B | 196 | | 28.280 | 38.650 | 7.092 | 1.00 | 37.20 | C |
| ATOM | 2070 | N | ASN | B | 197 | | 27.833 | 41.456 | 2.422 | 1.00 | 28.84 | N |
| ATOM | 2071 | CA | ASN | B | 197 | | 27.044 | 42.139 | 1.395 | 1.00 | 30.09 | C |
| ATOM | 2072 | C | ASN | B | 197 | | 26.733 | 41.278 | 0.172 | 1.00 | 28.57 | C |
| ATOM | 2073 | O | ASN | B | 197 | | 25.807 | 41.612 | -0.556 | 1.00 | 30.84 | O |
| ATOM | 2074 | CB | ASN | B | 197 | | 27.730 | 43.431 | 0.939 | 1.00 | 28.56 | C |
| ATOM | 2075 | CG | ASN | B | 197 | | 27.572 | 44.507 | 2.004 | 1.00 | 29.57 | C |
| ATOM | 2076 | OD1 | ASN | B | 197 | | 26.554 | 44.537 | 2.698 | 1.00 | 30.81 | O |
| ATOM | 2077 | ND2 | ASN | B | 197 | | 28.566 | 45.366 | 2.167 | 1.00 | 30.35 | N |
| ATOM | 2078 | N | HIS | B | 198 | | 27.505 | 40.221 | -0.004 | 1.00 | 29.69 | N |
| ATOM | 2079 | CA | HIS | B | 198 | | 27.488 | 39.396 | -1.227 | 1.00 | 30.23 | C |
| ATOM | 2080 | C | HIS | B | 198 | | 27.335 | 37.934 | -0.852 | 1.00 | 32.30 | C |
| ATOM | 2081 | O | HIS | B | 198 | | 28.156 | 37.105 | -1.236 | 1.00 | 33.61 | O |
| ATOM | 2082 | CB | HIS | B | 198 | | 28.827 | 39.570 | -1.910 | 1.00 | 29.65 | C |
| ATOM | 2083 | CG | HIS | B | 198 | | 28.980 | 40.938 | -2.469 | 1.00 | 28.06 | C |
| ATOM | 2084 | ND1 | HIS | B | 198 | | 28.510 | 41.283 | -3.707 | 1.00 | 28.62 | N |
| ATOM | 2085 | CD2 | HIS | B | 198 | | 29.473 | 42.082 | -1.937 | 1.00 | 28.53 | C |
| ATOM | 2086 | CE1 | HIS | B | 198 | | 28.771 | 42.554 | -3.963 | 1.00 | 30.66 | C |
| ATOM | 2087 | NE2 | HIS | B | 198 | | 29.365 | 43.067 | -2.895 | 1.00 | 30.31 | N |
| ATOM | 2088 | N | PHE | B | 199 | | 26.322 | 37.694 | -0.034 | 1.00 | 33.98 | N |
| ATOM | 2089 | CA | PHE | B | 199 | | 26.025 | 36.357 | 0.475 | 1.00 | 35.72 | C |
| ATOM | 2090 | C | PHE | B | 199 | | 25.616 | 35.376 | -0.639 | 1.00 | 36.49 | C |
| ATOM | 2091 | O | PHE | B | 199 | | 25.136 | 35.699 | -1.754 | 1.00 | 36.44 | O |
| ATOM | 2092 | CB | PHE | B | 199 | | 24.856 | 36.495 | 1.462 | 1.00 | 35.55 | C |
| ATOM | 2093 | CG | PHE | B | 199 | | 23.633 | 37.106 | 0.846 | 1.00 | 36.33 | C |
| ATOM | 2094 | CD1 | PHE | B | 199 | | 22.765 | 36.352 | 0.073 | 1.00 | 36.50 | C |
| ATOM | 2095 | CD2 | PHE | B | 199 | | 23.351 | 38.451 | 1.034 | 1.00 | 38.91 | C |
| ATOM | 2096 | CE1 | PHE | B | 199 | | 21.633 | 36.924 | -0.517 | 1.00 | 38.07 | C |
| ATOM | 2097 | CE2 | PHE | B | 199 | | 22.235 | 39.030 | 0.453 | 1.00 | 41.40 | C |
| ATOM | 2098 | CZ | PHE | B | 199 | | 21.376 | 38.269 | -0.322 | 1.00 | 39.60 | C |
| ATOM | 2099 | OXT | PHE | B | 199 | | 25.727 | 34.159 | -0.430 | 1.00 | 37.71 | O |

### DISCUSSION

A well-recognised rule of the retroviral integration mechanism is that the catalytic core of IN harbours an invariant dimeric interface built with two pairs of facing α-helices (α1/α5 and α5/α1). A similar interface was observed in RSV, HIV and PFV INs, in either the single CCD or the multi-domain structures. The crystallographic results presented here challenge this model, by revealing a novel dimeric assembly with three pairs of facing helices (α1/α1, α3/α5 and α5/α3). Although the Cα trace of each CCD monomer is preserved, this novel association might result in significant displacements of the two terminal domains fused to the CCD, affecting the entire protein.

### Validity of the novel interface

Bioinformatic analyses of the novel dimeric interface give standard values for the buried surface as well as for the free energy of solvation. In addition, the crystallogenesis study shows that the two crystal forms (tetragonal and hexagonal corresponding to the conventional and novel interface, respectively) can also be obtained for the RAV-1 IN CCD_{A182T} single mutant whose sequence is identical to that of the RSV IN CCD peptide The A182T mutation is located away from the interface area and is unlikely to be linked to the dimerisation mechanism. Taken together, these results indicate that the novel interface is not a crystallisation artefact and might be observed in all alpharetroviral INs.

### Novel interface association

To better understand the dimerisation process, we have characterised the oligomerisation state of RAV-1 IN CCD by SEC-MALS (Supplementary Figure 1). The resulting data establish that the domain is monomeric in solution at a concentration as high as 2.3 mg/ml (140 µM). Consequently, we assume that the dimeric association of either of the two forms occurs during crystallogenesis. The novel dimeric structure was crystallised in the presence of Zn²⁺ with a MES buffer at pH 6.0, while the conventional form can be obtained in a citrate buffer at a similar pH (38). Thus, an acidic solution does not seem to be required to form the novel quaternary structure. We instead favour the hypothesis that this structure requires both an appropriate pH and the presence of zinc, as a zinc ion is located at a central position in the novel dimeric interface. Zn²⁺ is known in the literature as a crucial divalent cation for INs. It is implicated in the folding of the NTD, in which it is coordinated by two histidine and two cysteine residues (12,44). Moreover, it increases the *in vitro* catalytic activity of IN (12,16), although Mg²⁺ is considered to be the biologically relevant cofactor.

Further, MES also contributes to the stability of the RAV-1 IN CCD dimer (Figure 1A). This suggests that the interface cavity occupied by the sulphonate moiety could be filled by an organic sulphate or phosphate *in vivo.* It also appears that the binding of the morpholino group induces an important movement of residue Phe-199 at the C-terminus of helix α5. This residue is spatially adjacent to Phe-185 in HIV IN, which is often mutated to lysine in crystallographic studies in order to increase protein solubility (22,45). Such a substitution may impair MES fixation and could have hindered the detection of the novel dimeric interface in HIV IN.

### The role of Zn²⁺ in avian INs

Zinc binding has been studied intensively for the conventional dimer of RSV IN CCD (16,36). Crystal-soaking experiments were performed in solutions containing 2 mM to 100 mM ZnCl₂. Four coordinated Zn²⁺ were subsequently observed at sites I and II of the catalytic pocket and at two distant sites termed III and IV. Interestingly, binding site similarities are observed between this soaked structure and the novel crystal structure of RAV-1 IN CCD. Site I is conserved in both forms, with a Zn²⁺ bridging the catalytic residues Asp-64 and Asp-121. This further demonstrates that the novel dimeric assembly has no influence on the topology of the active site. Site II is not occupied as in most retroviral INs. Structural similarities between sites III and IV raise more questions, because the local environments of the two dimeric forms are distinct. In conventional RSV IN CCD, the two remaining Zn²⁺ are found at the surface of the protein and are coordinated by His-103 (site III) and His-198 (site IV), respectively. In the novel RAV-1 IN CCD structure, His-103 is buried deep in the dimeric interface and is connected to His-103 of the complementary monomer via one Zn²⁺ ion (Figure 1A). His-198 is still located at the protein surface, but it is coordinated to site I of a symmetry-related monomer via a second Zn²⁺ (Figure 2A). Thus, sites I and IV of RSV IN CCD merge into a single penta-coordinated site I in RAV-1 IN CCD, while sites III of each RSV IN monomer fuse into a central Zn²⁺ site to lock the RAV IN dimer together.

The molecular interface of RAV-1 IN CDD involving His-103 is larger than the crystallographic one implicating His-198, which further strengthens our proposal that the novel dimer is formed first in crystallisation drops thanks to the combined effects of acidic pH and zinc. Furthermore, we tried to crystallise the H103A mutant of RAV1 IN CCD without success, while crystallisation trials of RAV-1 IN CCD using a wide range of pH levels from 6 to 10 in the presence of 10 mM of ZnCl₂ yielded crystals only at pH 6.0.

Finally, we tried to characterise the emergence of the novel quaternary assembly in solution by Dynamic Light Scattering (DLS) and Small Angle X-ray Scattering (SAXS). However, the CCD domain of RAV-1 IN tends to aggregate in the presence of zinc so no reliable measures were obtained. We also noticed during our crystallisation studies that the protein begins to precipitate in solution before crystals appear.

### Predicting the novel interface in other retroviral INs

The His-103 residue of RAV-1 IN seems to play a key role in the formation of the novel interface. However, this residue is conserved only in alpharetroviruses (Figure 1B). This is also true of most of the residues in the basic groove observed at the surface of RAV-1 IN, such as Arg-74 and Arg-95, which could stabilise the phosphodiester chain of a single-stranded nucleic acid. On the other hand, a multiple sequence alignment reveals that residues Trp-134 and Trp-138 are conserved in several other INs or that aromatic residues are within this sequence region (Figure 1B). Our docking experiments suggest that these residues, located at the level of the outer pockets, could determine the hydrophobic stacking of the heterocyclic bases of the nucleic acid strand. Thus, a molecular model of the novel dimeric association realized with the CCD of HIV-1 includes the three hydrophobic pockets, but the basic property of the groove is not found. In conclusion, we could not find any strong structural evidence to support the presence of the novel interface in other retroviral INs. Nevertheless, differences may exist across retroviral INs; for example, LEDFG/p75 interacts specifically with lentiviral IN but not with other INs during integration (46).

### The putative biological role of the novel interface

The biological relevance of the novel dimeric form is not yet clear. It is possible, for example, that the reshuffling of the CCD interface in the presence of Zn²⁺ at an acidic pH could be a driving force for the integration mechanism in alpharetroviruses. However, the 3' processing activity of RSV IN is most significant at pH 8.5 and negligible at pH 5.0 (16). This loss of activity at acidic pH is explained by side-chain reorientations in the active site due to residue protonation (36). Moreover, *in vitro* strand transfer reactions within the entire RAV-1 IN protein are optimal at alkaline pH in the absence of zinc (28). These experimental conditions are the opposite of those required for the formation of the novel interface; thus, we believe that a biological function apart from the integration mechanism should be investigated.

Most important, the identification of a basic groove with the ability to bind a single-stranded nucleic acid chain at the level of the novel interface suggests new possibilities for IN function. RSV reverse transcriptase (RT) is an αβ heterodimer, which contains the polymerase, RNase H, and IN domains within the 95 kDa β subunit. Cleavage of the IN domain from the β subunit produces the 63 kDa α subunit and free IN enzyme. One function of the IN domain in the β subunit is to increase the affinity of RT to its substrate (47,48). Under certain conditions, the formation of (αβ)₂ RSV RT tetramers can be observed (49). Therefore, we speculate that this new IN interface may contribute to the binding of viral RNA or the single-stranded strong stop DNA generated during reverse transcription. In HIV, although it is not included in the RT complex, IN is also required for the efficient reverse transcription of the HIV genome (50,51). This reaction probably occurs in a phosphate-rich medium, leading to the hypothesis that the phosphate molecule may replace the sulphonate group of MES to favour the formation of the novel dimeric interface. The newly assembled IN could then associate with HIV-1 RT and a putative cellular partner (52) to enhance RNA binding and reverse transcription.

An alternate possibility, based on the fact that our novel interface is observed at an acidic pH and that acidification was recently reported to affect RSV capsid assembly, is that a specific step of the avian retroviral cycle might take place in an acidic cell compartment (53). A motor role for acidification after an encapsidation step in late endosomes would explain the structural and biochemical results obtained for both the capsid and the IN dimeric interface, but this hypothesis will need further evaluation.

To conclude, our findings show that avian IN CCDs may have at least two intermolecular interfaces permitting multifunctionality. A parallel could be drawn to other retroviral proteins, such as Vif in lentivirus, which contain intrinsically disordered regions and can therefore interact with multiple partners (54). Retroviruses with limited genome length could use this strategy to generate proteins with flexible structures to mediate more than one step of the viral cycle.

### REFERENCES

1. Lewinski,M.K. and Bushman,F.D. (2005) Retroviral DNA integration--mechanism and consequences. Adv. Genet., 55, 147-181.
2. Pommier,Y. and Neamati,N. (1999) Inhibitors of human immunodeficiency virus integrase. Adv. Virus Res., 52, 427-458.
3. Hazuda,D.J., Young,S.D., Guare,J.P., Anthony,N.J., Gomez,R.P., Wai,J.S., Vacca,J.P., Handt,L., Motzel,S.L., Klein,H.J. et al. (2004) Integrase inhibitors and cellular immunity suppress retroviral replication in rhesus macaques. Science, 305, 528-532.
4. Sato,M., Motomura,T., Aramaki,H., Matsuda,T., Yamashita,M., Ito,Y., Kawakami,H., Matsuzaki,Y., Watanabe,W., Yamataka,K. et al. (2006) Novel HIV-1 integrase inhibitors derived from quinolone antibiotics. J. Med Chem., 49, 1506-1508.
5. Marchand,C., Maddali,K., Metifiot,M. and Pommier,Y. (2009) HIV-1 IN inhibitors: 2010 update and perspectives. Curr. Top. Med. Chem., 9, 1016-1037.
6. Skalka,A.M. and Katz,R.A. (2005) Retroviral DNA integration and the DNA damage response. Cell Death Differ., 12 Suppl 1, 971-978.
7. Smith,J.A. and Daniel,R. (2006) Following the path of the virus: the exploitation of host DNA repair mechanisms by retroviruses. ACS Chem. Biol., 1, 217-226.
8. Smith,J.A., Wang,F.X., Zhang,H., Wu,K.J., Williams,K.J. and Daniel,R. (2008) Evidence that the Nijmegen breakage syndrome protein, an early sensor of double-strand DNA breaks (DSB), is involved in HIV-1 post-integration repair by recruiting the ataxia telangiectasia-mutated kinase in a process similar to, but distinct from, cellular DSB repair. Virol. J., 5, 11.
9. Zhao,Z., McKee,C.J., Kessl,J.J., Santos,W.L., Daigle,J.E., Engelman,A., Verdine,G. and Kvaratskhelia,M. (2008) Subunit-specific protein footprinting reveals significant structural rearrangements and a role for N-terminal Lys-14 of HIV-1 Integrase during viral DNA binding. J. Biol. Chem., 283, 5632-5641.
10. Heuer,T.S. and Brown,P.O. (1997) Mapping features of HIV-1 integrase near selected sites on viral and target DNA molecules in an active enzyme-DNA complex by photo-cross-linking. Biochemistry, 36, 10655-10665.
11. Heuer,T.S. and Brown,P.O. (1998) Photo-cross-linking studies suggest a model for the architecture of an active human immunodeficiency virus type 1 integrase-DNA complex. Biochemistry, 37, 6667-6678.
12. Zheng,R., Jenkins,T.M. and Craigie,R. (1996) Zinc folds the N-terminal domain of HIV-1 integrase, promotes multimerization, and enhances catalytic activity. Proc. Natl Acad. Sci. USA, 93, 13659-13664.
13. Engelman,A., Hickman,A.B. and Craigie,R. (1994) The core and carboxyl-terminal domains of the integrase protein of human immunodeficiency virus type 1 each contribute to nonspecific DNA binding. J. Virol., 68, 5911-5917.
14. Andrake,M.D. and Slcalka,A.M. (1995) Multimerization determinants reside in both the catalytic core and C terminus of avian sarcoma virus integrase. J. Biol. Chem., 270, 29299-29306.
15. Bujacz,G., Jaskolski,M., Alexandratos,J., Wlodawer,A., Merkel,G., Katz,R.A. and Skalka,A.M. (1996) The catalytic domain of avian sarcoma virus integrase: conformation of the active-site residues in the presence of divalent cations. Structure, 4, 89-96.
16. Bujacz,G., Alexandratos,J., Wlodawer,A., Merkel,G., Andrake,M., Katz,R.A. and Skalka,A.M. (1997) Binding of different divalent cations to the active site of avian sarcoma virus integrase and their effects on enzymatic activity. J. Biol. Chem., 272, 18161-18168.
17. Leh,H., Brodin,P., Bischerour,J., Deprez,E., Tauc,P., Brochon,J.C., LeCam,E., Coulaud,D., Auclair,C. and Mouscadet,J.F. (2000) Determinants of Mg2+-dependent activities of recombinant human immunodeficiency virus type 1 integrase. Biochemistry, 39, 9285-9294.
18. Chow,S.A., Vincent,K.A., Ellison,V. and Brown,P.O. (1992) Reversal of integration and DNA splicing mediated by integrase of human immunodeficiency virus. Science, 255, 723-726.
19. Murzin,A.G., Brenner,S.E., Hubbard,T. and Chothia,C. (1995) SCOP: a structural classification of proteins database for the investigation of sequences and structures. J. Mol. Biol., 247, 536-540.
20. Nowotny,M. (2009) Retroviral integrase superfamily: the structural perspective. EMBO Rep., 10, 144-151.
21. Jaskolski,M., Alexandratos,J.N., Bujacz,G. and Wlodawer,A. (2009) Piecing together the structure of retroviral integrase, an important target in AIDS therapy. FEBS J., 276, 2926-2946.
22. Chen,J.C., Krucinski,J., Miercke,L.J., Finer-Moore,J.S., Tang,A.H., Leavitt,A.D. and Stroud,R.M. (2000) Crystal structure of the HIV-1 integrase catalytic core and C-terminal domains: a model for viral DNA binding. Proc. Natl Acad. Sci. USA, 97, 8233-8238.
23. Wang,J.Y, Ling,H., Yang,W. and Craigie,R. (2001) Structure of a two-domain fragment of HIV-1 integrase: implications for domain organization in the intact protein. EMBO J., 20, 7333-7343.
24. Yang,Z.N., Mueser,T.C., Bushman,F.D. and Hyde,C.C. (2000) Crystal structure of an active two-domain derivative of Rous sarcoma virus integrase. J. Mol. Biol., 296, 535-548.
25. Ren,G., Gao,K., Bushman,F.D. and Yeager,M. (2007) Single-particle image reconstruction of a tetramer of HIV integrase bound to DNA. J. Mol. Biol., 366, 286-294.
26. Michel,F., Crucifix,C., Granger,F., Eiler,S., Mouscadet,J.F., Korolev,S., Agapkina,J., Ziganshin,R., Gottikh,M., Nazabal,A. et al. (2009) Structural basis for HIV-1 DNA integration in the human genome, role of the LEDGF/P75 cofactor. EMBO J., 28, 980-991.
27. Hare,S., Gupta,S.S., Valkov,E., Engelman,A. and Cherepanov,P. (2010) Retroviral intasome assembly and inhibition of DNA strand transfer. Nature, 464, 232-236.
28. Moreau,K., Faure,C., Verdier,G. and Ronfort,C. (2002) Analysis of conserved and non-conserved amino acids critical for ALSV (Avian leukemia and sarcoma viruses) integrase functions in vitro. Arch. Virol., 147, 1761-1778.
29. Kabsch,W. (1993) Automatic processing of rotation diffraction data from crystals of initially unknown symmetry and cell constants. J. Appl. Cryst., 26, 795-800.
30. Navaza,J. (2001) Implementation of molecular replacement in AMoRe. Acta Crystallogr. D, 57, 1367-1372.
31. CCP4. (1994) Collaborative Computational Project Number 4. Acta Crystallogr. D, 50, 760-763.
32. Emsley,P. and Cowtan,K. (2004) Coot: model-building tools for molecular graphics. Acta Crystallogr. D, 60, 2126-2132.
33. Hooft,R.W., Vriend,G., Sander,C. and Abola,E.E. (1996) Errors in protein structures. Nature, 381, 272.
34. Morris,G.M., Goodsell,D.S., Halliday,R.S., Huey,R., Hart,W.E., Belew,R.K. and Olson,A.J. (1998) Automated Docking Using a Lamarckian Genetic Algorithm and and Empirical Binding Free Energy Function. J. Comput. Chem., 19, 1639-1662.
35. Trott,O. and Olson,A.J. (2010) AutoDock Vina: improving the speed and accuracy of docking with a new scoring function, efficient optimization, and multithreading. J. Comput. Chem., 31, 455-461.
36. Lubkowski,J., Yang,F., Alexandratos,J., Merkel,G., Katz,R.A., Gravuer,K., Skalka,A.M. and Wlodawer,A. (1998) Structural basis for inactivating mutations and pH-dependent activity of avian sarcoma virus integrase. J. Biol. Chem., 273, 32685-32689.
37. Lubkowski,J., Yang,F., Alexandratos,J., Wlodawer,A., Zhao,H., Burke,T.R.,Jr., Neamati,N., Pommier,Y., Merkel,G. and Skallca,A.M. (1998) Structure of the catalytic domain of avian sarcoma virus integrase with a bound HIV-1 integrase-targeted inhibitor. Proc. Natl Acad. Sci. USA, 95, 4831-4836.
38. Lubkowski,J., Dauter,Z., Yang,F., Alexandratos,J., Merkel,G., Skallca,A.M. and Wlodawer,A. (1999) Atomic resolution structures of the core domain of avian sarcoma virus integrase and its D64N mutant. Biochemistry, 38, 13512-13522.
39. Greenwald,J., Le,V., Butler,S.L., Bushman,F.D. and Choe,S. (1999) The mobility of an HIV-1 integrase active site loop is correlated with catalytic activity. Biochemistry, 38, 8892-8898.
40. Dias,A., Bouvier,D., Crepin,T., McCarthy,A.A., Hart,D.J., Baudin,F., Cusack,S. and Ruigrok,R.W. (2009) The cap-snatching endonuclease of influenza virus polymerase resides in the PA subunit. Nature, 458, 914-918.
41. Krissinel,E. and Henriclc,K. (2007) Inference of macromolecular assemblies from crystalline state. J. Mol. Biol., 372, 774-797.
42. Bujacz,G., Jaskolski,M., Alexandratos,J., Wlodawer,A., Merkel,G., Katz,R.A. and Skalka,A.M. (1995) High-resolution structure of the catalytic domain of avian sarcoma virus integrase. J. Mol. Biol., 253, 333-346.
43. Maroun,R.G., Gayet,S., Benleulmi,M.S., Porumb,H., Zargarian,L., Merad,H., Leh,H., Mouscadet,J.F., Troalen,F. and Fermandjian,S. (2001) Peptide inhibitors of HIV-1 integrase dissociate the enzyme oligomers. Biochemistry, 40, 13840-13848.
44. Cai,M., Zheng,R., Caffrey,M., Craigie,R., Clore,G.M. and Gronenborn,A.M. (1997) Solution structure of the N-terminal zinc binding domain of HIV-1 integrase. Nat. Strut. Biol., 4, 567-577.
45. Jenkins,T.M., Engelman,A., Ghirlando,R. and Craigie,R. (1996) A soluble active mutant of HIV-1 integrase: involvement of both the core and carboxyl-terminal domains in multimerization. J. Biol. Chem., 271, 7712-7718.
46. Busschots,K., Vercammen,J., Emiliani,S., Benarous,R., Engelborghs,Y., Christ,F. and Debyser,Z. (2005) The interaction of LEDGF/p75 with integrase is lentivirus-specific and promotes DNA binding. J. Biol. Chem., 280, 17841-17847.
47. Soltis,D.A. and Skalka,A.M. (1988) The alpha and beta chains of avian retrovirus reverse transcriptase independently expressed in Escherichia coli: characterization of enzymatic activities. Proc. Natl Acad. Sci. U S A, 85, 3372-3376.
48. Werner,S. and Wohrl,B.M. (1999) Soluble Rous sarcoma virus reverse transcriptases alpha, alphabeta, and beta purified from insect cells are processive DNA polymerases that lack an RNase H 3' --> 5' directed processing activity. J. Biol. Chem., 274, 26329-26336.
49. Lin,T.H., Quinn,T., Walsh,M., Grandgenett,D. and Lee,J.C. (1991) Avian myeloblastosis virus reverse transcriptase. Effect of glycerol on its hydrodynamic properties. J. Biol. Chem., 266, 1635-1640.
50. Dobard,C.W., Briones,M.S. and Chow,S.A. (2007) Molecular mechanisms by which human immunodeficiency virus type 1 integrase stimulates the early steps of reverse transcription. J. Virol., 81, 10037-10046.
51. Wu,W., Henderson,L.E., Copeland,T.D., Gorelick,R.J., Bosche,W.J., Rein,A. and Levin,J.G. (1996) Human immunodeficiency virus type 1 nucleocapsid protein reduces reverse transcriptase pausing at a secondary structure near the murine leukemia virus polypurine tract. J. Virol., 70, 7132-7142.
52. Nishitsuji,H., Hayashi,T., Takahashi,T., Miyano,M., Kannagi,M. and Masuda,T. (2009) Augmentation of reverse transcription by integrase through an interaction with host factor, SIP1/Gemin2 Is critical for HIV-1 infection. PLoS One, 4, e7825.
53. Bailey,G.D., Hyun,J.K., Mitra,A.K. and Kingston,R.L. (2009) Proton-linked dimerization of a retroviral capsid protein initiates capsid assembly. Structure, 17, 737-748.
54. Reingewertz,T.H., Shalev,D.E. and Friedler,A. (2010) Structural Disorder in the HIV-1 Vif Protein and Interaction-Dependent Gain of Structure. Pootein Pept. Lett., 17, 988-998.
55. Gouet,P., Robert,X. and Courcelle,E. (2003) ESPript/ENDscript: Extracting and rendering sequence and 3D information from atomic structures of proteins. Nucl. Acids Res., 31, 3320-3323.
56. Nicholls,A., Sharp,K.A. and Honig,B. (1991) Protein folding and association: insights from the interfacial and thermodynamic properties of hydrocarbons. Proteins, 11, 281-296.

## Claims

1. A crystal of a catalytic core domain (CCD) of retroviral integrase obtainable in a MES buffer at pH 6.0.

2. The crystal of claim 1, wherein said CCD consists of residues 54-199 of RAV-1 integrase.

3. The crystal of claim 1 or claim 2, wherein the MES buffer also comprises Zn(II).

4. The structure of RAV-1 CCD as defined by the coordinates listed in Table 2.

5. The use of the structure of claim 4, as a structural model.

6. The use of the structural model according to claim 5, for high-throughput chemical screening.

7. The use according to claim 5 or claim 6, for the identification of one or more molecular species that modulate the CCD of retroviral integrase to inhibit at least part of its activity.

8. The use according to any of claims 5 to 7, for the identification of an anti-retroviral drug.

9. The use of claim 8, for the identification of a drug for preventing, alleviating or curing an HIV infection.

10. The use according to claim 5, for determining at least a portion of the three-dimensional structure of a molecular species.

11. A machine-readable data storage medium which comprises a data storage material encoded with machine readable data defined by the structure coordinates of the CCD of RAV-1 according to Table 2 or a homologue of this structure.
